(19) Europäisches Patentamt
European Patent Office
Office européen des brevets

(11) EP 0 644 947 B1

(12) EUROPEAN PATENT SPECIFICATION

(45) Date of publication and mention of the grant of the patent:
**21.04.1999 Bulletin 1999/16**

(21) Application number: **93909997.4**

(22) Date of filing: **14.05.1993**

(51) Int Cl.6: **C12P 21/08**, A61K 39/395, G01N 33/68, G01N 33/577, C07K 1/00

(86) International application number:
**PCT/EP93/01215**

(87) International publication number:
**WO 93/24647 (09.12.1993 Gazette 1993/29)**

(54) **ANTI-IDIOTYPIC MONOCLONAL ANTIBODIES AGAINST THE LEWIS Y-SPECIFIC MONOCLONAL ANTIBODY BR55-2 AND THEIR USES**

ANTIIDIOTYPISCHE MONOKLONALE ANTIKÖRPER GEGEN DEN LEWIS Y-SPEZIFISCHEN MONOKLONALEN ANTIKÖRPER BR55-2 UND DEREN VERWENDUNGEN

ANTICORPS MONOCLONAUX ANTI-IDIOTYPES DIRIGES CONTRE LES ANTICORPS MONOCLONAUX BR55-2 SPECIFIQUES DE LEWIS Y ET LEURS UTILISATIONS

(84) Designated Contracting States:
**AT BE CH DE DK ES FR GB GR IE IT LI LU NL PT SE**

(30) Priority: **22.05.1992 GB 9210930**
**22.05.1992 GB 9210929**
**22.05.1992 GB 9210944**

(43) Date of publication of application:
**29.03.1995 Bulletin 1995/13**

(73) Proprietors:
- **Novartis AG**
  **4058 Basel (CH)**
  Designated Contracting States:
  **BE CH DE DK ES FR GB GR IE IT LI LU NL PT SE**
- **Novartis-Erfindungen Verwaltungsgesellschaft m.b.H.**
  **1230 Wien (AT)**
  Designated Contracting States:
  **AT**

(72) Inventors:
- **ECKERT, Helmut**
  **CH-4101 Oberwil (CH)**
- **JAKSCHE, Herbert**
  **A-1050 Vienna (AT)**
- **JANZEK, Evelyne**
  **A-1230 Vienna (AT)**
- **LOIBNER, Hans**
  **A-1238 Vienna (AT)**
- **SCHOLZ, Dieter**
  **A-1040 Vienna (AT)**

(56) References cited:
**EP-A- 0 445 078** **WO-A-90/00617**
**WO-A-92/03165**

- **JOURNAL OF IMMUNOLOGY vol. 145, no. 1, 1 July 1990, BALTIMORE MD, US pages 224 - 232 K. HIRASHIMA ET AL. 'High idiotypic connectivity of the Vh7183-encoded antibodies directed to a murine embryonic carbohydrate antigen Lewis Y, as ascertained by syngenic anti-idiotype monoclonal antibodies.'**
- **INTERNATIONAL JOURNAL OF CANCER vol. 50, no. 1, 2 January 1992, GENEVA, SWITZERLAND pages 86 - 92 H. LEHMANN ET AL. 'Tumor-antigen-specific humoral immune response of animals to anti-idiotypic antibodies and comparative serological analysis of patients with small cell lung carcinoma.'**
- **BRITISH JOURNAL OF CANCER vol. 63, no. S14, 1991, LONDON, GB pages 67 - 70 C. ZWICKY ET AL. 'Polyclonal anti-idiotypic antibodies mimicking the small cell lung carcinoma antigen cluster-5A interact with a panel of antibodies and induce specific immune response in animals.'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### 1. BACKGROUND AND INTRODUCTION

One approach towards manipulating the immune system is based on idiotypic interactions. The unique antigenic determinants in and around the antigen-combining site of an Ig molecule which make one antibody distinct from another are defined as idiotopes. The totality of all idiotopes present on the variable portion of a given antibody is referred to as its idiotype (id). The molecular structure of an idiotype has been localized to both the complementarity determining regions and the framework regions of the variable domain and is generally but not always contributed to by both the heavy and the light chains in specific association.

Idiotypes are serologically defined entities since injection of an antibody (often referred to as Ab1) into a syngeneic, allogeneic, or xenogeneic recipient induces the production of anti-idiotypic antibodies (often referred to as Ab2). Based on the assumption that idiotype/anti-idiotype interactions exist, physiologically a receptor-based regulation of the immune system was postulated by Niels Jerne (Ann. Immunol. 125C, 373, 1974). His network theory views the immune system as a collection of Ig molecules and receptors on T-lymphocytes, each capable of recognizing an antigenic determinant (epitope) through Its combining site (paratope), and each capable of being recognized by other antibodies or cell-surface receptors of the system through the idiotopes that it displays. Many studies have indeed demonstrated that idiotypic and anti-idiotypic receptors are present on the surface of both B- and T-lymphocytes as well as on secreted antibodies.

When the binding between Ab1 and Ab2 is inhibited by the antigen to which Ab1 is directed, the idiotype is considered to be binding-site-related, since it involves a site on the antibody variable domain that is engaged in antigen recognition. Those idiotypes which conformationally mimic an antigenic epitope are called the internal image of that epitope. Since both an Ab2 and an antigen bind to the relevant Ab1, they may share a similar three-dimensional conformation which represents the internal image of the given antigen. Internal image anti-idiotypic antibodies in principle can be seen as substitute of the antigen from which they have been derived via the idiotypic network. Therefore these surrogate antigens may be used in active immunization protocols. For example they offer advantages if the original antigen is not sufficiently immunogenic to induce a significant immune response. Thus, appropriate internal image anti-idiotypic antibodies that mimic a non-immunogenic carbohydrate antigen may be especially useful for certain vaccination approaches. In the following these topics are adressed more specifically.

As the result of the introduction of the hybridoma technology monoclonal antibodies (Mabs), mostly of mouse origin, have been made to many types of human cancer. Almost most of the markers defined by xenogeneic Mabs are not strictly tumor specific but are differentiation antigens shared by tumors and certain normal and/or fetal tissues. Therefore, they are best referred to as tumor associated antigens (TAAs). Whether human tumor markers detected by xenogeneic Mabs are capable of evoking an antitumor response in cancer patients, and whether such antigens are indeed related to the response to autologous tumors in cancer patients, depends on the nature of the respective TAA and is still not fully understood. TAAs which are either naturally immunogenic in the syngeneic host or can be made immunogenic might potentially be used to induce antitumor immunity for therapeutic and possibly prophylactic benefit.

Tumor associated antigens are often a part of "self" and evoke a very poor immune response in cancer patients. In contrast, internal image anti-idiotypic antibodies expressing three-dimensional shapes which resemble structural epitopes of the respective TAA are recognized as foreign molecules in the tumor bearing host. Therefore, the immune response raised by therapeutic or even prophylactic immunization with appropriate anti-id Mabs may cause antitumor immunity.

Therapeutic immunization against cancer with anti-id Mabs may be especially successful in earlier stages of the disease: At the time of surgery of a primary tumor, frequently occult single tumor cells already have been disseminated in various organs of the patient. These micrometastatic cells are known to be the cause for the later growth of metastases, often years after diagnosis and surgical removal of all clinically proven tumor tissue. So far in almost all cases metastatic cancer of epithelial origin is incurable. Therefore an effective treatment of "minimal residual cancer", e.g. destruction of occult micrometastatic cells in order to prevent the growth of macrometastases is an urgent medical need. At this stages of the disease (adjuvant setting) conventional chemotherapeutic approaches are rather unsuccessful. However, induction of a specific antitumor immunity at the time of minimal residual disease via immunization with appropriate internal image anti-id Mabs, micrometastatic cells may be selectively eliminated by the immune system, leading to an increased relapse-free survival time.

Monoclonal antibodies with specificity of BR55-2 (disclosed in e.g. Wistar EP 285 059, M.Blaszcyk-Thurin et al., J.Biol.Chem. 262 (1987) 372-379, or Z.Steplewski et al., Hybridoma 9 (1990) 201-210) define the Lewis Y6 antigen, a carbohydrate determinant selectively expressed on a majority of human solid tumors. Based on their properties antibodies BR55-2 can be used for passive immunotherapy of, basically, epithelial cancer.

The tumor associated Lewis Y oligosaccharide determinant which is also expressed during certain stages of embryonic development is almost not immunogenic by itself. However, monoclonal anti-idiotypic antibodies (Ab2) against BR55-2 (Ab1) with internal image properties by resembling structural epitopes of the Lewis Y antigen are useful for induction of a protective antitumor immunity, particularily in earlier stages of the disease.

In addition to its expression on cancer of epithelial origin the Lewis Y carbohydrate antigen is also involved in the pathogenesis of infection with Human Immunodeficiency Virus (HIV). Acquired immune deficiency syndrome (AIDS) is recognized as a distinct disease whose etiology has been identified as being associated with infection of a lymphotrophic retrovirus (HIV). The disease is characterized by a disorder associated with an impaired cell-mediated immunity and absolute lymphopenia, particularly reduced helper T-lymphocytes (CD4). AIDS may be preceded by a presyndrome that is usually manifested by a complex of designated clinical features and helper T-lymphopenia. The presyndrome is called AIDS-related complex (ARC).

HIV belongs to a group of viruses that have been intensively studied over the past two decades. When a retrovirus invades a human or animal cell, the RNA is turned into DNA and inserted into the host cell, which is then duped into treating the virus's genes as its own. HIV can remain latent in these cells for years, safe from attack by the body's immune system and blindly copied each time the host cell divides. Only in case of triggering rapid viral replication by activation of the infected cells the produced virus particles kill these cells and spill into the bloodstream.

Because of the specific features of HIV a cure by elimination of both the virus and the proviral genetic information already transcribed into the human genom from already infected patients is hard to achieve. Therefore, most therapeutic attempts have been concentrated on agents that slow down the development of the disease by Interfering with essential steps for the viral replication.

Prevention of HIV-infection and therapeutic intervention in already infected patients by means of a safe and effective vaccine is a major goal. Several vaccine approaches are tested in preclinical or early clinical phases. They are mainly based on viral structures as antigen, particularly on the major envelope glycoprotein gp120.

The naturally occurring immune response to the virus consists of antibodies to all viral proteins as well as activation of the cellular immunity. However, this host reaction to the HIV-infection does not appear to finally halt the progress of the disease after an asymptomatic phase which frequently lasts for years. Therefore, vaccination approaches based on the same antigens which cause the naturally occurring and finally non-protective immune response remain doubtful. One major problem is the extensive heterogeneity of HIV by which this virus escapes from the attack of type-specific neutralizing anti-gp120 antibodies.

Effective protection of HIV by vaccination requires two defense strategies: one against free virus traveling in the bloodstream, and another against cells that are already infected. It is known that HIV-infected cells in vitro and in vivo express on their surface an altered glycosylation pattern, namely the Lewis Y carbohydrate determinant. As this antigen normally occurs only during certain fetal development stages and is also associated with a variety of malignancies, the expression on HIV-infected cells may reflect their altered differentiation status induced by retroviral transformation. Therefore, this surface phenotype resembles an unique cellular host reaction to the transfection of the human genom by HIV.

The HIV envelope glycoprotein is performed by the glycosylation machinery of the infected cells. Therefore, changes in the glycosylation pattern of infected cells producing HIV are also found on free released virus partides. In consequence, the envelope glycoprotein of HIV generated in such cells also consists of Lewis Y carbohydrate determinants. Thus, the Lewis Y oligosaccharide represents a specific host response expressed both on HIV-infected cells and free HIV-particles.

Based on the above described considerations the Lewis Y structure fulfils important requirements for the use in a vaccination strategy against both free virus and HIV-infected cells. Furthermore, being a host reaction to HIV in general, the Lewis Y antigen is independent of HIV strain and not influenced by the genetic variability of this virus. Unfortunately, based on its carbohydrate structure and its 'self' properties as fetal differentiation antigen, Lewis Y is almost not immunogenic by itself. No natural immune response against this antigen has been detected in man. However, monoclonal anti-idiotypic antibodies (Ab2) against BR55-2 (Ab1) with internal image properties by resembling structural epitopes of the Lewis Y antigen may be useful for induction of prophylactic and therapeutic immunity against both free HIV and HIV-infected cells independent of virus strain.

In addition to their prophylactic and therapeutic use as unique vaccines, monoclonal internal image anti-id antibodies are highly specific reagents for the idiotype of the antibody from which they have been generated. They almost exclusively recognize the binding region of Ab1. This recognition pattern is independent of other determinants of Ab1. In other words, appropriate anti-id Mabs selectively define any molecule which consists of the unique idiotype of Ab1 in its correct three-dimensional shape. Therefore, these anti-id Mabs bind with comparable affinity to $F(ab')_2$-, Fab- and Fv-fragments of Ab1 as well as to its switch variants (switch variants consist of different constant regions, e. g. murine IgG2a, IgG2b, IgG1, but share the identical idiotype). In addition, this highly specific recognition pattern of internal image anti-id Mabs also includes variants of a parent murine Mab obtained by recombinant DNA technology.

In order to overcome possible problems of repeated use of murine antibodies for passive immunotherapy in humans, mouse/human chimeric Mabs can be generated by combining the variable domains of the parent murine Mab of choice with human constant regions. For the detection of such mouse/human chimeric Mabs in human serum highly specific reagents are required, since these chimeric Mabs bear constant regions identical to those of naturally occuring human immunoglobulins. Internal image anti-id Mabs generated against the parent murine Ab1 also recognize the chimeric antibodies derived thereof. Thus, such anti-idiotypic Mabs are useful reagents for the specific and sensitive quantitative determination of e.g. serum concentrations of mouse/human chimeric Mabs in the presence of a huge excess of normal human immunoglobulins.
To further improve the properties of Mabs with therapeutically interesting properties for uses in passive immunotherapy, "fully humanized" antibodies can be constructed by recombinant DNA technology in which only the minimum necessary parts of the parent mouse antibody, the complementarity determining regions (CDRs), are combined with human variable region frameworks and human constant regions. For the design and construction of these "fully humanized" Mabs, sequence homology and molecular modeling is used to select a combination of mouse and human sequence elements that would further reduce immunogenicity while retaining the binding properties. Certain internal image anti-Id Mabs generated against the idiotype of the parent murine Ab1 may still bind to CDR-grafted humanized variants if the following requirements are fulfilled:

a) The 3-dimensional structure of the combining region of the humanized variant is very similar to that of its parent mouse antibody.

b) The particular anti-id antibody exactly recognizes this 3-dimensional shape of the combining regions ("true internal image antibody")

Thus, anti-idiotypic Mabs bearing the above mentioned property are especially useful reagents for the specific and sensitive quantitative determination of e.g. serum concentrations of fully humanized (CDR-grafted) Mabs in the presence of a huge excess of normal human immunoglobulins.

The present invention concems the generation, production and characterization of murine monoclonal internal image anti-idiotypic antibodies (Ab2) to monoclonal antibodies BR55-2 (Ab1). A further part of the invention is the use of these anti-idiotypic Mabs for active therapeutic and prophylactic immunization against cancer of epithelial origin and small cell lung cancer as well as against diseases caused by HIV-infection. Furthermore the use of these anti-idiotypic Mabs for the highly selective and quantitative determination of antibodies or their fragments and derivatives with specificity of BR55-2 including chimerized and fully humanized variants (as described in WO 92/03165) as well as a generally applicable method for selective immuno-affinity purification of molecules with specificity of BR55-2 is also a part of the invention.

## 2. GENERATION AND CHARACTERIZATION OF MURINE MONOCLONAL ANTI-IDIOTYPIC ANTIBODIES AGAINST THE IDIOTYPE OF ANTIBODIES BR55-2

In an attempt to minimize undesired anti-isotypic immune responses, the $F(ab')_2$-fragment of BR55-2, murine IgG3, was chosen for immunization. For the successful generation of murine anti-id Mabs against the idiotype of the murine Mab BR55-2, it is important to maximize the immunogenicity in order to raise an appropriate immune response in the syngeneic host. Therefore the $F(ab')_2$-fragment which is devoid of the Fc-part (cleavage and purification described in EPA 528.767) was coupled to Keyhole Limpet Hemocyanin (KLH) as immunogenic carrier using the heterobifunctional linker N-succinimidyl 3-(2-pyridyldithio)propionate (=SPDP) according to described methods (J.Carlsson et al., Biochem.J. 173, 723, 1978).

Balb/c mice were immunized with this BR55-2/murine IgG3-$F(ab')_2$-KLH-conjugate using Freund complete adjuvant based on a typical protocol for the generation of murine Mabs. Following repeated immunizations the murine spleen cells were fused with the murine myeloma cell line SP2/0 (for experimental details see example 1).

For an appropriate selection of the cultured hybridoma cells a sequence of tests of their supernatants was performed. This selection was based on the following criteria:

a) Secretion rate of hybridomas by determination of the concentration of murine IgG in the supernatants (for experimental details see example 2). Cells producing high amounts of murine IgG were subcloned to single cell cultures.

b) Binding of selected supernatants to the $F(ab')_2$-fragment of BR55-2/murine IgG3 (for experimental details see example 3).

c) Inhibition of binding of BR55-2/murine IgG2a to Lewis Y antigen positive SKBR5 human breast cancer cells by selected supernatants (for experimental details see example 4).

The latter test is designed to be indicative for internal image properties of Ab2's. The murine IgG2a switch variant of BR55-2 was used for binding in order to minimize detection of Ab2's recognizing remaining constant regions of the $F(ab')_2$-fragment of BR55-2/murine IgG3 used for immunization. This test was performed in a quantitative manner based on the IgG concentration determined in test a) (example 2). Furthermore an excess of unspecific mouse-IgG was added to this inhibition experiment in order to avoid any detection of Ab2's that are not specific for the idiotype of BR55-2. Hybridomas were chosen which produce IgG with an inhibition capacity of more than 95% (inhibition of binding of BR55-2/murine IgG2a to the SKBR5 cell line).

Using the test procedures mentioned above six different hybridomas were finally selected and expanded (E4, C11, B3, B9, G6, G9). All six hybridomas produce murine IgG1 as detected by subtype ELISA using rabbit-anti-mouse-IgG1/peroxidase (such as the reagents of Zymed).

All six hybridomas were cultured in roller flasks (37°C, 5% $CO_2$ in medium G; change of medium every 3 to 4 days) and the supernatants were collected for subsequent purification.

Each supernatant containing the respective anti-id BR55-2 Mab was purified using immunoaffinity chromatography. In general, affinity chromatography is based on the interaction between an immobilized ligand and the substance of interest. In the case of anti-idiotypic antibodies BR55-2, the highly specific ligand for the affinity column is Mab BR55-2/murine IgG2a which binds the anti-idiotypic Mabs of choice (for experimental details see example 5).

The degree of purity of the isolated anti-id BR55-2 Mabs (E4, C11, B3, B9, G6, G9) was tested by analytical FPLC ion-exchange-chromatography, size-exclusion-chromatography, SDS-PAGE and isoelectric focussing. Purity of all six anti-id BR55-2 Mabs was >95% (for experimental details see example 6; SDS-PAGE and isoelectric focussing is shown in figures 1 and 2).

The purified anti-id Mabs were quantitatively characterized by determination of their capacity to inhibit binding of BR55-2/murine IgG3 to the Lewis Y antigen positive SKBR5 cell line. All anti-id Mabs inhibit the binding of Ab1 to its antigen based on a 1:1 stoechiometry (for experimental details see example 7; representative results are shown in figure 3).

A crucial proof of the internal image properties of the anti-id BR55-2 Mabs described above and their use as surrogate tumor antigen is based on their ability to generate an Ab3 response to the tumor associated antigen in different species. According to the network theory of N.Jerne antibodies (Ab3) induced by immunization with internal image anti-id Mabs (Ab2) have a binding specificity similar to that of Ab1. Therefore the immune response evoked by immunization with anti-id BR55-2 Mabs should be specific for Lewis Y antigen positive tumor cells. In consequence protective antitumor immunity may be induced in man by immunization with anti-id BR55-2 Mabs.

For the investigation of the properties of an Ab3 response rabbits as well as rhesus monkeys were immunized with anti-id BR55-2 #E4 and aluminium hydroxide as carrier and adjuvant. This mild adjuvant is widely used in different vaccines for human use. As a negative control the animals were also immunized with the same amount of unspecific mouse-IgG1. After four immunizations during 5 weeks sera were collected at week 9 (for experimental details see example 8). Binding of serum Ig to the Lewis Y antigen positive SKBR5 breast cancer cell line and to the Lewis Y antigen negative WM9 melanoma cell line was determined (for experimental details see examples 9 and 10).

Anti-id BR55-2 #E4 elicits a high titered humoral immune response both in rabbits and rhesus monkeys. Serum Ig of animals immunized with anti-id BR55-2 #E4 selectively binds to the Lewis Y antigen positive SKBR5 cell line but not to the Lewis Y antigen negative WM9 cell line. In contrast, by immunization with unspecific mouse-IgG1 almost no tumor cell binding serum Ig is detected. These results are summarized in Table 1. In figures 4 and 5 representative Ig-binding curves obtained with pre- and immune sera of rabbit- and rhesus monkeys in a cell-ELISA (SKBR5 cells) are shown. Binding of serum Ig of animals immunized with anti-id BR55-2 #E4 to SKBR5 cells can still be detected at a serum dilution of 1:10 000.

Two years after the initial immunization course the respective rhesus monkeys received a first single boost injection of anti-id BR55-2 #E4 or unspecific mouse-IgG1. In the same way a second single boost was given three years after initial immunization (= one year after first boost; for experimental details see example 8). Sera were collected before and after these boosts.

The total humoral immune response of the rhesus monkeys vaccinated with anti-id BR55-2#E4 was determined using an ELISA based on anti-id BR55-2#E4 coated to the microtiterplates (for experimental details see example 13). In the sera of all rhesus monkeys a very high titered immune response is found after the first immunization course as well as after the first and second boost. Ig binding to anti-id BR55-2 #E4 still is detected at serum dilutions of 1:50 000. A somewhat lower but still very significant titer is found in sera before the boost injections. These results are shown in in figures 6-8.

The humoral immune response of the rhesus monkeys vaccinated with unspecific mouse-IgG1 was also determined using the same ELISA mentioned above (for experimental details see example 13). As expected, the serum titers of Ig binding to anti-id BR55-2 #E4 are lower than the titers found in sera of monkeys vaccinated with anti-id BR55-2#E4. While the immune response of the rhesus monkeys immunized with unspecific mouse-IgG1 cross-reacts with the constant regions of anti-id BR55-2#E4, the fraction of the immune response directed to the hypervariable regions of the anti-id obviously is missing. These results are shown in in figures 9-10.

For proof of the tumor specificity of the immune response after the initial immunization course as well as before and after the boost immunizations, binding of monkey serum Ig to several Lewis Y antigen positive cancer cell lines (breast, gastric, colon and small cell lung cancer) as well as to a Lewis Y negative melanoma cell line was determined (for experimental details see example 10). In rhesus monkeys treated with anti-id BR55-2 #E4 two years after first immunization and before first boost immunization serum-Ig that binds to Lewis Y antigen positive tumor cells still can be detected. Following the first boost immunization tremendously increased titers of serum-Ig specifically binding to Lewis Y antigen positive tumor cells are found in animals immunized with anti-id BR55-2 #E4 but almost no binding to an antigen negative cell line is detected. No specific binding is observed at any time point in sera of rhesus monkeys immunized with unspecific mouse IgG1. Similar results are found with sera obtained before and after the second boost immunization. These results of titrations of sera obtained at various time points in Cell-ELISAS using several tumor cell lines are shown in figures 11- 20.

A similar pattern of immunoreactivity of Ig of different monkey sera before and after first immunization course as well as before and after the first boost immunization can also be demonstrated by binding experiments to a membrane preparation of Lewis Y antigen positive SKBR5 cells (for experimental details see example 11). The results are summarized in Table 2.

In order to further analyze in more detail the humoral immune response induced in rhesus monkeys by immunizations with anti-id BR55-2 #E4, different sera of vaccinated monkeys were immunopurified on a column loaded with anti-id BR55-2 #E4 coupled to Sepharose (for experimental details see example 12). By this selective one-step procedure the complete humoral immune response against anti-id BR55-2 #E4 is separated from all other irrelevant serum proteins. In a first experiment the Ig of a rhesus monkey immunized with anti-id BR55-2#E4 was immunopurified from the serum obtained at week 9 after the initial immunization course. In a second experiment the Ig of a rhesus monkey immunized with anti-id BR55-2#E4 was immunopurified from the pooled sera obtained at week 4 and 9 after the second boost. Based on their retention on the anti-id BR55-2#E4-column, the obtained Ig-fractions specifically bind to various epitopes of the anti-id BR55-2 #E4 antibody molecule. These Ig-fractions contain antibodies against the constant regions of anti-id BR55-2 #E4 (anti-isotypic immune response) as well as antibodies against the variable regions including the combining region of anti-id BR55-2 #E4 (anti-idiotypic immune response). Based on the idiotypic network theory mentioned in the introduction, the latter portion (AB3) is especially important since it exhibits binding properties comparable to those of antibodies with specificity of BR55-2 (AB1).

The binding of the immunopurified Ig-fraction from the pooled sera obtained at week 4 and 9 after the second boost and of the flow through fraction to anti-id BR55-2#E4 was determined (for experimental details see example 13). While the immunopurified Ig-fraction strongly binds to anti-id BR55-2#E4, no binding is detected for the flow-through fraction. By this result the selectivity and efficiency of this immunopurification method is proven (the results are shown in figure 21).

Next, binding properties of the immunopurified Ig-fraction from the pooled sera obtained at week 4 and 9 after the second boost, of the flow through fraction and of a humanized version of the parent murine Mab (AB1) were compared on SKBR5 breast cancer cells. This humanized AB1 variant (BR55-2/humanized IgG1) was chosen in order to facilitate the comparison since it can be detected with the same reagent used also for detection of the rhesus monkey Ig (goat-anti-human-Ig/peroxidase which similarily binds also to the closely related rhesus monkey Ig). Almost all tumor cell binding reactivity is accumulated in the immunopurified Ig-fraction. By this result the crossreactivity of rhesus monkey Ig induced by immunization with anti-id BR55-2 #E4 with a Lewis Y antigen positive tumor cell line is proven. Furthermore, on a weight to weight basis the binding strength of the immunopurified Ig-fraction is only 5 times lower than that of BR55-2/humanized IgG1 (AB1). Taking into consideration that only a certain portion of the immunopurified Ig-fraction is directed to the combining region of anti-id BR55-2 #E4 and according to the network theory only this portion exhibits binding properties comparable to AB1, this result is remarkable. Based on the yield of the immunoaffinity purification of rhesus monkey sera the titer of the immunopurified Ig-fraction amounts to approx. 200 μg/ml serum, corresponding to approx. 40 μg/ml of Ig with tumor cell binding properties similar to those of BR55-2/humanized IgG1 (the results are shown in figure 22, for experimental details see examples 10 and 12).

A comparison of the binding properties of the immunopurified monkey Ig (AB3) with those of BR55-2/humanized IgG1 on the Lewis Y positive breast cancer cell line SKBR5 and the Lewis Y negative melanoma cell line WM9 is shown in figure 23 (for experimental details see example 10). While both the AB3 fraction and BR55-2/humanized IgG1 strongly bind to the Lewis Y positive cell line as described above, both antibodies do not bind specifically to the Lewis Y negative cell line. By these results the similarity of the binding pattern of the AB3-fraction and the humanized AB1 variant is proven.

A similar pattern of immunoreactivity of the AB3-fraction is also observed in an ELISA using membrane fractions of the Lewis Y positive SKBR5 cell line and the Lewis Y negative cell line WM9. Substantial binding of the AB3-fractlon is only detected to the Lewis Y positive cell membranes (the results are shown in figure 24, for experimental details see example 11).

The binding properties of the immunopurified monkey Ig (AB3) were further substantiated on several Lewis Y positive human tumor cell lines (small cell lung cancer, gastric cancer, colon cancer, breast cancer). The immunopurified monkey Ig strongly binds to all tested cell lines (the results are shown in figure 25, for experimental details see example 10).

The AB1 antibody BR55-2/murine IgG3 following binding to tumor cells mediates tumor cell destruction via activation of human effector mechanisms. In order to test the capacity for tumor cell destruction of rhesus monkey Ig induced by immunization with anti-id BR55-2#E4, immunopurified monkey Ig (AB3) was used. For this experiment the AB3-fraction was immunopurified (see above) from serum of a rhesus monkey obtained 9 weeks after the initial immunization course with anti-id BR55-2#E4. This AB3-fraction was compared with BR55-2/murine IgG3 (AB1) in an ADCC experiment using human PBMC as effector cells (the results are shown in figure 26, for experimental details see example 14). The immunopurified AB3-fraction at higher concentrations mediates tumor cell destruction via activation of human effector cells comparably to the murine AB1. By this result the induction of selective tumor cell cytotoxicity by vaccination with anti-id BR55-2#E4 is demonstrated. A higher concentration of AB3 is required since only a certain portion of the immunopurified Ig-fraction is directed to the combining region of anti-id BR55-2 #E4 and only this portion is expected to bind to the tumor cells and to mediate ADCC.

As mentioned in the introduction, it is known that the Lewis Y carbohydrate antigen is also expressed on human leucocytes infected with HIV. In order to investigate the binding behavior of serum Ig of rhesus monkeys immunized with anti-id BR55-2 #E4 or with unspecific mouse IgG1 to HIV-infected cells, monkey sera obtained before and 9 weeks after the initial immunization course were tested in a commercially available test kit that originally has been designed for detection of HIV-seropositivity in human sera. This test is based on the ability of human serum Ig to bind to the HIV-infected human T cell line PALL as detected by indirect immunofluorescence using anti-human IgG-FITC. Because of the great similarity of human and rhesus monkey Ig the original reagent of the test kit can be also applied for rhesus monkey Ig. Binding to non-infected PALL cells serves as control (for experimental details see example 15).

With normal rhesus monkey serum the background immunofluorescence to both HIV-infected and non-infected PALL cells is slightly higher than the background immunofluorescence detected with normal human serum. However, only serum Ig of rhesus monkeys immunized with anti-id BR55-2 #E4 substantially binds to the HIV-infected PALL cells while almost no binding to uninfected PALL cells is detected. The reactivity of serum Ig of rhesus monkeys immunized with unspecific mouse-IgG1 to HIV-infected PALL cells is clearly less pronounced and close to the background immunofluorescence observed with normal monkey sera (the results are summarized in Table 3).

The Ig of a rhesus monkey immunized with anti-id BR55-2#E4 was immunopurified from the pooled sera obtained at week 4 and 9 after the second boost, using an anti-id BR55-2#E4 column (see above). The binding properties of this immunopurified Ig (AB3) were compared with those of BR55-2/murine IgG3 (AB1) in the already mentioned commercially available test kit designed for detection of HIV-seropositivity in human sera (for experimental details see example 15). The immunopurified Ig-fraction (AB3) selectively binds to the HIV-infected PALL cells, no binding is detected to non-infected PALL cells. A similar binding pattern is found for BR55-2/murine IgG3 (AB1). These results are summarized in table 4.

In addition to their use as unique vaccines for prophylaxis for and treatment of various cancers and diseases caused by HIV, the internal image anti-idiotypic Mabs raised against the binding region of BR55-2 described in this invention are powerful tools for the quantitative determination of monoclonal antibodies, their derivatives or fragments with binding specificity of BR55-2. For example they can be used for the selective determination of the concentration of all murine subtypes of BR55-2 in human sera. Because of the recognition of the three-dimensional shape of the binding region of BR55-2 these anti-id Mabs only bind to immunoreactive BR55-2 structures. This property leads to detection systems superior to those based on conventional anti-constant region reagents. An ELISA-system for the quantitative determination of immunoreactive BR55-2/murine IgG3 or BR55-2/murine IgG2a is described in example 16 and a typical standard curve of these Mabs in human serum is shown in figure 27.

A similar ELISA-system based on the anti-id Mabs described in this invention can be applied for the highly selective quantitative determination of immunoreactive mouse/human chimeras of BR55-2 in human serum. These chimeras consist of the variable domains of BR55-2 and of human constant regions (e.g. human IgG1 or human IgG3). Because of the huge excess of normal human Ig in human serum (>10 mg/ml), such mouse/human chimeras cannot be detected in human serum using conventional anti-human-Fc reagents which bind to any human Ig present in serum. The selective ELISA-system is described in examples 17 and 18, typical standard curves in human serum are shown in figures 28 and 29.

Fully humanized variants of BR55-2 have been constructed by grafting the complementarity determining regions (CDRs) of the murine parent Mab to human framework and constant regions. By means of computer modeling some point mutations in the human framework chosen lead to humanized variants without substantial loss in binding affinity to Lewis Y positive tumor cells in comparison to the parent murine Mab. Thus, in these humanized variants only the small portion of the original murine amino acid sequences determining the binding characteristics remains unchanged. Remarkably, all anti-id BR55-2 Mabs described in this invention also strongly bind to the idiotype of these humanized IgG1 variants of the parent mouse IgG3 Mab. The binding behavior of the anti-id Mabs to the humanized variants of AB1 is comparable to that to the mouse/human chimeric Mabs mentioned above. This is demonstrated by biospecific interaction analysis using the BIAcore™ system from Pharmacia. In most cases at higher concentrations almost a 1:1 stoechiometry between anti-id BR55-2 and BR55-2/chimeric human IgG1 or BR55-2/humanized IgG1 is observed. Experimental details are described in example 19, titration curves are shown in figures 30-35. These findings highlight the binding selectivity of the anti-id Mabs to the intact three-dimensional shape of the hypervariable region of all variants of BR55-2 and prove their internal image properties.

Based on this unique and selective recognition pattern the anti-id BR55-2 Mabs can also be used for the highly selective quantitative determination of immunoreactive humanized antibodies with specificity of BR55-2 in human serum despite of the huge excess of human immunoglobulin present in serum. The same ELISA-system is also useful for the quantitative determination of humanized antibodies with specificity of BR55-2 in monkey serum. This is e.g. important for monkey toxicity- and pharmacokinetic studies required during the preclinical development of a humanized BR55-2 Mab for passive immunotherapy. The ELISA-system is described in example 20, a typical standard curve in human serum is shown in figure 36.

The recognition of the binding region of all variants of BR55-2 by anti-id BR55-2 Mabs is also demonstrated by the competition of binding of BR55-2/murine IgG3 to anti-id BR55-2 #E4 by BR55-2/chimeric human IgG1 and BR55-2/chimeric human IgG3. Both chimeras competitively inhibit the binding of a fixed concentration of the parent murine Mab to anti-id BR55-2 #E4 in a dose dependent manner. This competition ELISA is described in example 21 and the results are shown in figure 37.

Binding of BR55-2/murine IgG3 to antigen positive tumor cell lines is a prerequisite for complement mediated destruction. Thus, inhibition of tumor cytotoxicity by anti-id BR55-2 Mabs reflect their ability to block the binding of Ab1 to its antigen on the cell surface. The potent neutralization by anti-id BR55-2 #E4 of complement dependent cytotoxicity to SKBR5 human breast cancer cells mediated by BR55-2/murine IgG3 is shown in figure 38, for experimental details see example 22.

Due to the highly specific recognition of all structures with intact binding region of BR55-2 the anti-id BR55-2 Mabs can be used for a single step immunopurification procedure of all variants of BR55-2. Anti-id BR55-2 #E4 has been coupled to CH-Sepharose 4B™. This immunoaffinity material for example can be used for a straight forward purification of BR55-2/chimeric human IgG1 cultured in vitro. The chimeric Mab was obtained in a yield of 75% with a purity of >95%. Because of the much higher selectivity this immuno-purification method is superior to affinity purification using Protein A. For experimental details see example 23, SDS-PAGE is shown in figure 39. No impurities are detected. The same method can be applied successfully with similar results for a highly efficient one-step purification of cell culture supernatants of a humanized variant of BR55-2 obtained by grafting of CDRs.

In conclusion, immunization of rabbits and rhesus monkeys with a murine monoclonal IgG1 anti-idiotypic antibody against the idiotype of antibodies with specificity of BR55-2 (anti-id BR55-2#E4) leads to a high titered immune response that specifically binds to Lewis Y positive human tumor cells. As demonstrated in the experiments in rhesus monkeys, by repeated boost immunizations the anti-tumor immunity is maintained for years. The Ig elicited by these immunizations in the presence of human effector cells exhibits tumor cytotoxicity in ADCC. The specificity of the anti-id immunization is proven by immunization of a control group of rhesus monkeys with unspecific mouse IgG1. Despite of a substantial immune response to the unspecific mouse IgG1 no specific binding to any kind of tumor cells is detected.

By these results the use of anti-id BR55-2 Mabs as surrogate of the Lewis Y tumor associated antigen for therapeutic and prophylactic active immunization with the aim of induction of a protective antitumor immunity in man is highlighted.

Furthermore, immunization of rhesus monkeys with an anti-id BR55-2 Mab leads to a high titered immune response which also selectively binds to HIV-infected positive cells. These cells are known to express Lewis Y. In contrast, immunization of rhesus monkeys with unspecific mouse IgG1 does not lead to any specific binding to HIV-positive or negative cells.

By these results the use of anti-id BR55-2 Mabs as surrogate for the Lewis Y carbohydrate antigen for therapeutic and prophylactic vaccination of man with the aim of induction of a protective immunity against HIV and diseases caused by HIV-infection is highlighted.

In addition to the use of anti-id BR55-2 Mabs as prophylactic and therapeutic vaccines, these internal image anti-id Mabs are useful reagents for the highly selective and quantitative determination of molecules with specificity of BR55-2 including chimerized and humanized variants in serum or other body fluids. These anti-id Mabs by covalent coupling to an appropriate matrix can also be used for highly selective single step immuno-affinity purifications of all molecules with specificity of BR55-2 with high yield.

The following examples illustrate the invention. The abbreviations have the following meanings:

ADCC: antibody dependent cellular cytotoxicity

| | |
|---|---|
| BSA: | bovine serum albumin |
| CDC: | complement dependent cytotoxicity |
| DMEM: | Dulbecco modified Eagle Medium |
| EDC: | N-ethyl-N'-(3-dimethylaminopropyl) carbodiimide hydrochloride |
| ELISA: | enzyme-linked immunosorbent assay |
| FCS: | fetal calf serum |
| HBS: | hepes-buffered saline |
| Mab: | monoclonal antibody |
| NHS: | N-hydroxysuccinimide |
| PBMC: | peripheral blood mononuclear cells |
| PBS: | phosphate-buffered saline |
| RAM-IgG1: | rabbit anti mouse IgG1 immunoglobulin |
| RPMI: | Roswell Park Memorial Institute |
| SDS: | sodium dodecyl sulfate |
| KLH: | keyhole limpet hemocyanin |
| SPDP: | N-succinimidyl-3-(2-pyridyl-dithio-propionate) |
| PAGE: | polyacrylamide gel electrophoresis |
| IEF: | isoelectric focussing |
| PEG: | poly-ethylene-glycol |
| FITC: | fluoreine isothiocyanate |
| IFA: | immunofluorescence assay |

The materials referred to in the examples are as follows:

Microtiterplates: Immunoplates II (Nunc)

Cell lines:

| | |
|---|---|
| SKBR5: | human breast cancer cell line |
| CATO: | human gastric cancer cell line |
| SW948: | human colon cancer cell line |
| SW2: | human small cell lung cancer line |
| WM9: | human melanoma cell line |
| PALL: | human T-cell line |
| SP2: | mouse myeloma cell line |

Medium A: RPMI 1640 + 2 g/l $NaHCO_3$
100 U/ml penicillin G
100 µg/ml streptomycin sulfate
4 mM glutamine
10% FCS (heat-inactivated, γ-globulin-free)

Medium B: RPMI 1640 + 2 g/l $NaHCO_3$
100 U/ml penicillin G
100 µg/ml streptomycin sulfate
4 mM glutamine
5% FCS (heat-inactivated)

Medium C: DMEM
10% NCTC-135 (synthetic medium, Gibco)
1% MEM non essential amino acids (Gibco)
0.5% sodium pyruvate
0.5% oxalacetic acid (Sigma)
20% FCS heat inactivated
4 mM glutamine
100 U/ml penicillin G
100 µg/ml streptomycin sulfate

| | |
|---|---|
| Medium D: | Medium C + 1.36 mg/l hypoxanthine<br>0.39 mg/l thymidine |
| Medium E: | Medium D + 0.4 mg/l aminopterine |
| Medium F: | Medium C + mouse thymocytes (thymocytes of one Balb/c mouse resuspended in 25 ml medium C) |
| Medium G: | DMEM<br>10% FCS heat inactivated<br>4 mM glutamine<br>100 U/ml penicillin G<br>100 µg/ml streptomycin sulfate |
| Medium H: | 10 mM N-(2-hydroxyethyl)piperazine-N'-2-ethanesulfonic acid<br>3,4 mM ethylenedinitrilo tetraacetic acid<br>0.15 M NaCl<br>0.005% P20 (Pharmacia Biosensor AB, Uppsala, Sweden) |
| PEG: | poly-ethylene-glycol (MW = 3400)<br>1 g is dissolved in 1 ml DMEM |
| PBS deficient: | 138.0 mM NaCl<br>1.5 mM KOH<br>2.7 mM KCl<br>6.5 mM $Na_2HPO_4$<br>pH 7.2 |

| | |
|---|---|
| Coating buffer: | 15 mM $Na_2CO_3$<br>35 mM $NaHCO_3$<br>3 mM $NaN_3$<br>pH 9.6 |
| Staining buffer: | 24.3 mM citric acid<br>51.4 mM $Na_2HPO_4$<br>pH 5.0 |
| Washing buffer: | 2% NaCl<br>0.2% Triton X-100<br>in PBS deficient |
| Substrate solution: | 40 mg o-phenylenediaminedihydrochloride<br>100 ml staining buffer<br>20 µl $H_2O_2$ 30% |
| Binding buffer: | 0.1 M Tris/HCl<br>0.2 M NaCl<br>pH 7.5 |
| Elution buffer: | 0.15 M glycine/HCl<br>0.2 M NaCl<br>pH 2.8 |
| Coupling buffer: | 0.1 M $NaHCO_3$<br>0.5 M NaCl<br>pH 8.0 |

In K.Hirashima et al., J.Immunol. 145 (1990) 224-232 anti-idiotypic Mabs against Lewis Y antigen Mabs are disclosed where the Lewis Y antigen is expressed as a cancer-associated antigen. Specifically, anti-id Mabs against Lewis Y antigen Mabs AH-6 are used. This prior art solution is applied to the immunization of mice whereas in the present invention internal image anti-id Mabs BR55-2 can replace the carbohydrate Lewis Y antigen for protective immunity in humans.

**Example 1: Generation of anti-id BR55-2 Mabs**

1.1: Immunization of mice

Balb/c mice are immunized with each 100 µg F(ab')$_2$-fragment of BR55-2/murine IgG3, coupled to KLH via SPDP as described (J.Carlsson et al., Biochem. J. 173, 723, 1978) by intraperitoneal injection in the following scheme:

| | |
|---|---|
| day 0: | 100 µg conjugate (1 mg/ml in PBS def.) + 100 µl Freund's complete adjuvant |
| day 7 and 28: | 100 µg conjugate (1 mg/ml in PBS def.) + 100 µl Freund's incomplete adjuvant |

On days 8, 9, 10 and 11 after primary immunization i.v. a total of 4 boost injections (each 100 µg conjugate in 100 µl PBS def.) are given. On day 12 the spleens are taken out aseptically, suspended in PBS def. and washed thrice in PBS def.

1.2: Hybridization

These spleen cells are added to a suspension of SP2/0 cells in a ratio 1:1 and centrifuged at 900 g for 5 minutes. 1 ml PEG-solution (37°C) is added dropwise to the cell pellet within 1 minute and diluted with 1 ml PBS def. (37°C) within the next minute. 10 ml medium C are added under gently rotation and the suspension is diluted to 50 ml with PBS def. The suspension is centrifuged at 800 g for 5 min, the pellet resuspended in medium D and the cells are transferred into the wells of a microtiterplate (Nunc 96) at a concentration of 2.5 x $10^5$ cells/well. After ovemight incubation at 37°C, 5% $CO_2$ 100 µl/well of medium E are added. After 72 hrs and then every four days the medium is replaced by medium D.

**Example 2: Quantitative determination of mouse IgG In hybridoma supernatants**

100 µl aliquots of rabbit-anti-mouse-IgG (such as the reagent of Nordic; 1:1000 in coating buffer) are added to the wells of microtiter plates, and incubated at 37°C for 60 minutes. The plates are washed 6 times with washing buffer, 200 µl of PBS def./5% FCS are added and incubated for 30 minutes at 37°C. The plates are washed as described above. 100 µl aliquots of the hybridoma supernatants obtained after 2 weeks culture are added and the plates are incubated for 60 minutes at 37°C. Unbound antibody is washed out as described above and 100 µl aliquots of peroxidase-conjugated antibody (rabbit-anti-mouse-IgG/peroxidase such as the reagents of Dianova; 1:1000 in PBS/2% FCS) are added. After incubation for 30 minutes at 37°C the plates are washed 4 times with washing buffer and twice with staining buffer. 100 µl aliquots of substrate solution are added and color development is stopped after 5 minutes with 50 µl aliquots of 4N $H_2SO_4$. Photometric extinction is measured at 492 nm (reference measurement 620 nm).

**Example 3: Specific binding of hybridoma supematant-IgG to BR55-2 F(ab')$_2$-fragment (ELISA)**

Hybridomas producing sufficient mouse IgG (i.e. more as 10-fold optical density than the medium-blank) are subcloned to single cell culture in medium F and cultured in medium C for additional 2 weeks. The supernatants are tested as described in example 2, using 100 µl aliquots of F(ab')$_2$-fragment of BR55-2 (10 µg/ml; dilution in coating buffer).

**Example 4: inhibition of binding of BR55-2/murine IgG2a to SKBR5 human breast cancer cells by hybridoma supernatant-IgG (cell ELISA)**

All hybridoma supernatants which are positive in the above described assay are tested as follows:

Microtiter plates are pretreated with poly-L-lysine hydrobromide (20-30 kD; 20 µg/ml in PBS def.; 100 µl/well; 30 minutes, room temperature), washed twice with PBS def. (200 µl/well) and then incubated overnight at 4°C with 50 µl/well of a suspension of SKBR5 cells In medium B (4 x $10^6$ cells/ml). After removal of the supernatant the cells are fixed with 50 µl glutardialdehyde/well (0.1% in physiological saline) for 5 minutes at room temperature, the supernatants are removed, the cells resuspended in 200 µl/well of PBS def./1% BSA/0.1% $NaN_3$ and left for 1 hour at room temperature. Supernatants are removed and the plates are washed twice with 200 µl/well of PBS containing 0.05% Tween 20™.

Hybridoma supernatants adjusted to 1 µg/ml mouse-IgG are preincubated with 10-fold excess of unspecific mouse-IgG for 30 min at 37°C. Then these samples are preincubated with 0.5 µg/ml BR55-2/murine IgG2a for 30 min at 37°C. 100 µl of this mixture are added to the cells and the plates are incubated for 1 hour at 37°C.

Working up of the mixture: Unbound antibody is washed out twice with 100 µl/well of ice-cold PBS containing 0.05% Tween 20™. 100 µl aliquots of peroxidase-conjugated antibody (rabbit-anti-mouse IgG2a/peroxidase such as the reagents of Zymed; 1:1000 in PBS def./2% FCS) are added. After incubation for 45 minutes at 37°C the wells are washed thrice with the PBS/Tween 20™ solution mentioned above and then 100 µl of the substrate solution is added to each well. After 5 minutes the color development is stopped by addition of 50 µl of 4 N $H_2SO_4$/well. Binding of the antibody to the cells is determined by measuring extinction at 492 nm (reference measurement 620 nm).

**Example 5: Immunoaffinity purification of anti-id BR55-2 Mabs**

**5.1: Preparation of BR55-2/murine IgG2a Sepharose**

10 g freeze dried activated CH-Sepharose 4B is suspended in 1mM HCl, transfered to a sinter glass filter and washed with 2 l 1 mM HCl for 15 minutes. The ligand (120 mg BR55-2/murine IgG2a) dissolved in 50 ml coupling buffer is mixed with the washed gel in a stoppered vessel and rotated end over end for one hour at room temperature. The gel is washed with coupling buffer and incubated for one hour with 50 ml 1 M ethanolamine for blocking of any remaining active groups. The affinity sorbent is then washed with three cycles of alternating pH. Each cycle consists of a wash at pH 4 (0.1 M acetate, 0.5 M NaCl) followed by a wash at pH 8 (0.1 M Tris, 0.5 M NaCl).

**5.2: Isolation of the anti-id BR55-2 Mabs**

The chromatography is performed at 4°C. The column (BIO REX MP™ column diameter 1.5 cm) is filled with Mab BR55-2/murine IgG2a Sepharose (volume 35 ml). The gel is washed with binding-buffer and elution buffer. After equilibration with binding buffer conditioned medium containing anti-id BR55-2 is loaded onto the column at a flow rate of 15 ml/min. After elution of the breakthrough fraction, the bound anti-id BR55-2 is desorbed with elution buffer and neutralized immediately after desorption with 1 M Tris/HCl buffer pH 7.5.

**5.3: Concentration of the anti-id BR55-2 Mabs**

Concentration of the eluted antibody solution (0.12 mg/ml) is performed in a stirred Amicon ultrafiltration cell using a PM 10 Diaflo membrane. The solute rejection for IgG is more than 98%, the final concentration of IgG amounts to 3.7 mg/ml.

**Example 6: Characterization of purified anti-id BR55-2 Mabs**

**6.1: Ion-exchange-chromatography on Mono-Q**

| | |
|---|---|
| Column | Mono-Q HR5/5 (Pharmacia) |
| Buffer A | 20 mM tri-ethanolamine, pH 7.7 |
| Buffer B | 20 mM tri-ethanolamine, 1 M NaCl, pH 7.7 |
| Flow rate | 1 ml/min |
| Detection | UV 280 nm |
| Gradient | linear 2%/min |
| Results | >95% purity found for all anti-id BR55-2 Mabs |

**6.2: High performance size-exclusion-chromatography**

| | |
|---|---|
| Column | Zorbax GF250™, 9.4 x 250 mm |
| Buffer | Sodium phosphate 0.1 M, 0.2 M NaCl, pH 7.0 |
| Flow rate | 1 ml/min |
| Detection | UV 280 nm |
| Results | >95% purity found for all anti-id BR55-2 Mabs |

6.3: SDS-PAGE

Experiments are performed both under reducing and non-reducing conditions according to the method of Laemmli using 10% acrylamide gels. No impurities are detected (results are shown in figure 1).

6.4: Isoelectric focussing

Analysis is performed with the Phast-system (Pharmacia) using a pH-gradient 3-9 (Phast gel IEF 3-9) and by silver staining (results are shown in figure 2).

**Example 7: Binding of BR55-2/murine IgG3 to SKBR5 cell line (cell-ELISA) - inhibition by purified anti-id Mabs**

The pretreatment of the microtiter plates is carried out as described in example 4. The respective anti-id BR55-2 Mab is diluted in PBS def. containing 2% FCS (10 to 0.5 µg/ml). To each of these dilutions 1 µg/ml BR55-2/murine IgG3 is added. 100 µl of this mixture are added to the cells and the plates are incubated for 1 hour at 37°C. The mixture is worked up as described in example 4. The extent of binding of BR55-2/murine IgG3 to the cells is determined by measuring extinction at 492 nm (reference measurement 620 nm).

**Example 8: Immunization of rabbits and rhesus monkeys with anti-id BR55-2 #E4**

**8.1: Immunization of rabbits with anti-id BR55-2 #E4**

Three female chinchilla rabbits are immunized by intradermal application of 300 µg anti-id BR55-2 #E4 adsorbed on aluminium hydroxide (1 mg antibody plus 3.3 mg $Al(OH)_3$/ml PBS def.) on days 1, 8, 15 and 36. Three rabbits are immunized with the same amount of unspecific mouse-IgG1 as negative control under the same conditions. Sera were collected before immunization and at week 9 after first immunization.

**8.2: Immunization of rhesus monkeys with anti-id BR55-2 #E4**

Three rhesus monkeys are immunized by subcutaneous (s.c.) application of 0.1 mg anti-id BR55-2 #E4/kg adsorbed on aluminium hydroxide (1 mg antibody plus 3.3 mg $Al(OH)_3$/ml PBS def.) on days 1, 8, 15 and 36. Two rhesus monkeys are immunized with the same amount of unspecific mouse-IgG1 as negative control under the same conditions. Sera are collected before immunization and at weeks 4 and 9 after first immunization.

Two years after the initial immunization course the same monkeys receive a first s.c. boost injection with anti-id BR55-2 #E4 or unspecific mouse-IgG1 respectively (same amount and formulation as above). Sera are collected before as well as 1 week and 4 weeks after this first boost immunization.

Three years after the initial immunization course (= one year after the first boost injection) the same monkeys receive a second s.c. boost injection with anti-id BR55-2 #E4 or unspecific mouse-IgG1 respectively (same amount and formulation as above). Sera are collected before as well as 1 week, 4 weeks and 9 weeks after this second boost immunization.

**Example 9: Binding of rabbit serum Ig to SKBR5 or WM9 cell line (cell-ELISA)**

The pretreatment of the microtiter plates is carried out as described in example 4. 100 µl aliquots of rabbit sera in appropriate predilutions are added to the cells and the plates are incubated for 1 hour at 37°C. Binding of the antibody to the cells Is determined by measuring extinction at 492 nm (reference measurement 620 nm).

**Example 10: Binding of rhesus monkey serum Ig or immunopurified Ig (AB3) to SKBR5, CATO, SW948, SW2 and WM9 human tumor cell lines (cell-ELISA)**

The pretreatment of the microtiter plates is carried out as described in example 4. 100 µl aliquots of rhesus monkey sera or immunopurified rhesus monkey Ig (see also example 12) and BR55-2 humanized IgG1 as control in appropriate predilutions are added to the cells and the plates are incubated for 1 hour at 37°C. The mixture is worked up as described in example 4. Binding of the antibody to the cells is determined by measuring extinction at 492 nm (reference measurement 620 nm).

**Example 11: Binding of rhesus monkey serum Ig or immunopurified Ig (AB3) to a SKBR5- or WM9-tumor cell-membrane preparation (ELISA)**

Membranes of the Lewis Y antigen positive SKBR5 human breast cancer cell line and the Lewis Y antigen negative WM9 melanoma cell line were prepared as described by D.Thom et al., Biochem.J. 168, 187-194 (1977). 100 µl aliquots of the respective membrane preparation (10 µg/ml; dilution in coating buffer) are added to the wells of microtiter plates, and incubated at +4°C overnight. The plates are washed 4 times with washing buffer, 200 µl of PBS def./5% FCS are added and incubated for 30 minutes at 37°C. The plates are washed as described above. 100 µl aliquots of rhesus monkey sera or immunopurified rhesus monkey Ig (see also 2) in appropriate dilutions in PBS def./ 2% FCS are added and the plates are incubated for 60 minutes at 37°C. Unbound antibody is washed out as described above and 100 µl aliquots of peroxidase-conjugated antibody (goat-anti-human-Ig/per-oxidase such as the reagents of Chemicon & Co., 1:1000 in PBS/2% FCS) are added. After incubation for 30 minutes at 37°C the plates are washed twice with washing buffer and twice with staining buffer.

100 µl aliquots of substrate solution are added and color development is stopped after 5 minutes with 50 µl aliquots of 4 N $H_2SO_4$. Optical density (OD) is measured at 492 nm (reference measurement 620 nm).

**Example 12: Immunoaffinity purification of serum Ig of rhesus monkeysimmunized with anti-id BR55-2 #E4 (AB3) using anti-id BR55-2 #E4-Sepharose™**

**12.1: Preparation of anti-id BR55-2 #E4-Sepharose™**

9 g freeze dried activated CH-Sepharose 4B™ is suspended in 1 mM HCl, transfered to a sinter glass filter and washed with 2 1 1 mM HCl for 15 minutes. The ligand (95 mg anti-id BR55-2 #E4) dissolved in 50 ml coupling buffer is mixed with the washed gel in a stoppered vessel and rotated end over end for one hour at room temperature. The gel is washed with coupling buffer and incubated for one hour with 50 ml 1M ethanolamine for blocking of any remaining active groups. The affinity sorbent is then washed with three cycles of alternating pH. Each cycle consists of a wash at pH 4 (0.1 M acetate, 0.5 M NaCl) followed by a wash at pH 8 (0.1 M Tris, 0.5 M NaCl).

**12.2: Isolation of serum Ig of rhesus monkeys (AB3) immunized with anti-id BR55-2 #E4**

The chromatography is performed at 4°C. The column (Pharmacia column HR 5/2, bed dimension 5 x 25 mm) is filled with anti-id BR55-2 #E4-Sepharose (volume 0,5 ml). The gel is washed with binding buffer and elution buffer. Starting materials are 2 ml of serum of a rhesus monkey immunized with anti-id BR55-2 #E4 obtained 9 weeks after start of immunizations. For another series of experiments 2ml of pooled sera of a rhesus monkey immunized with anti-id BR55-2 #E4 obtained 4 and 9 weeks after second boost was used. The respective serum is diluted 1:2 in binding buffer and loaded onto the column. After elution of the breakthrough fraction, the bound rhesus monkey immunoglobulin is desorbed with elution buffer and neutralized immediately after desorption with 1 M Tris/HCl buffer pH 7.5.

**Example 13: Binding of serum Ig or immunopurified Ig of rhesus monkeys immunized with anti-id BR55-2 #E4 or unspecific mouse IgG1 to anti-id BR55-2 #E4 (ELISA)**

100 µl aliquots of anti-id BR55-2 #E4 (10 µg/ml; dilution in coating buffer) are added to the wells of microtiter plates and incubated at 37°C for 60 minutes. The plates are washed 6 times with washing buffer, 200 µl of PBS def./ 5% FCS are added and incubated for 30 minutes at 37°C. The plates are washed as described above. Monkey sera or immunopurified monkey Ig are diluted in PBS def./2% FCS. 100 µl aliquots of these samples are added to the wells of the microtiter plates and incubated for 60 minutes at 37°C. Unbound antibody is washed out as described above and 100 µl aliquots of peroxidase-conjugated antibody (goat-anti-human Ig/peroxidase such as the reagents of Chemicon & Co., 1:1000 in PBS/2% FCS) are added. After incubation for 30 minutes at 37°C the plates are washed 4 times with washing buffer and twice with staining buffer. 100 µl aliquots of substrate solution are added and color development is stopped after 5 minutes with 50 µl aliquots of 4N $H_2SO_4$. Optical density (OD) is measured at 492 nm (reference measurement 620 nm).

**Example 14: Antibody dependent cellular cytotoxicity (ADCC) using human PBMC to SKBR5 cells mediated by immunopurified monkey Ig (AB3) or BR55-2/murine IgG3**

On the day preceding the assay the SKBR5 cells are transferred into fresh medium A and kept at 37°C/5% $CO_2$ in a cell culture flask.

$^{51}Cr$ labeling of the target cells:

The cells are collected from the culture flask and incubated at a concentration of $5x10^6$ cells in 800 µl of medium A at 37°C/5% $CO_2$ for 1 hour with 100 µCi $Na_251CrO_4$. The cells are then washed with medium A to remove the excess $^{51}Cr$, resuspended in fresh medium A, and their concentration is adjusted to $2.5x10^5$ cells/ml.

Isolation of PBMC:

50 ml of heparinized fresh human blood are diluted with 60 ml of PBS complete containing 0.1% glucose. 15 ml aliquots of this solution are layered on top of 15 ml of Ficoll-Paque solution and the tubes are centrifuged at 400 g for 30 to 60 minutes. The plasma supernatants are discarded, the PBMC layers are collected and diluted to 50 ml with PBS complete + 0.1% glucose. After centrifugation at about 80 g (10 minutes), resuspension of the pellet in 25-30 ml PBS complete + 0.1% glucose, and recentrifugation (80 g, 10 minutes), the pellet is collected, suspended in medium A, the cells are counted and the suspension is diluted with medium A to about $2 \times 10^6$ to $9 \times 10^6$ cells/ml. 100 µl aliquots are pipetted into each well of a microtiter plate and the effector cells are incubated overnight at 37°C/5% $CO_2$.

ADCC:

100 µl of $^{51}Cr$-labeled target cells are added to the preincubated effector cells in the desired ratio of effector cells to target cells. 50 µl of immunopurified monkey Ig (AB3) or BR55-2/murine IgG3 diluted to the desired concentrations with PBS def. are added and the plate is incubated overnight (about 18 hours) at 37°C/5% $CO_2$. The supernatants are then harvested with a Skatron-Harvesting Press and counted in a γ-counter. This yields the value for the experimental release. Total $^{51}Cr$ release is determined as above by replacing PBMC with 100 µl of 2% SDS, 50 mM $Na_2CO_3$ and 10 mM EDTA and by replacing the antibody solution with 50 µl of PBS def. Spontaneous $^{51}Cr$ release is obtained by replacing PBMC with 100 µl of medium A and the antibody solution with 50 µl of PBS def.

After counting the results are calculated as follows:

$$\%\text{lysis} = \frac{\text{experimental release - spontaneous release}}{\text{total release - spontaneous release}} \times 100$$

**Example 15: Binding of rhesus monkey sera, immunopurified rhesus monkey Ig or BR55-2/murine IgG3 to HIV-infected/non-infected PALL-cells(indirect immunofluorescence, IFA-Anti-HIV1-Kit Waldheim)**

In a commercially available immunofluorescence assay kit (Waldheim & Co; purchased for determination of HIV seropositivity) HIV-infected and non-infected PALL cells (human T cell line) are fixed on slides. The experimental procedure in principle is based on the guidelines of the supplier. After incubation with 1% BSA for 30 minutes at 37°C the slides are incubated with rhesus monkey sera (concentrated and 1:10 diluted in PBS def.) or immunopurified rhesus monkey Ig (AB3) diluted In normal human serum or BR55-2/murine IgG3 for 1 hour at 37°C. HIV positive human serum (delivered as part of the test kit) serves as positive control, normal human serum as negative control. Unbound determinants of the test samples are washed out thrice with PBS def. and a 1:20 dilution of the anti-human-IgG-FITC reagent (part of the test kit) in PBS def. containing 2% FCS or anti-murine-IgG-FITC (for detection of the murine Mab) is added. After incubation for 30 minutes at 37°C, washing thrice with PBS def. and embedding of the slides the immunofluorescence is observed in a fluorescence microscope and scored (0 = no fluorescence, 5 = maximum fluorescence).

**Example 16: ELISA for the determination of immunoreactive BR55-2/murine IgG3 or BR55-2/murine IgG2a in human serum using anti-id BR55-2 #E4**

100 µl aliquots of anti-id BR55-2 #E4 (10 µg/ml; dilution in coating buffer) are added to the wells of microtiter plates and incubated at 37°C for 60 minutes.

The plates are washed 6 times with washing buffer, 200 µl of PBS def./5% FCS are added and Incubated for 30 minutes at 37°C. The plates are washed as described above. Human sera containing BR55-2/murine IgG3 or BR55-2/murine IgG2a are tested in appropriate dilutions in PBS def./2% FCS. 100 µl aliquots of these samples are added to the wells of the microtiter plates and incubated for 60 minutes at 37°C. As standard BR55-2/murine IgG3 or BR55-2/murine IgG2a is prediluted in normal human serum to 10 µg/ml. Appropriate dilutions in PBS def./2% FCS are treated as above. Unbound antibody is washed out as described above and 100 µl aliquots of peroxidase-conjugated antibody (rabbit-anti-mouse IgG3/peroxidase or rabbit-anti-mouse IgG2a/peroxidase such as the reagents of Zymed, 1:1000 in PBS/2% FCS) are added. After incubation for 30 minutes at 37°C the plates are washed 4 times with washing buffer and twice with staining buffer.

100 µl aliquots of substrate solution are added and color development is stopped after 5 minutes with 50 µl aliquots of 4N $H_2SO_4$. Optical density (OD) is measured at 492 nm (reference measurement 620 nm).

The OD values of the serum samples are read on the standard curve and expressed in µg/ml.

**Example 17: ELISA for the determination of immunoreactive BR55-2/chimeric human IgG1 in human serum using anti-id BR55-2 #E4**

Human sera containing BR55-2/chimeric human IgG1 are tested as described in example 16 using peroxidase-conjugated antibody (goat-anti-human IgG/peroxidase such as the reagents of Chemicon & Co., 1:1000 In PBS/ 2% FCS).

**Example 18: ELISA for the determination of immunoreactive BR55-2/chimeric human IgG3 in human serum using anti-id BR55-2 #E4**

Human sera containing BR55-2/chimeric human IgG3 are tested as described in example 17.

**Example 19: Real-time biospecific interaction analysis of BR55-2/chimeric human IgG1 and BR55-2/humanized IgG1 with anti-id BR55-2 Mabs**

Experiments were performed with a BIAcore™ system from Pharmacia Biosensor AB, Uppsala, Sweden. Binding of BR55-2/ chimeric human IgG1 or BR55-2/humanized IgG1 to the various murine IgG1 anti-id BR55-2 Mabs catched to immobilized RAM-IgG1 (such as the reagent from Pharmacia Biosensor AB, Uppsala, Sweden) was determined using real-time biospecific interaction analysis.

The flow rate of the system was set to 5 µl/minute. A sensor chip was activated with a mixture of 0.201 mg NHS and 1.313 mg EDC dissolved in 35 µl destilled water. RAM-IgG1 dissolved in 35 µl of 10 mM sodium acetate buffer pH 5.0 at a concentration of 30 µg/ml was then reacted with the activated sensor surface. Remaining free activated sites were blocked with 35 µl of 1 M ethanolamine hydrochloride/NaOH, pH 8.5.

The analysis was performed in three steps:

1) The respective anti-id BR55-2 Mab (E4, C11, B3, B9, G6, G9) was diluted with medium H to a final concentration of 10 to 17 µg/ml. For each analysis the anti-idiotypic antibody was bound to the RAM-IgG1 by passing 35 µl of this solution over the sensor surface. The "Response Units" which are proportional to the mass of the bound anti-id BR55-2 Mab were recorded and stored.

2) 20 µl of dilutions of the mouse/human chimeric IgG1 and the humanized IgG1 in medium H at concentrations of 1 - 100 µg/ml were then injected and allowed to bind to the catched anti-id. The "Response Units" which are proportional to the mass of bound Mab were recorded and stored.

3) The sensor surface was then regenerated for the next analysis with subsequent injections of 5 µl 1M formic acid and 5 µl 8M urea.

The binding rate was determined by calculating the ratio of the maximum Response Units obtained with the second antibody (BR55-2/ chimeric human IgG1 or BR55-2/humanized IgG1) and the maximum Response Units obtained with the first antibody (respective anti-idiotypic Mab).

**Example 20: ELISA for the determination of immunoreactive BR55-2/humanized IgG1 in human serum using anti-id BR55-2 #E4**

Human sera containing BR55-2/humanized IgG1 are tested as described in example 17.

**Example 21: Competition of binding of BR55-2/murine IgG3 to anti-id BR55-2 #E4 by BR55-2/chimeric human IgG1 or BR55-2/chimeric human IgG3**

100 µl aliquots of anti-id BR55-2 #E4 (10 µg/ml; dilution in coating buffer) are added to the wells of microtiter plates and incubated at 37°C for 60 minutes.

The plates are washed 6 times with washing buffer, 200 µl of PBS def./5% FCS are added and Incubated for 30 minutes at 37°C. The plates are washed as described above. Chimeric human IgG1 or chimeric human IgG3 is diluted in PBS def./2% FCS (0.5 µg/ml to 3 ng/ml). To each dilution, 0.05 µg/ml BR55-2/murine IgG3 are added. 100 µl aliquots of these mixture are added to the wells of the microtiterplates and incubated for 60 minutes at 37°C. Unbound antibody is washed out as described above and 100 µl aliquots of peroxidase-conjugated antibody (goat-anti-human IgG/peroxidase such as the reagents of Chemicon Co.; 1:1000 in PBS/2% FCS) are added.

After incubation for 30 minutes at 37°C the plates are washed 4 times with washing buffer and twice with staining buffer.

100 µl aliquots of substrate solution are added and color development is stopped after 5 minutes with 50 µl aliquots of 4N $H_2SO_4$. Optical density (OD) is measured at 492 nm (reference measurement 620 nm).

**Example 22: Complement dependent cytotoxicity (CDC) to SKBR5 cell line mediated by BR55-2/murine IgG3 - inhibition by anti-id BR55-2 #E4**

On the day preceding the assay the SKBR5 cells are transferred into fresh medium A and kept at 37°C/5% $CO_2$ in a cell culture flask.

$^{51}$Cr labelling of the target cells:

The cells are collected from the culture flask and Incubated at a concentration of 5x$10^6$ cells in 800 µl of medium A at 37°C/5% $CO_2$ for 1 hour with 100 µCi $Na_2 51CrO_4$. The cells are then washed with medium A to remove the excess $^{51}$Cr, resuspended in fresh medium A, and their concentration is adjusted to 2.5x$10^5$ cells/ml.

CDC:

100 µl aliquots of this suspension of target celts are pipetted into the wells of microtiter plates. 50 µl aliquots of anti-id BR55-2 #E4 diluted to the desired concentrations in PBS def. are added. Then 100 µl aliquots of a human serum containing 2 µg/ml BR55-2/IgG3 are added per well and the cells are incubated overnight at 37°C/5% $CO_2$. The supernatants are harvested with a Skatron-Harvesting-Press and counted in a γ-counter. This yields the value for the experimental release.

For determination of total $^{51}$Cr release the cells are treated as above replacing the human serum by a solution of 2% SDS, 50 mM $Na_2CO_3$ and 10 mM EDTA and the anti-id BR55-2 #E4 solution by 50 µl PBS def. The value for spontaneous $^{51}$Cr release is obtained by replacing the human serum by medium A and the anti-id BR55-2 #E4 solution by 50 µl PBS def.

After counting the result is calculated as follows:

$$\%\text{lysis} = \frac{\text{experimental release - spontaneous release}}{\text{total release - spontaneous release}} \times 100$$

**Example 23: Immunoaffinity purification of BR55-2/chimeric human IgG1 using anti-id BR55-2 #E4-Sepharose**

**23.1: Preparation of anti-id BR55-2 #E4-Sepharose**

9 g freeze dried activated CH-Sepharose 4B is suspended in 1mM HCl, transfered to a sinter glass filter and washed with 2 l 1 mM HCl for 15 minutes. The ligand (95 mg anti-id BR55-2 #E4) dissolved in 50 ml coupling buffer is mixed with the washed gel in a stoppered vessel and rotated end over end for one hour at room temperature. The gel is washed with coupling buffer and incubated for one hour with 50 ml 1M ethanolamine for blocking of any remaining active groups. The affinity sorbent is then washed with three cycles of alternating pH. Each cycle consists of a wash at pH 4 (0.1 M acetate, 0.5 M NaCl) followed by a wash at pH 8 (0.1 M Tris, 0.5 M NaCl).

**23.2: Isolation of the BR55-2/chimeric human IgG1**

The chromatography is performed at 4°C. The column (BIO REX MP column diameter 1.5 cm) is filled with anti-id BR55-2 #E4-Sepharose (volume 35 ml). The gel is washed with binding buffer and elution buffer. Starting material are 100 l of BR55-2/chimeric human IgG1 conditioned medium. After concentration 5:1 using an Amicon DC2 concentration system equipped with a hollow fiber cartridge P 10, the concentrate was loaded onto the column. After elution of the breakthrough fraction, the bound BR55-2/chimeric human IgG1 is desorbed with elution buffer and neutralized immediately after desorption with 1 M Tris/HCl buffer pH 7.5.

**23.3: Concentration of the BR55-2/chimeric human IgG1**

Concentration of the eluted antibody solution is performed in a stirred Amicon ultrafiltration cell using a PM 10 Diaflo membrane. The solute rejection for IgG is more than 98%, the final concentration of IgG amounts to 3.36 mg/ml.

**23.4: Characterization of purified BR55-2/chimeric human IgG1**

**23.4.1: Ion-exchange-chromatography on Mono-Q**

| | |
|---|---|
| Column | Mono-Q HR5/5 (Pharmacia) |
| Buffer A | 20 mM TEA, pH 7.7 |
| Buffer B | 20 mM TEA, 1 M NaCl, pH 7.7 |
| Flow rate | 1 ml/min |
| Detection | UV 280 nm |
| Gradient | linear 2%/min |
| Result | >95% purity |

**23.4.2: High performance size-exclusion-chromatography**

| | |
|---|---|
| Column | Zorbax GF250, 9.4 x 250 mm |
| Buffer | Sodium phosphate 0.1 M, 0.2 M NaCl, pH 7.0 |
| Flow rate | 1 ml/min |
| Detection | UV 280 nm |
| Result | >95% purity |

**23.4.3: SDS-PAGE**

Experiments are performed under reducing conditions according to the method of Laemmli using 10% acrylamide gels (results are shown in figure 10).

**STARTING MATERIAL**

Murine monoclonal antibodies BR55-2 are available from e.g. hybridomas BR55.2 (BR55-2/IgG3) and, respectively, BR55.2S2a (BR-55-2/IgG2a).
These hybridomas were originally deposited on February 17, 1987 and, respectively, March 10, 1987 with the American Type Culture Collection, Rockville, MD 20852, USA, under the provisions of the Budapest Treaty, under deposit numbers ATCC HB 9324 and, respectively, ATCC HB 9347.

**Claims**

1. Monoclonal murine internal image anti-idiotypic antibodies (Ab2) to monoclonal antibodies BR55-2 (ATCC HB 9324) (Ab1).

2. Process for the production of anti-idiotypic antibodies according to claim 1 which comprises immunizing mice with BR55-2/murine IgG3-F $(ab')_2$-KLH-conjugate, fusing the murine spleen cells with the murine myeloma cell line SP 2/0, selecting the cultured hybridoma cells which produce IgG with an inhibition capacity of more than 95% (inhibition of binding of BR55-2 murine IgG2a to the SKBR5 cell line), purifying and isolating the anti-idiotypic antibody.

3. An anti-idiotypic antibody according to claim 1 for use in the preparation of a medicament for prophylactic and/or therapeutic immunization against HIV-infections.

**4.** The antibody according to claim 1 which is anti-id BR55-2 #E4.

**5.** A pharmaceutical composition which comprises as active agent an anti-idiotypic antibody according to claim 1 together with a pharmaceutically acceptable adjuvant, carrier or diluent for use in the prophylactic and/or therapeutic immunization against HIV-infections.

**6.** An anti-idiotypic antibody according to claim 1 for use in the preparation of a medicament for immunization against cancer of epithelial origin and against small cell lung cancer.

**7.** A pharmaceutical composition which comprises as active agent an anti-idiotypic antibody according to claim claim 1 together with a pharmaceutically acceptable adjuvant, carrier or diluent for use in immunization against cancer of epithelial origin and against small cell lung cancer.

**8.** Use of anti-idiotypic antibodies according claim 1 for the quantitative determination of monoclonal antibodies, their derivatives or fragments with binding specifity of BR55-2.

**9.** Use of anti-idiotypic antibodies according claim 1 for the quantitative determination of monoclonal mouse/human chimeras of BR55-2 and of fully humanized variants of BR55-2.

**10.** Use of anti-idiotypic antibodies according claim 1 for a single step immuno-purification of variants of BR55-2.

**Patentansprüche**

**1.** Monoklonale antiidiotypische, ein internes Abbild darstellende Antikörper der Maus (Ab2) gegen die monoklonalen Antikörper BR55-2 (ATCC HB9324) (Ab1).

**2.** Verfahren zur Herstellung von antiidiotypischen Antikörpern nach Anspruch 1, gekennzeichnet durch Immunisierung von Mäusen mit einem BR55-2/Maus-IgG3-F(ab')$_2$-KLH Konjugat, Fusion der Mausmilzzellen mit der Mausmyelomzellinie SP20, Auswahl der kultivierten Hybridomzellen, die IgG mit einer Hemmkapazität (Hemmung der Bindung des Maus IgG2a BR55-2 an die SKBRS Zellinie) von mehr als 95 % bilden, Reinigung und Isolierung des antiidiotypischen Antikörpers.

**3.** Antiidiotypischer Antikörper nach Anspruch 1 zur Verwendung bei der Herstellung eines Arzneimittels zur prophylaktischen und/oder therapeutischen Immunisierung gegen HIV Infektionen.

**4.** Antikörper nach Anspruch 1, der anti-id BR55-2 Nr. E4 ist.

**5.** Pharmazeutische Zusammensetzung, die als Wirkstoff einen antiidiotypischen Antikörper nach Anspruch 1 zusammen mit einem pharmazeutisch annehmbaren Adjuvans, Träger oder Verdünnungsmittel zur Verwendung bei der prophylaktischen und/oder therapeutischen Immunisierung gegen HIV-Infektionen enthält.

**6.** Antiidiotypischer Antikörper nach Anspruch 1 zur Verwendung bei der Herstellung eines Arzneimittels zur Immunisierung gegen Krebs epithelialen Ursprungs und gegen kleinzelligen Lungenkrebs.

**7.** Pharmazeutische Zusammensetzung, die als Wirkstoff einen antiidiotypischen Antikörper nach Anspruch 1 zusammen mit einem pharmazeutisch annehmbaren Adjuvans, Träger oder Verdünnungsmittel zur Verwendung bei der Immunisierung gegen Krebs epithelialen Ursprungs und gegen kleinzelligen Lungenkrebs enthält.

**8.** Verwendung von antiidiotypischen Antikörpern nach Anspruch 1 zur quantitativen Bestimmung von monoklonalen Antikörpern, ihren Derivaten oder Fragmenten mit der Bindungsspezifität von BR55-2.

**9.** Verwendung von antiidiotypischen Antikörpern nach Anspruch 1 zur quantitativen Bestimmung von monoklonalen Maus/Mensch-Chimären von BR55-2 und vollkommen humanisierten Varianten von BR55-2.

**10.** Verwendung von antiidiotypischen Antikörpern nach Anspruch 1 für eine Einschrittimmunreinigung von Varianten von BR55-2.

## Revendications

1. Anticorps monoclonaux anti-idiotype murins ayant des propriétés d'image interne (Ab2), dirigés contre des anticorps monoclonaux BR55-2 (ATCC HB 9324) (Ab1).

2. Procédé pour la production d'anticorps anti-idiotype selon la revendication 1, qui comprend l'immunisation des souris avec un conjugué $F(ab')_2$ de BR55-2/IgG3 mutine-hémocyanine de patelle, la fusion des cellules spléniques murines avec la lignée cellulaire de myélome murin SP 2/0, la sélection des cellules d'hybridome en culture qui produisent une IgG avec une capacité d'inhibition de plus de 95% (inhibition de la liaison de l'IgG2a murine de BR55-2 à la lignée cellulaire SKBR5), la purification et l'isolement de l'anticorps anti-idiotype.

3. Anticorps anti-idiotype selon la revendication 1, à utiliser dans la préparation d'un médicament destiné à l'immunisation prophylactique et/ou thérapeutique contre les infections par le VIH.

4. Anticorps selon la revendication 1, qui est un anti-BR55-2 id. #E4.

5. Composition pharmaceutique qui comprend, en tant qu'agent actif, un anticorps anti-idiotype selon la revendication 1, ainsi qu'un adjuvant, véhicule ou diluant pharmaceutiquement acceptable, à utiliser pour l'immunisation prophylactique et/ou thérapeutique contre des infections par le VIH.

6. Anticorps anti-idiotype selon la revendication 1, à utiliser dans la préparation d'un médicament destiné à l'immunisation contre le cancer d'origine épithéliale et contre le cancer du poumon à petites cellules.

7. Composition pharmaceutique qui comprend, en tant qu'agent actif, un anticorps anti-idiotype selon la revendication 1, ainsi qu'un adjuvant, véhicule ou diluant pharmaceutiquement acceptable, à utiliser pour l'immunisation contre le cancer d'origine épithéliale et contre le cancer du poumon à petites cellules.

8. Utilisation d'anticorps anti-idiotype selon la revendication 1 pour la détermination quantitative d'anticorps monoclonaux, de leurs dérivés ou de leurs fragments ayant une spécificité de liaison de BR55-2.

9. Utilisation d'anticorps anti-idiotype selon la revendication 1 pour la détermination quantitative de chimères monoclonales de souris/humaines de BR55-2 et de variants complètement humanisés de BR55-2.

10. Utilisation d'anticorps anti-idiotype selon la revendication 1 pour une immunopurification à une seule étape de variants de BR55-2.

Table 1

**Immunization of rabbits and rhesus monkeys with anti-id BR55-2 #E4 or unspecific mouse IgG1**

**Relative titer increase at week 9 compared to 1:4 diluted preserum (Cell ELISA)**

| | Rabbit | | Monkey | |
|---|---|---|---|---|
| | anti-id BR55-2 | mouse IgG1 | anti-id BR55-2 | mouse IgG1 |
| **SKBR5 (Y+)** | 1000 | 16 | 1000 | 8 |
| **WM9 (Y-)** | 3 | n.t. | 25 | n.t. |

Table 2

**Boost immunization of rhesus monkeys with anti-Id BR55-2 #E4 or unspecific mouse IgG1**

**Binding of monkey serum Ig to a SKBR5-membrane preparation (ELISA) Relative titer increase compared to 1:4 diluted preserum**

| | 9 weeks after first course | 2 years after first course | 1 week after first boost | 4 weeks after first boost |
|---|---|---|---|---|
| anti-Id BR55-2 | 40 | 1 | 80 | 50 |
| mIgG1 | 1 | 2 | n.t. | 2 |

Table 3

**Binding of rhesus monkey's serum Ig to HIV-infected/non-infected PALL-cell line (indirect Immunofluorescence, IFA-Anti-HIV1-Kit Waldheim)**

| | Immunization of rhesus monkeys with anti-Id BR55-2 #E4 | | unspecific mouse-IgG1 | |
|---|---|---|---|---|
| PALL: | HIV+ | HIV- | HIV+ | HIV- |
| **preserum** | | | | |
| conc. | 1 | 1 | 1 | 1 |
| 1:10 | 1 | 1 | 1 | 1 |
| **serum 9 weeks after first immunziation** | | | | |
| conc. | 3 | 1 | 1-2 | 1 |
| 1:10 | 3 | 1 | 1-2 | 1 |

| Human serum controls (Kit) | | |
|---|---|---|
| PALL: | HIV+ | HIV- |
| HIV-neg. human serum | | |
| | 0 | 0 |
| HIV-pos. human serum | | |
| | 5 | 0 |

0-5 = intensity of fluorescence (anti-human IgG-FITC)
0 = no fluorescence
5 = maximum fluorescence

Table 4

**Binding of rhesus monkey Ig immunopurified on anti-id BR55-2 #E4 (AB3) and BR55-2 / murine IgG3 (AB1) to HIV-infected/non-infected PALL-cell line (indirect immunofluorescence, IFA-Anti-HIV1-Kit Waldheim)**

| | AB3 (20 µg/ml) | | BR55-2 / murine IgG3 (20 µg/ml) | |
|---|---|---|---|---|
| PALL: | HIV+ | HIV- | HIV+ | HIV- |
| | 3 | 0 | 2 | 0 |

| Human serum controls (Kit) | | |
|---|---|---|
| PALL: | HIV+ | HIV- |
| HIV-neg. human serum | | |
| | 0 | 0 |
| HIV-pos. human serum | | |
| | 5 | 1 |

0-5 = Intensity of fluorescence (anti-human IgG-FITC)
0 = no fluorescence
5 = maximum fluorescence

Figure 1
SDS - PAGE

St = Standards

E4, C11, B3, B9, G6, G9 = anti-id BR55-2 Mabs

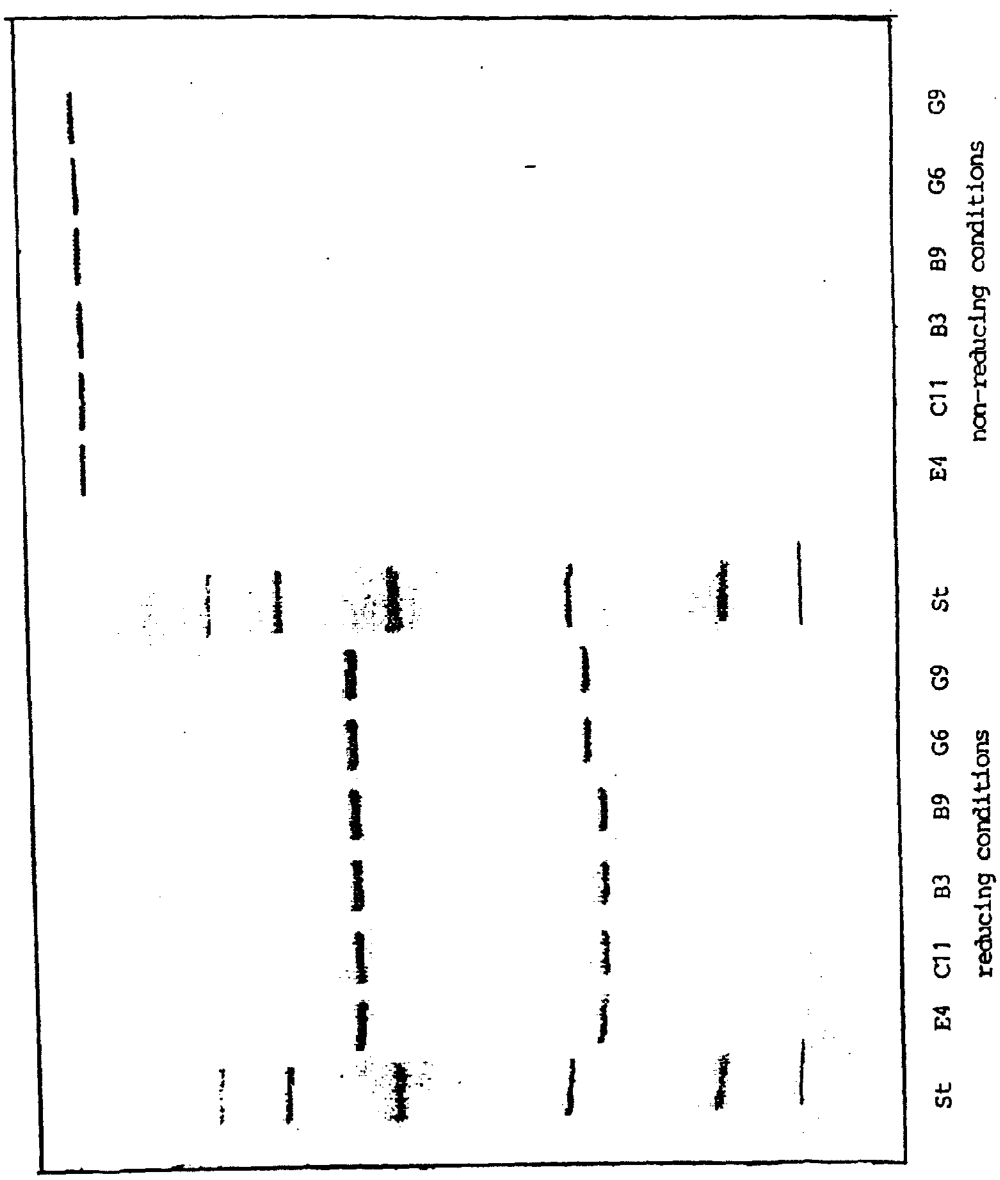

Figure 2
Isoelectric Focussing

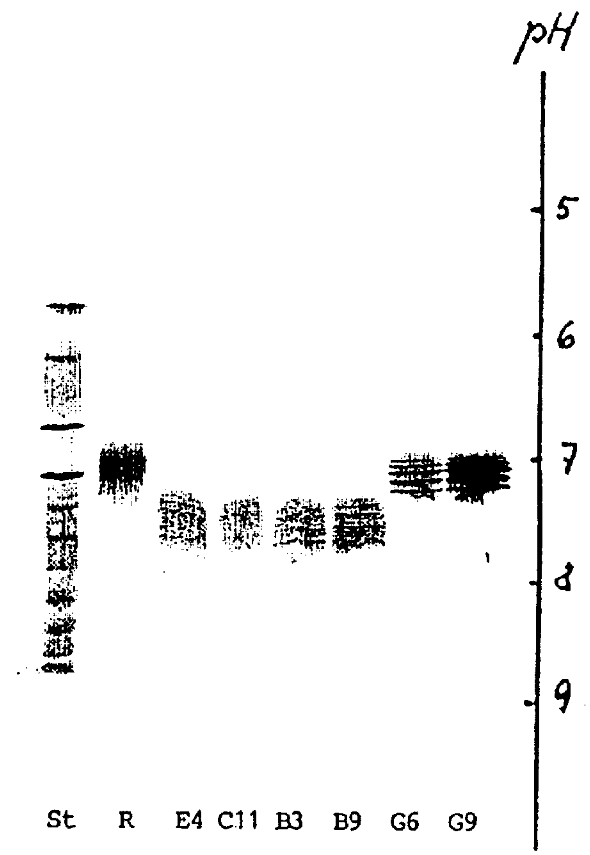


St = Standard.
R = Reference Mab
E4, C11, B3, B9, G6 and G9 = anti-id BR55-2 Mabs

**Figure 3**
**Binding of BR55-2/murine IgG3 to SKBR5 cell line**
**Inhibition by anti-Id BR55-2 #E4**

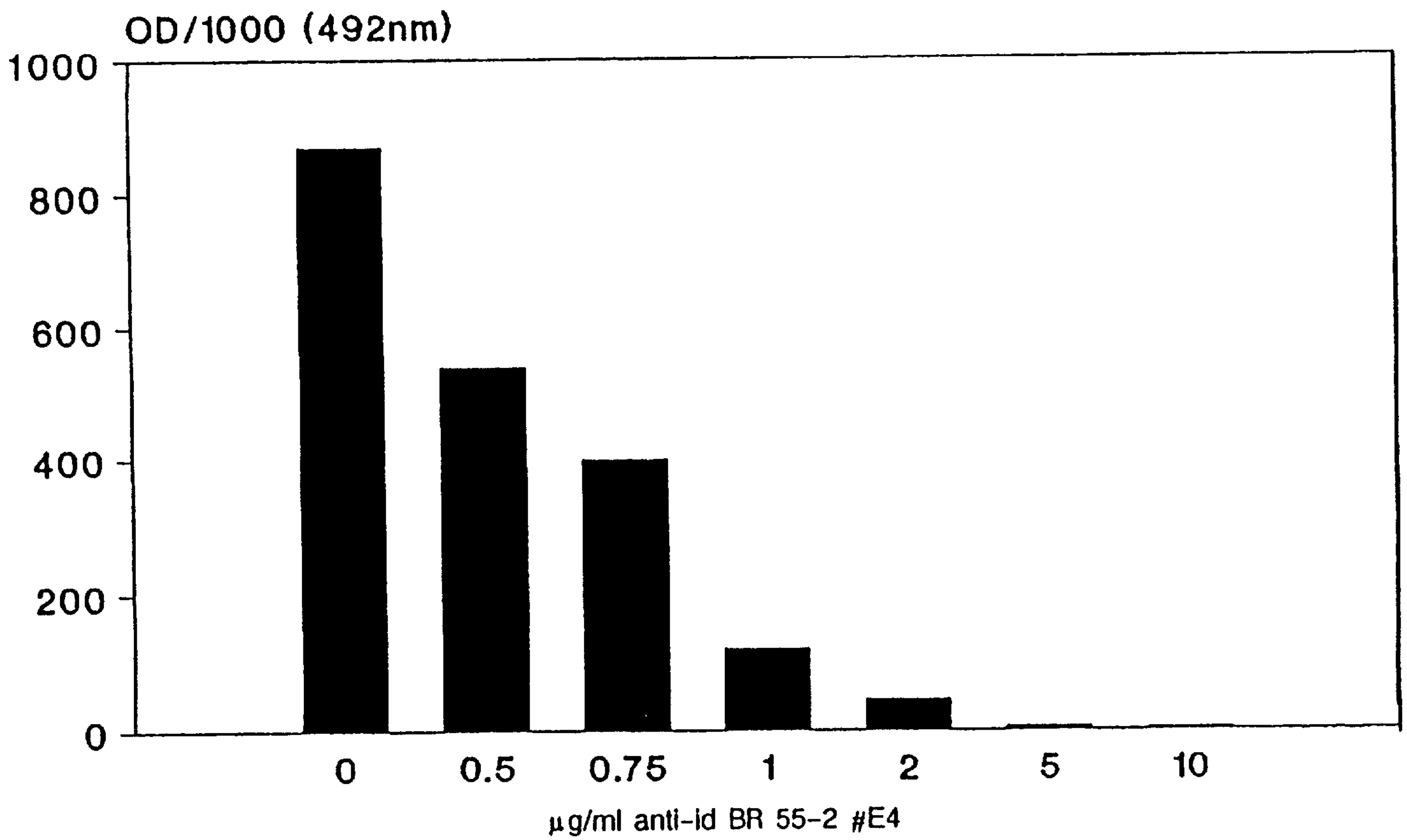


BR 55-2/murine IgG3: 1 μg/ml

Figure 4

Binding of rabbit Ig to SKBR5 before and after immunization with anti-Id BR55-2 #E4 (Cell-ELISA)

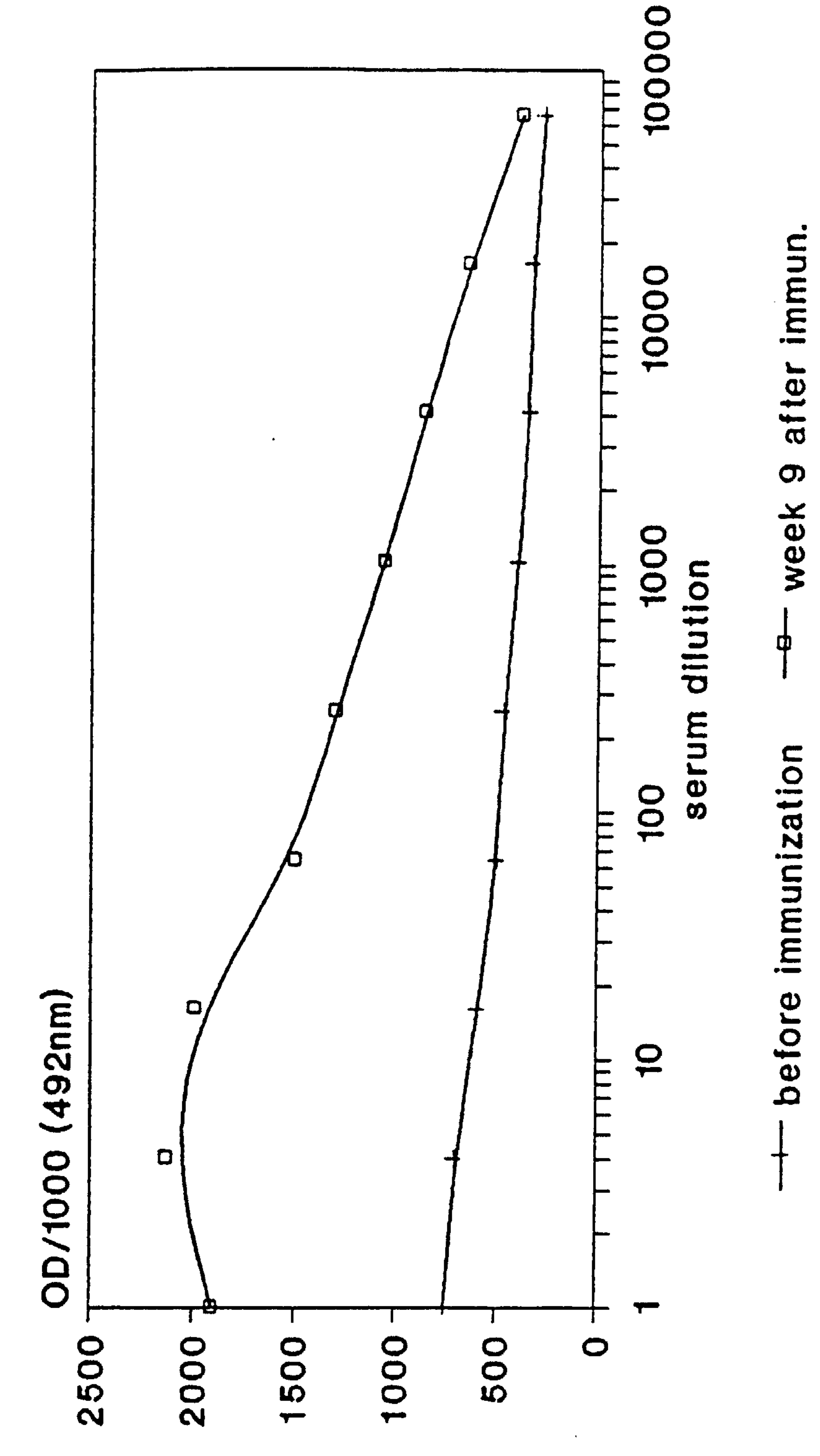

Figure 5
Binding of rhesus monkey Ig to SKBR5 before and after immunization with anti-id BR55-2 #E4 (Cell-ELISA)

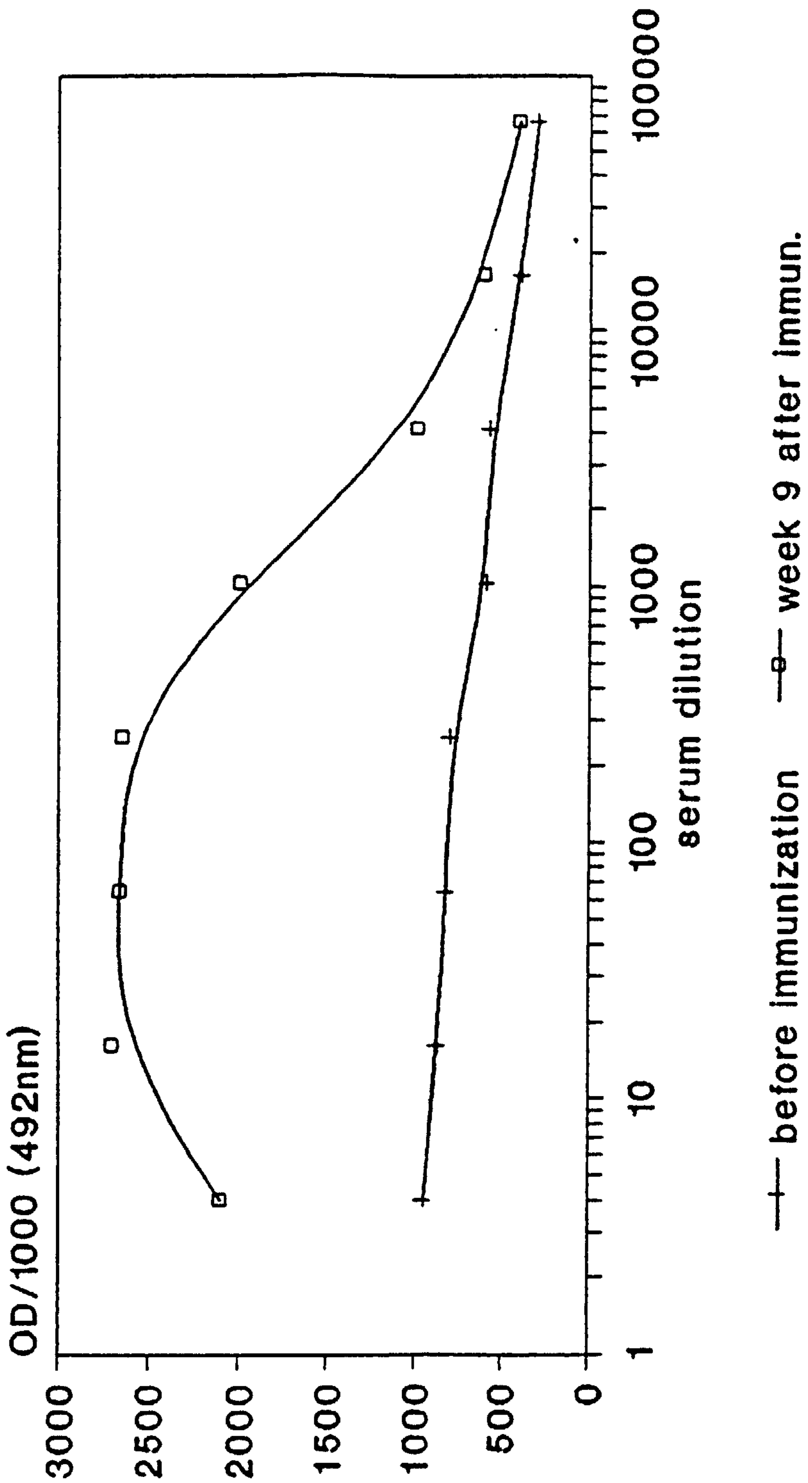

Figure 6

# Immunization of monkey 246 with anti-id BR55-2#E4 Serum Ig binding to anti-id BR55-2#E4 (ELISA)

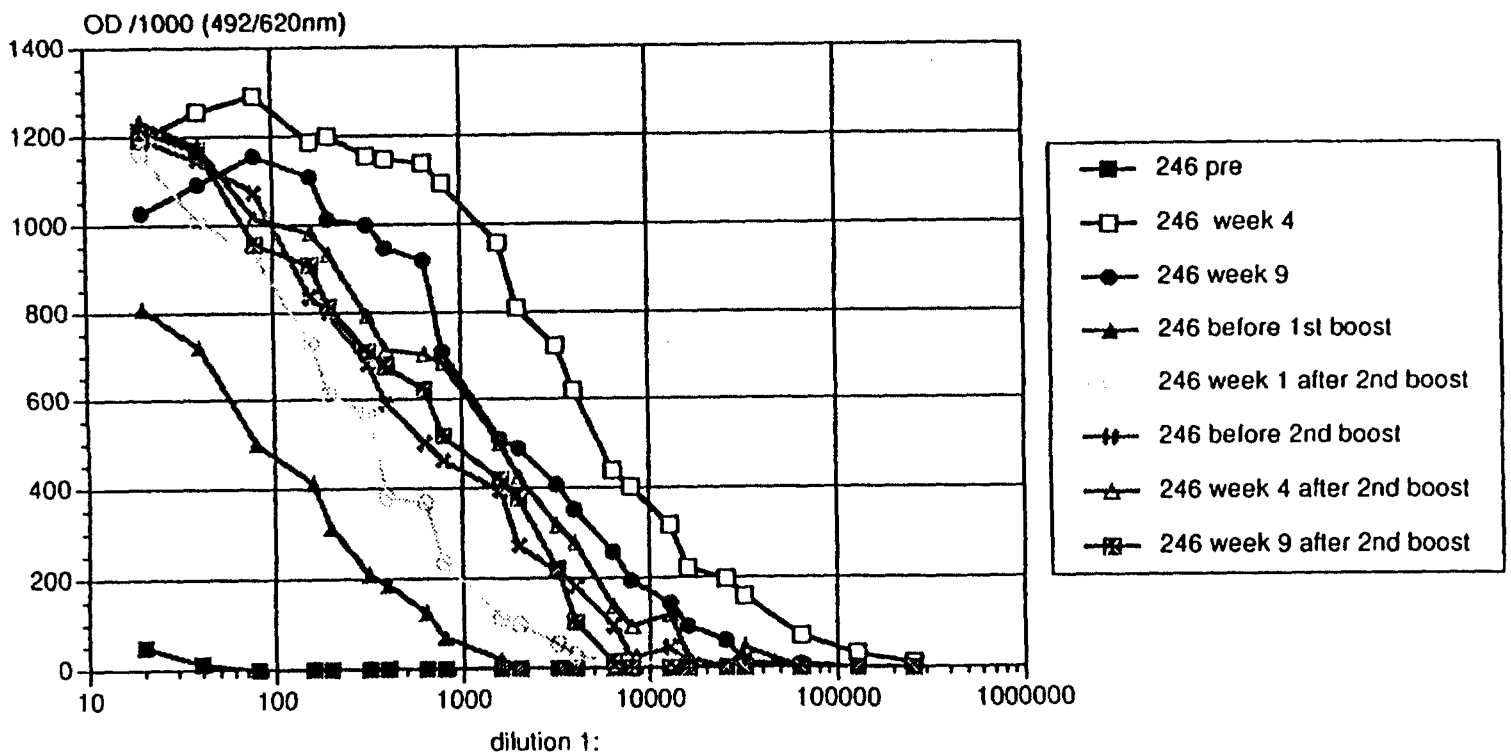

Figure 7

## Immunization of monkey 287 with anti-id BR55-2#E4
## Serum Ig binding to anti-id BR55-2#E4
## (ELISA)

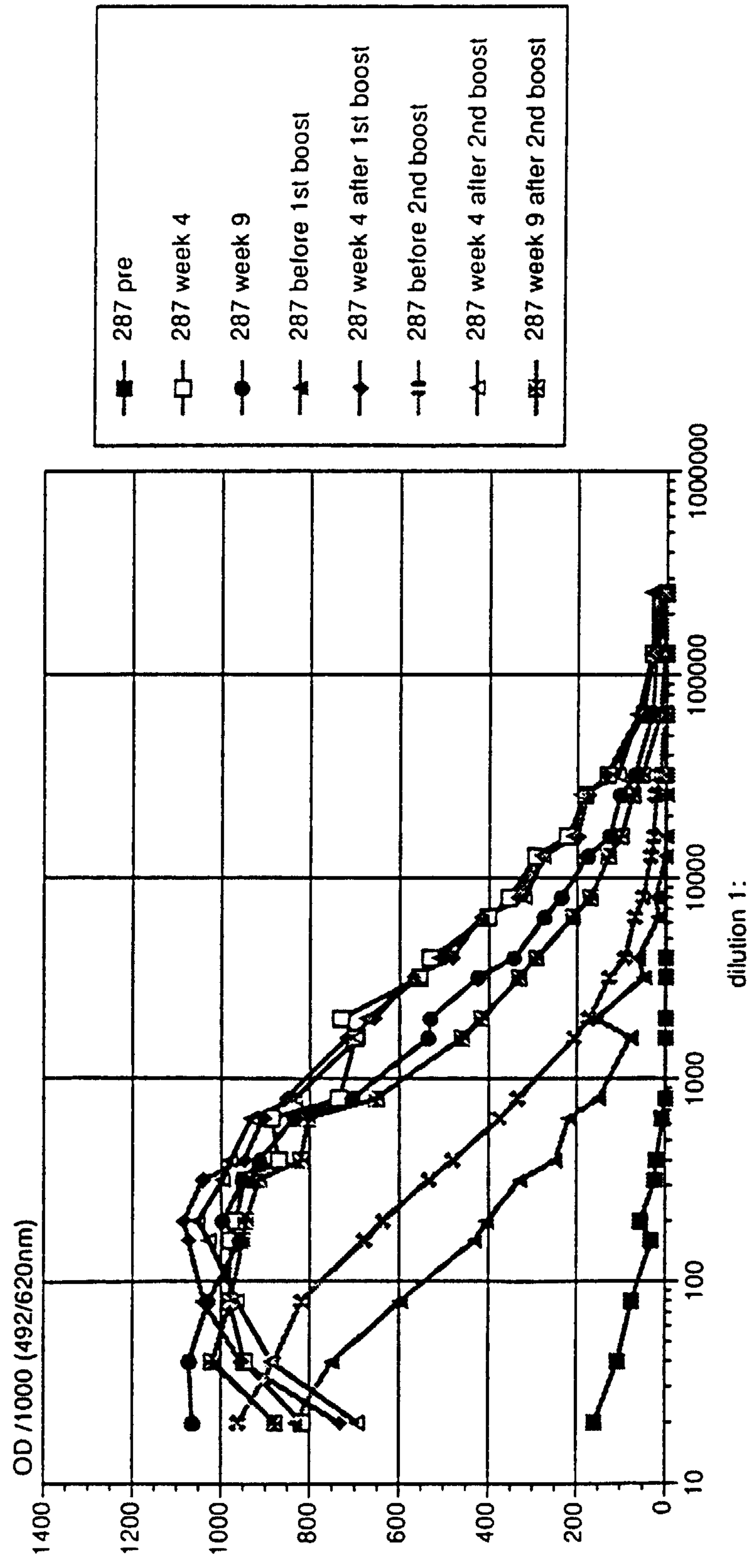

Figure 8

Immunization of monkey E23 with anti-id BR55-2#E4
Serum Ig binding to anti-id BR55-2#E4
(ELISA)

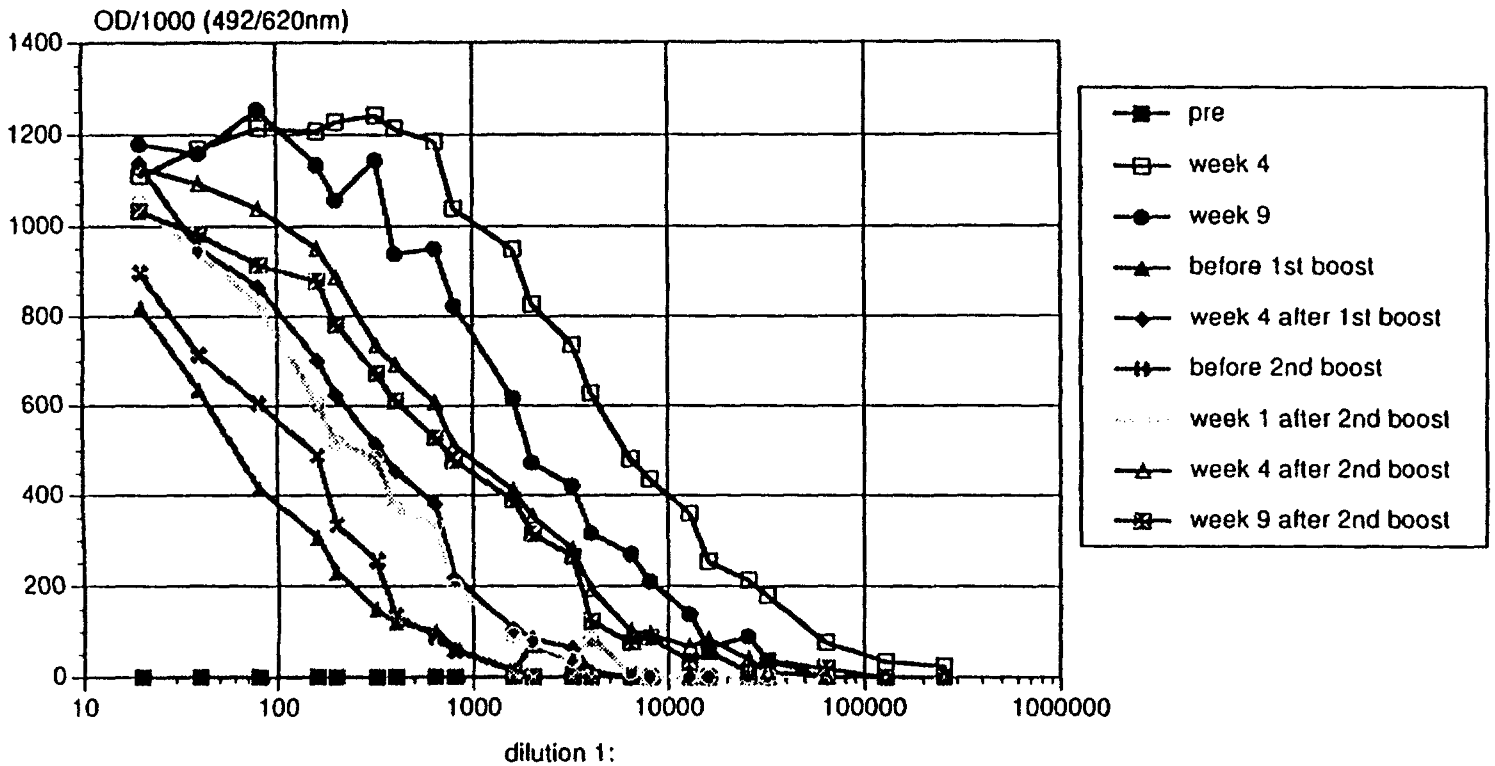

Figure 9

# Immunization of monkey 215 with irrelevant mouse IgG1
# Serum Ig binding to anti-id BR55-2#E4 (ELISA)

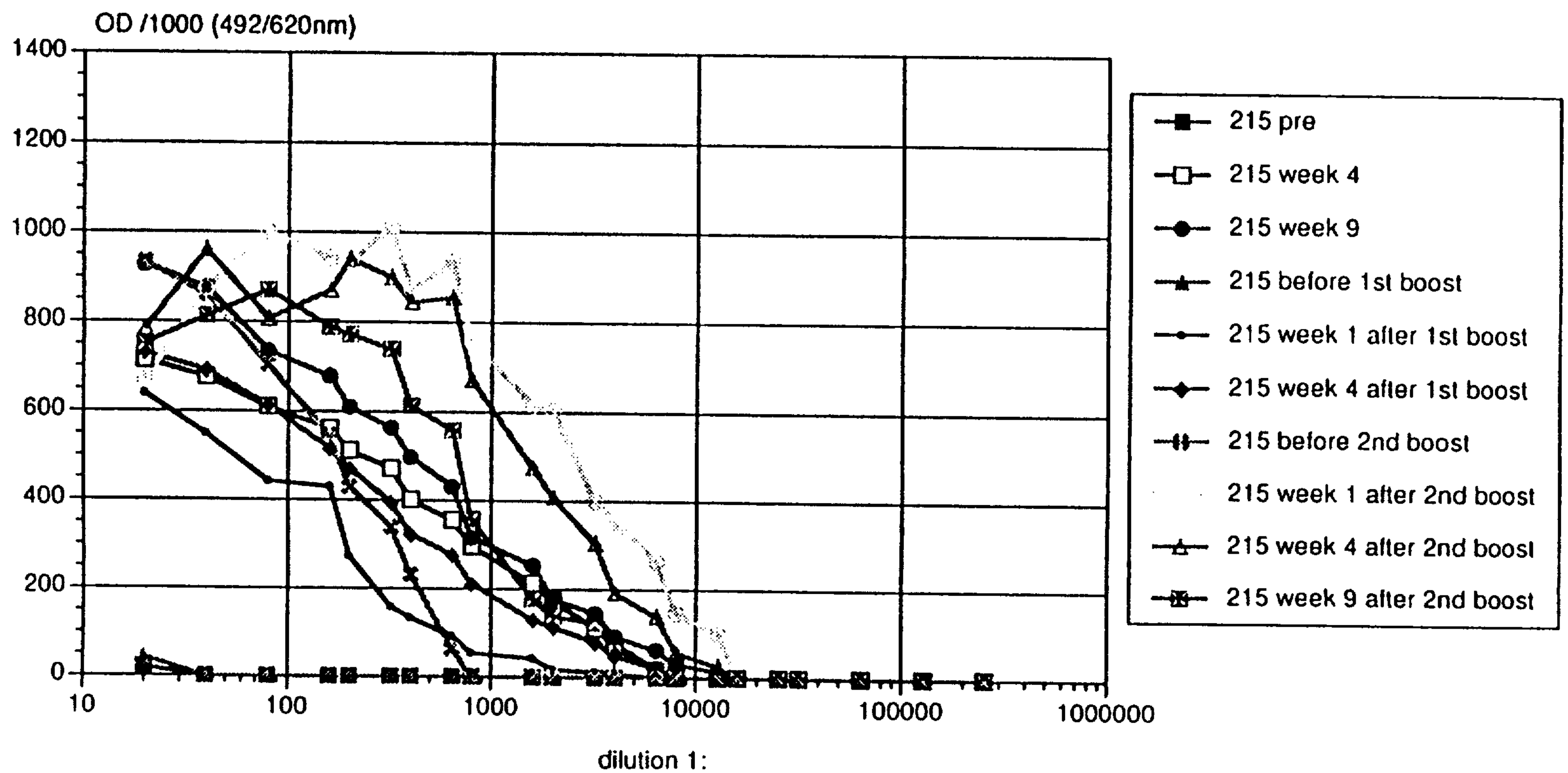

Figure 10

Immunization of monkey 252 with irrelevant mouse IgG1
Serum Ig binding to anti-id BR55-2#E4 (ELISA)

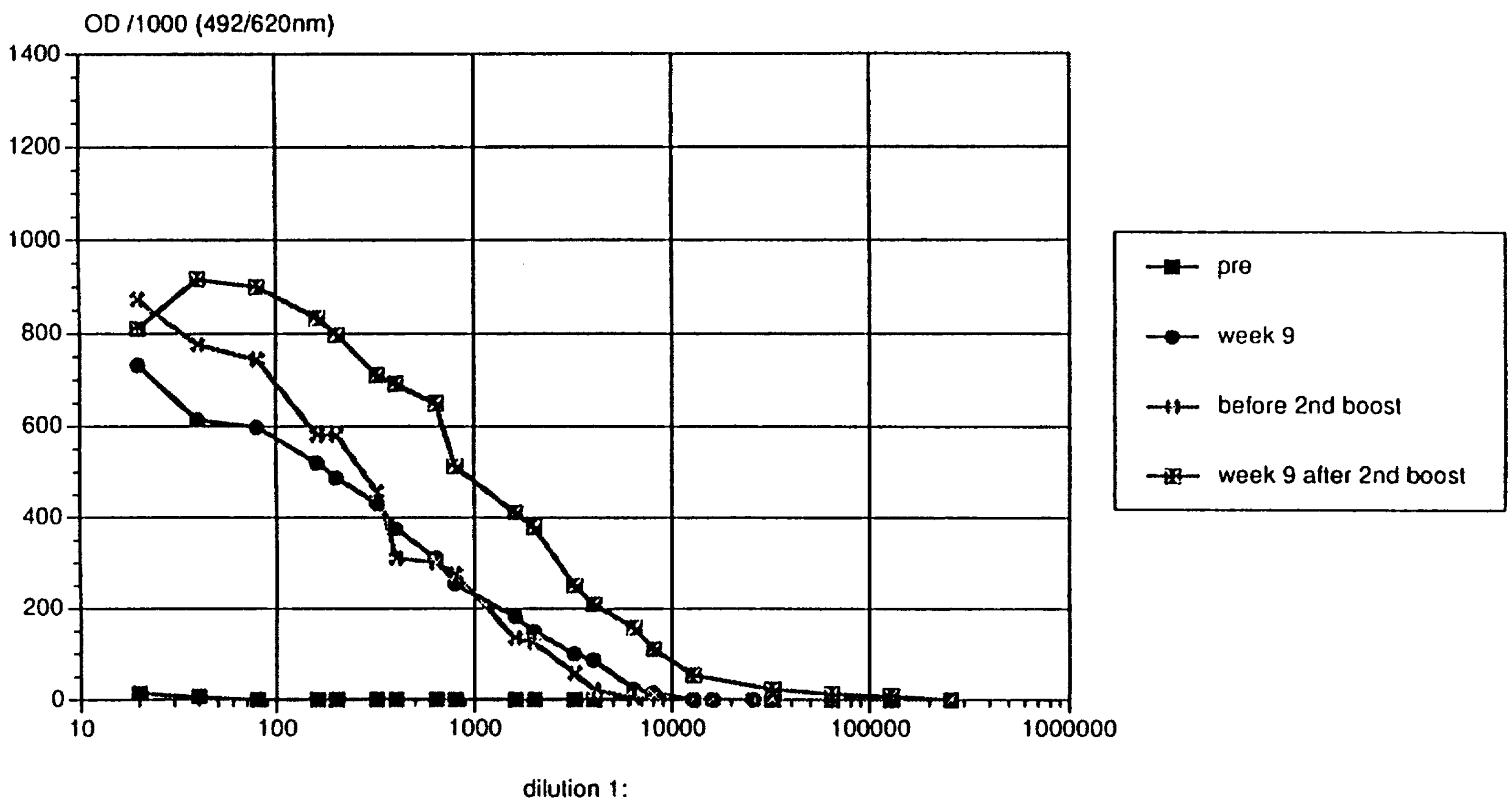

Figure 11

Immunization of monkey 246 with anti-id BR55-2#E4
Serum Ig binding to SKBR5 breast cancer cells
(cell- ELISA)

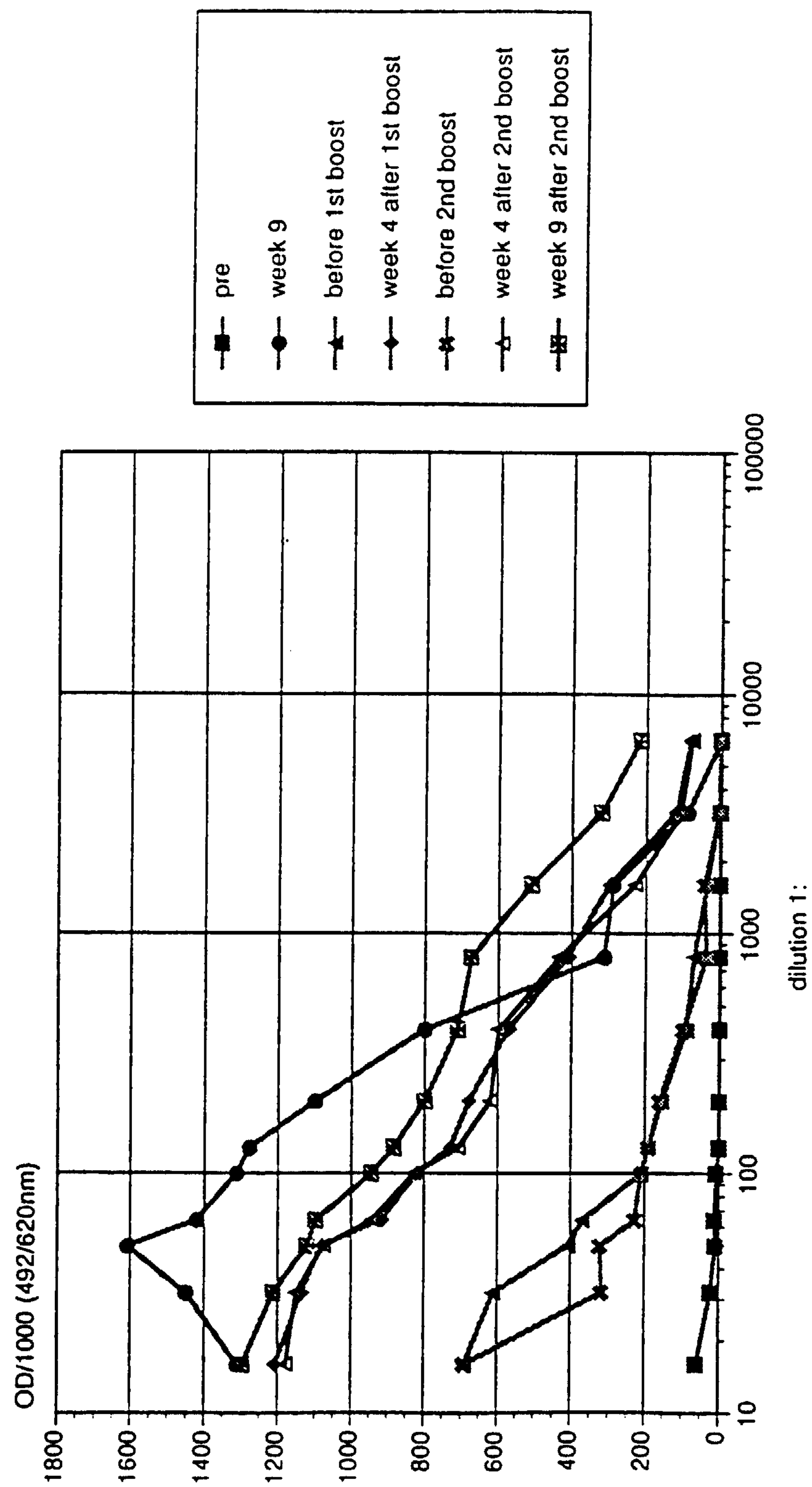

Figure 12

# Immunization of monkey 287 with anti-id BR55-2#E4 Serum Ig binding to SKBR5 breast cancer cells (cell- ELISA)

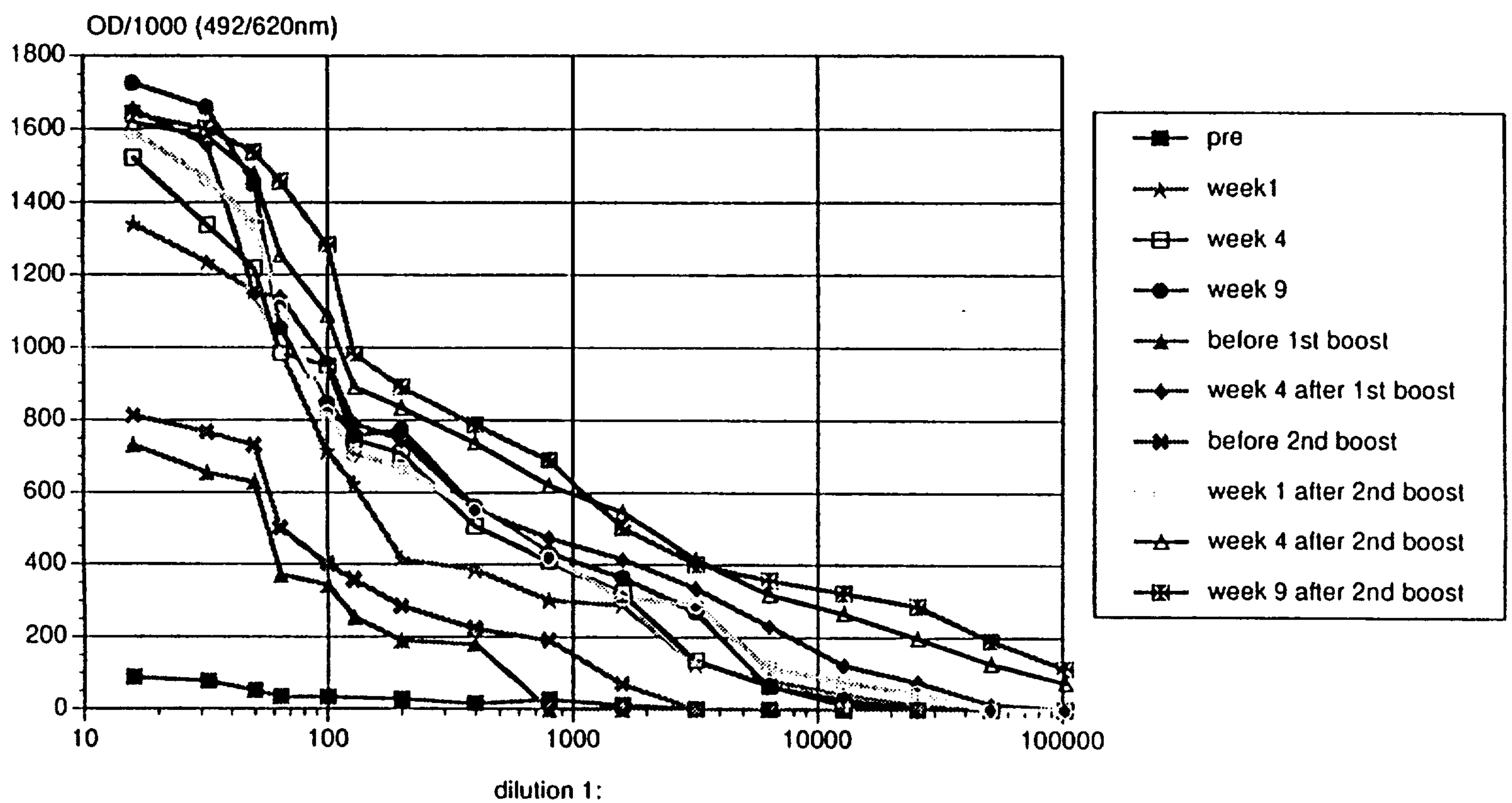

Figure 13

Immunization of monkey E23 with anti-id BR55-2#E4
Serum Ig binding to SKBR5 breast cancer cells
(cell- ELISA)

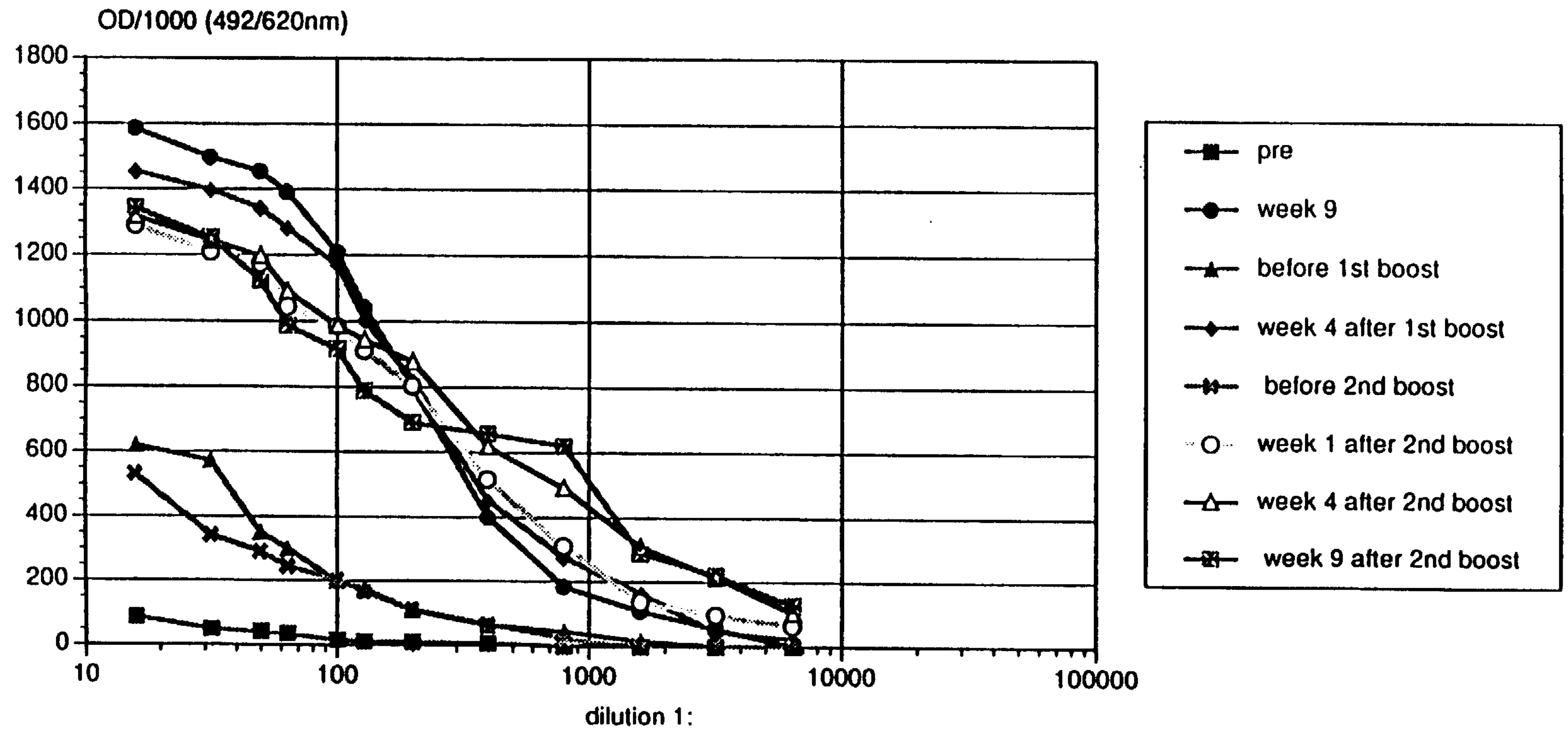

Figure 14

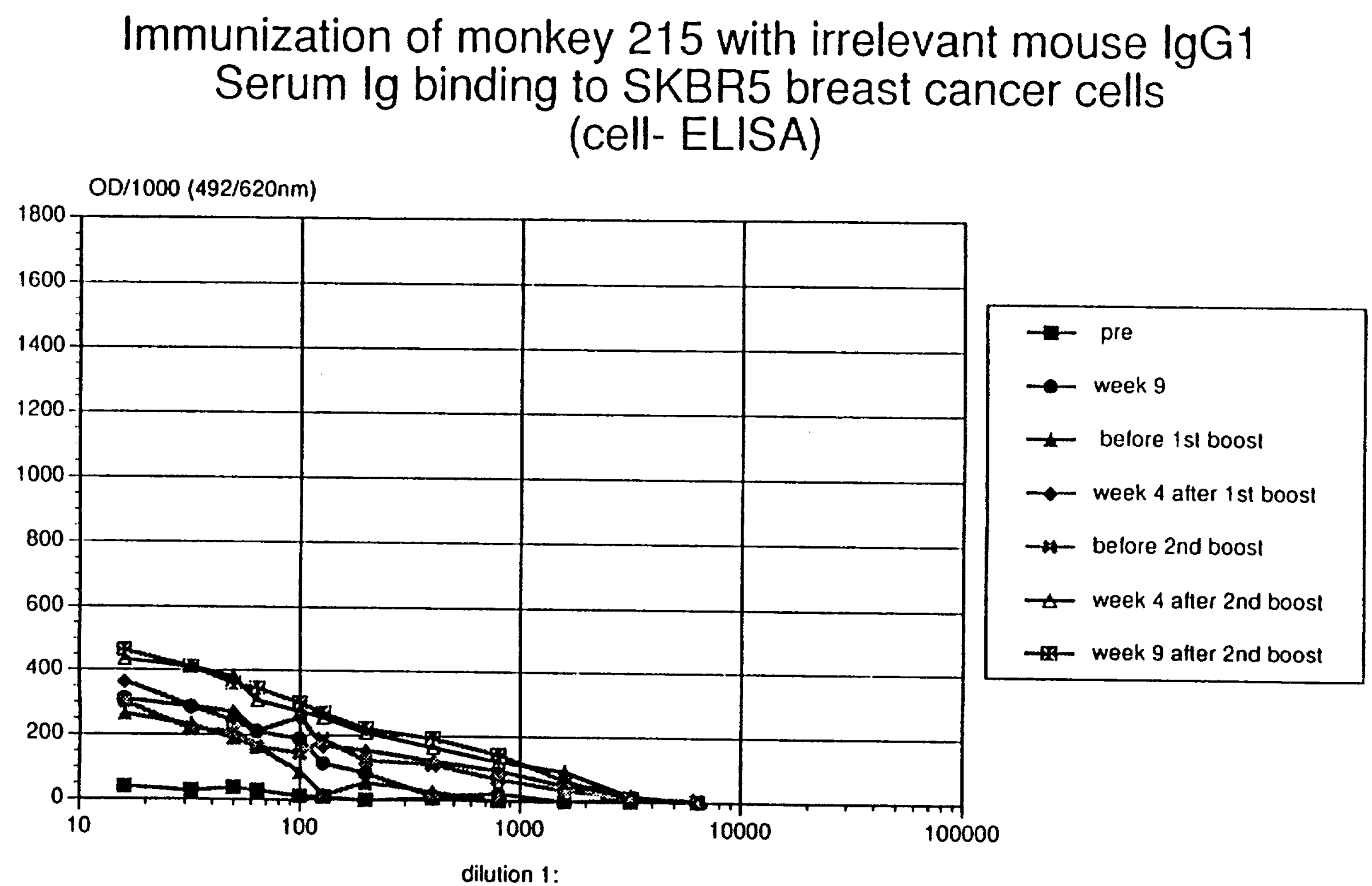
Immunization of monkey 215 with irrelevant mouse IgG1
Serum Ig binding to SKBR5 breast cancer cells
(cell- ELISA)
OD/1000 (492/620nm)
1800
1600
1400
1200
1000
800
600
400
200
0
10
100
1000
10000
100000
dilution 1:
pre
week 9
before 1st boost
week 4 after 1st boost
before 2nd boost
week 4 after 2nd boost
week 9 after 2nd boost

Figure 15

Immunization of monkey 246 with anti-id BR55-2#E4
Serum Ig binding to CATO gastric cancer cells
(cell- ELISA)

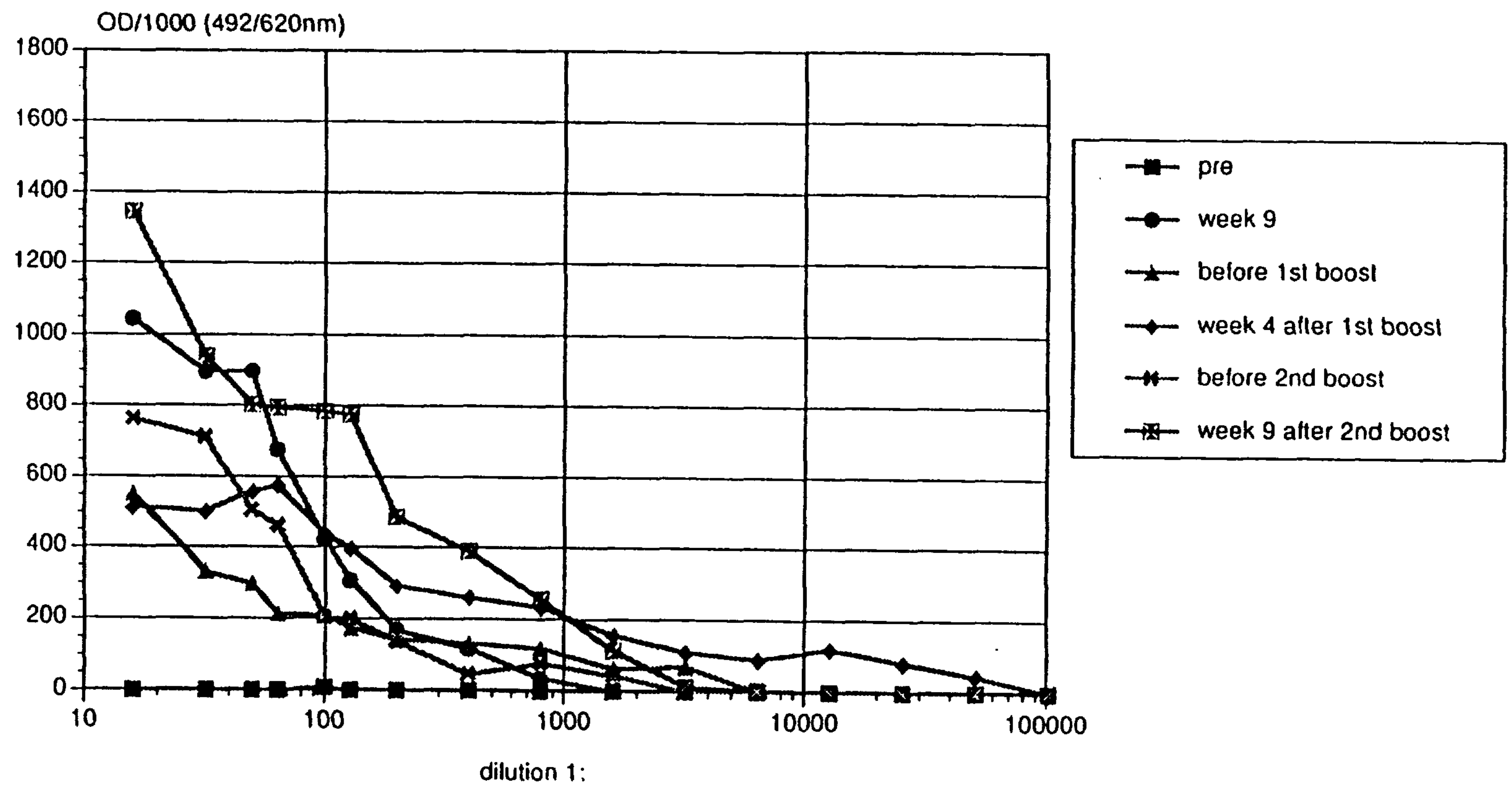

Figure 16

# Immunization of monkey 215 with irrelevant mouse IgG1 Serum Ig binding to CATO gastric cancer cells (cell-ELISA)

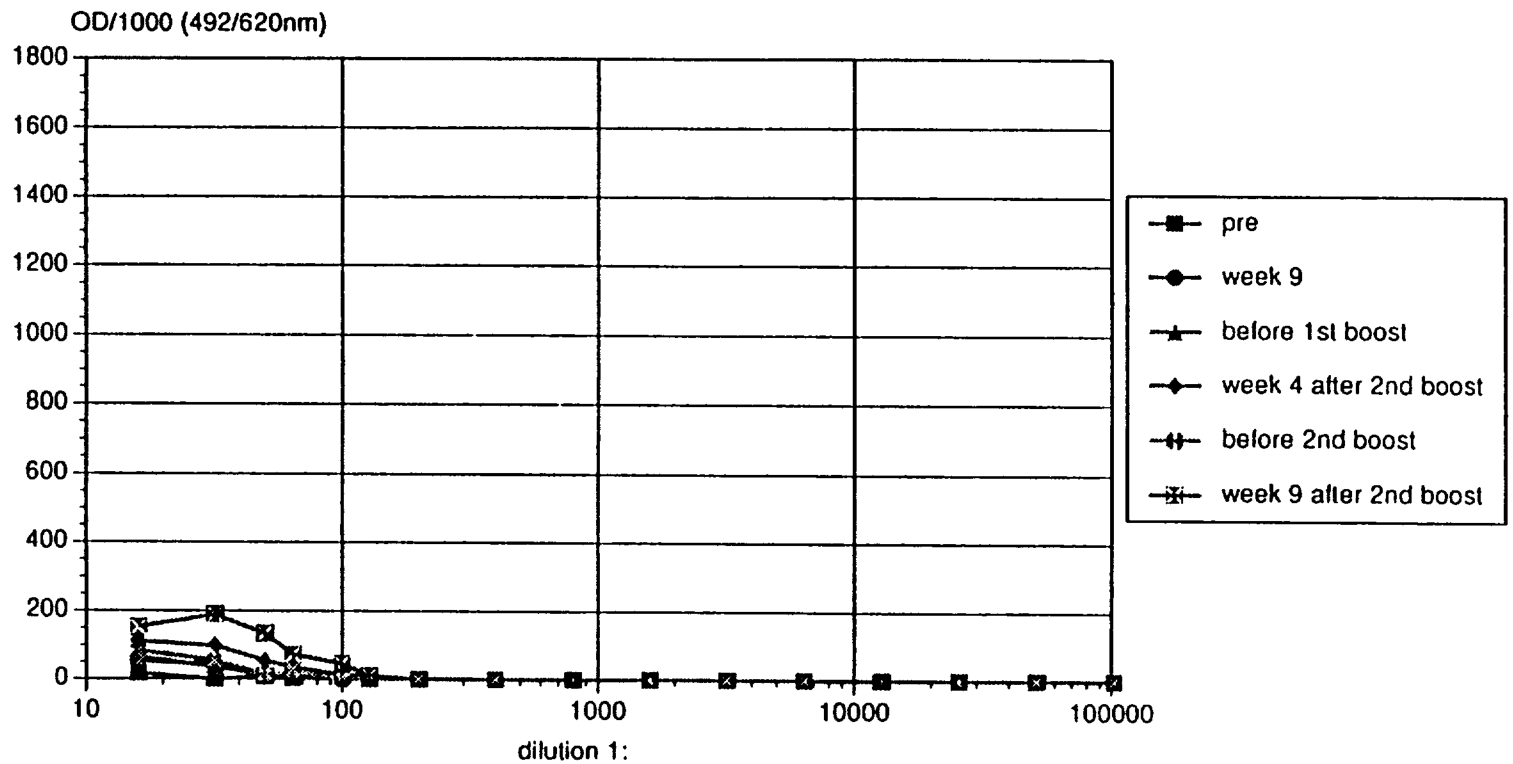

Figure 17

Immunization of monkey E 23 with anti-id BR55-2#E4
Serum Ig binding to SW2 small cell lung cancer cells
(cell- ELISA)

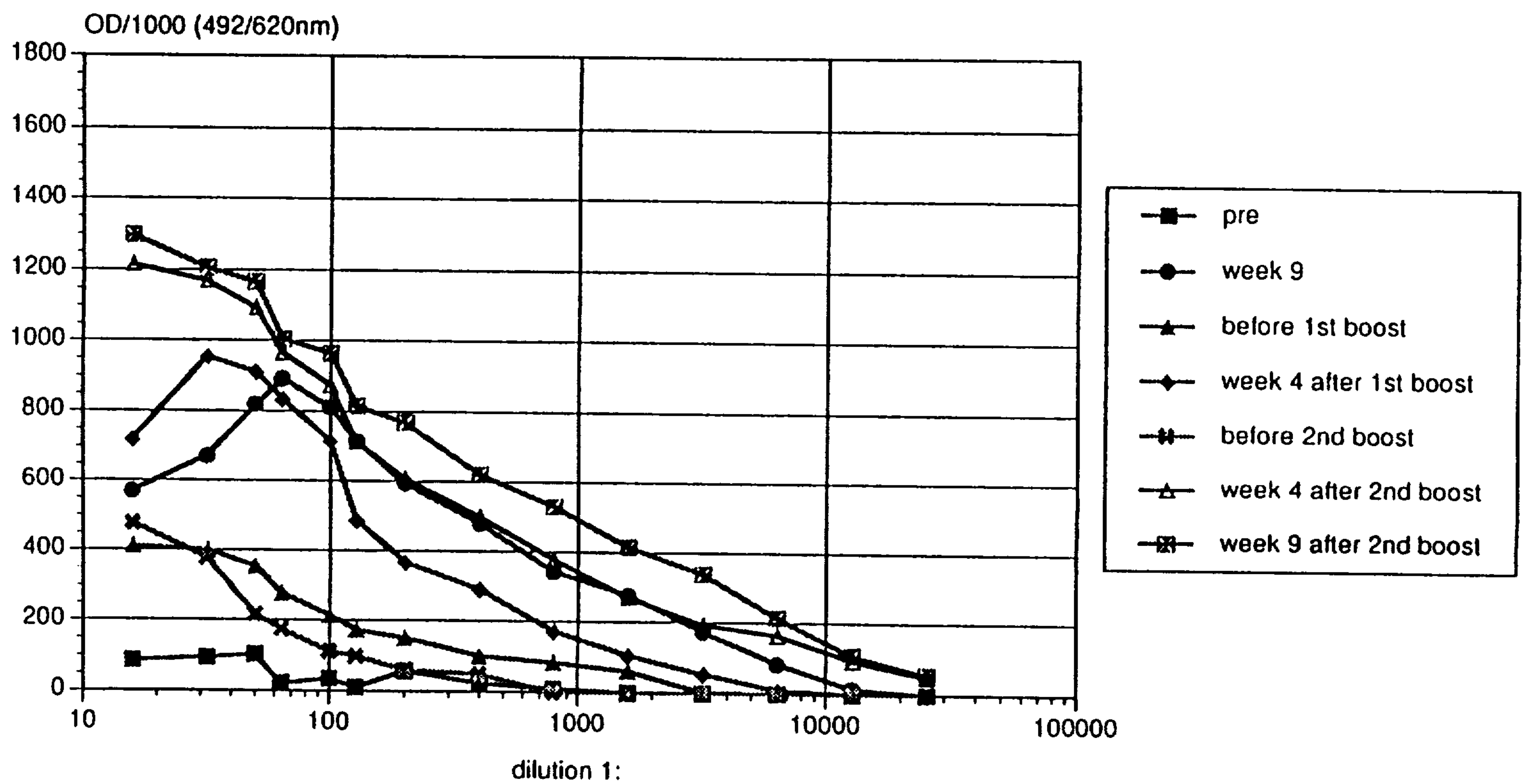

Figure 18

Immunization of monkey 215 with irrelevant mouse IgG1
Serum Ig binding to SW2 small cell lung cancer cells
(cell- ELISA)

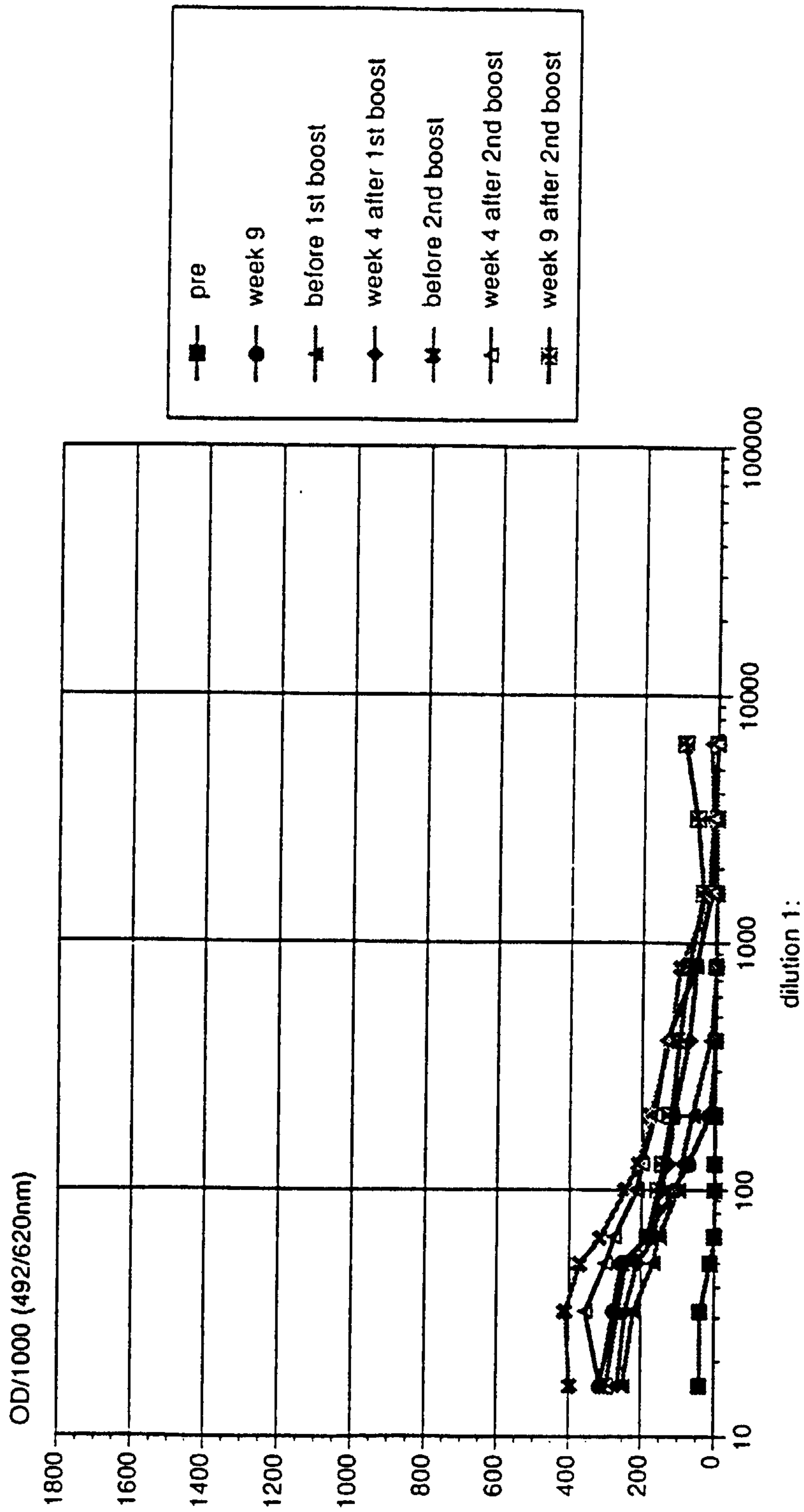

Figure 19

# Immunization of monkey E 23 with anti-id BR55-2#E4 Serum Ig binding to SW 948 colon cancer cells (cell- ELISA)

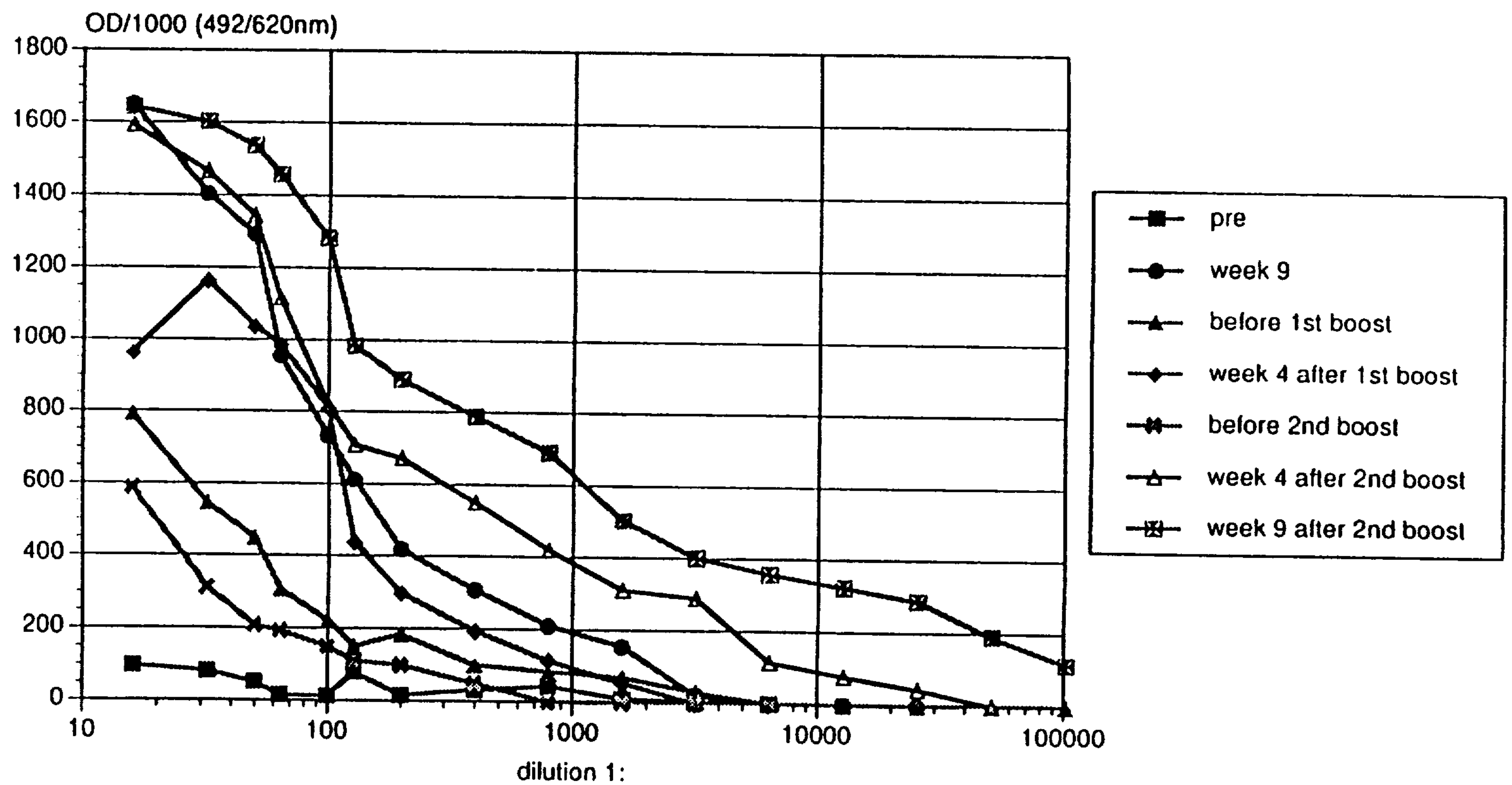

Figure 20

Immunization of monkey E23 with anti-id BR55-2#E4
Serum Ig binding to WM 9 melanoma cells
(cell- ELISA)

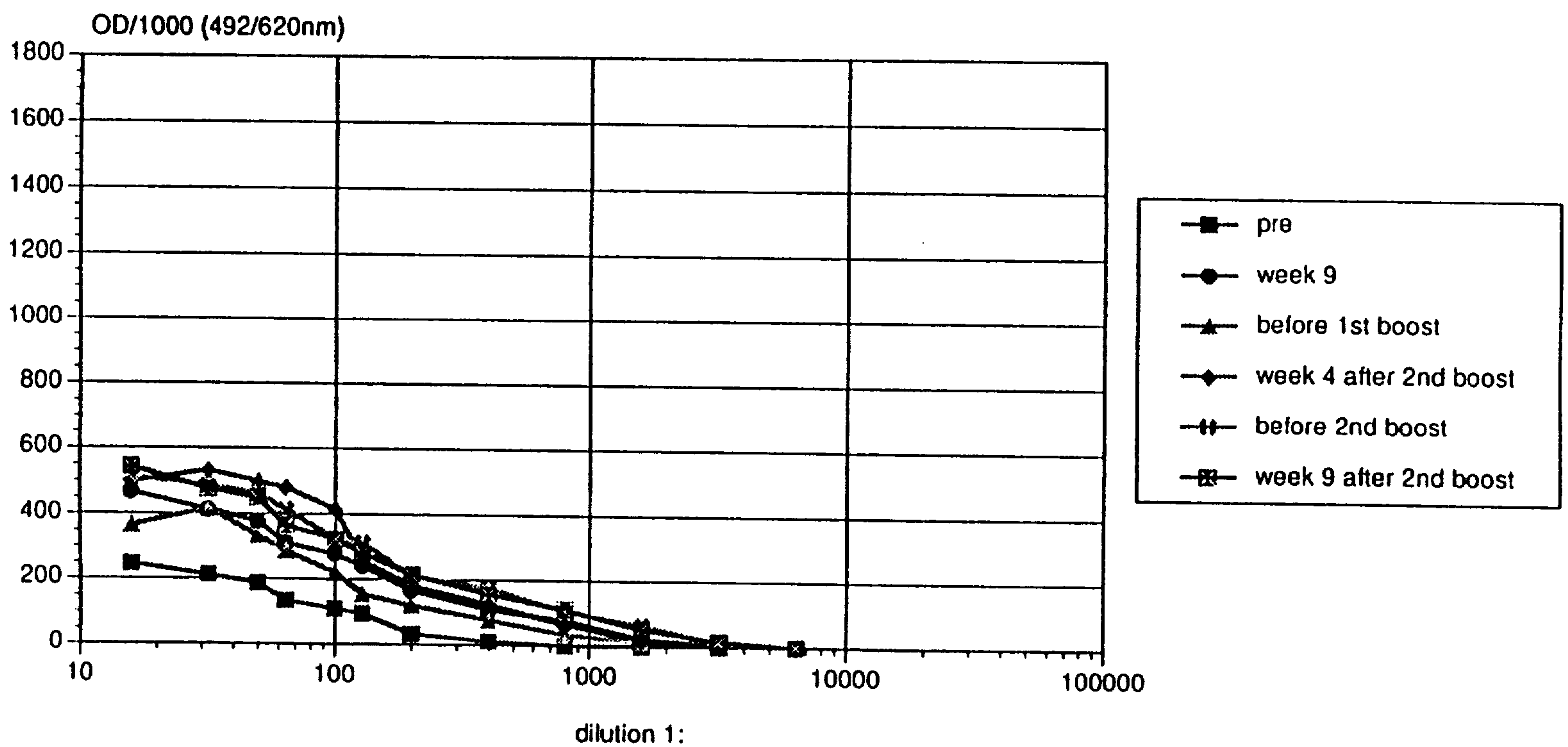

Figure 21

# Rhesus monkey Ig immunopurified on anti-id BR55-2#E4*: binding to anti-id BR55-2#E4 (ELISA)

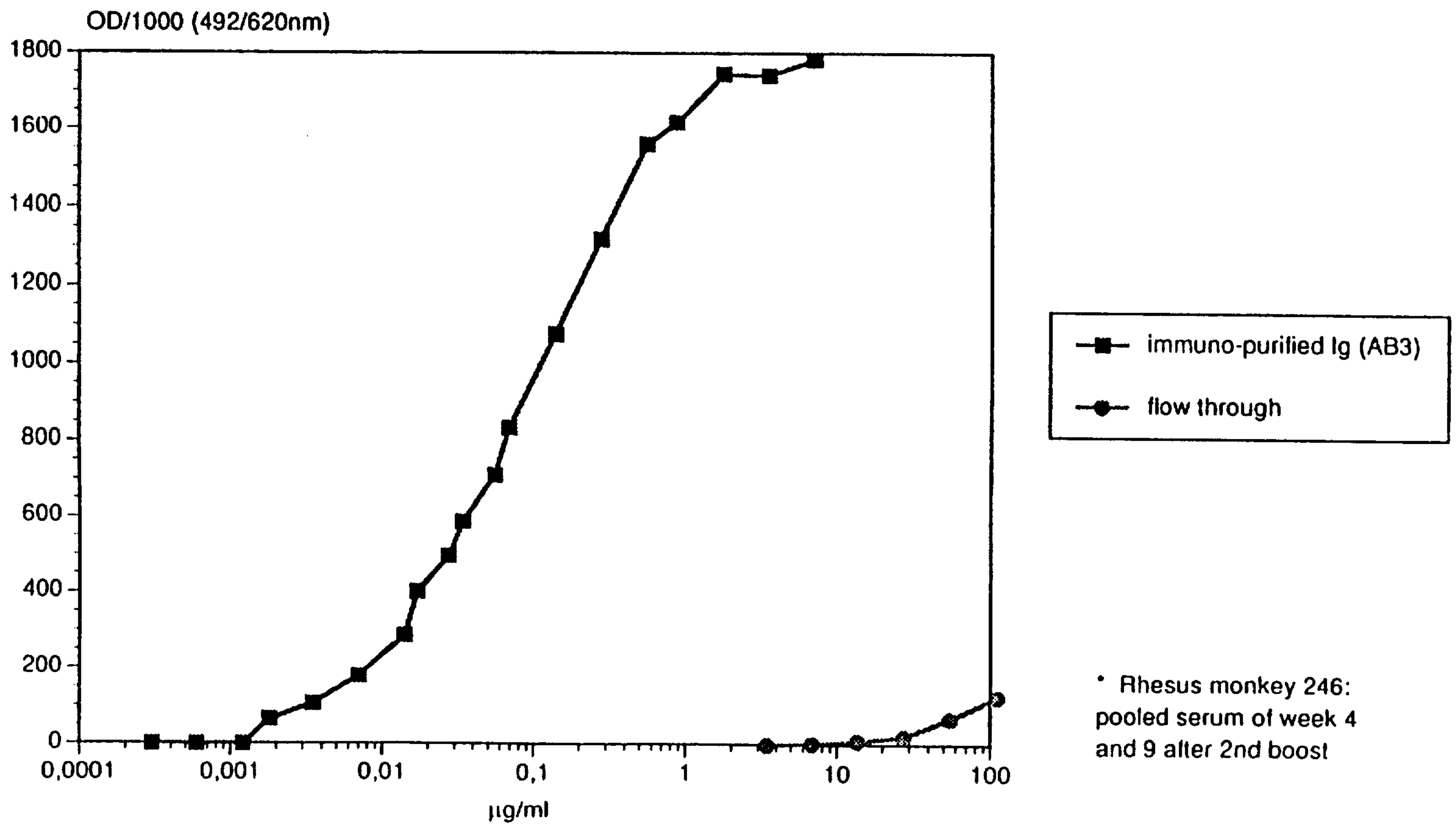

Figure 22

# Rhesus monkey Ig immunopurified on anti-id BR55-2#E4*: binding to SKBR5 breast cancer cells (cell-ELISA)

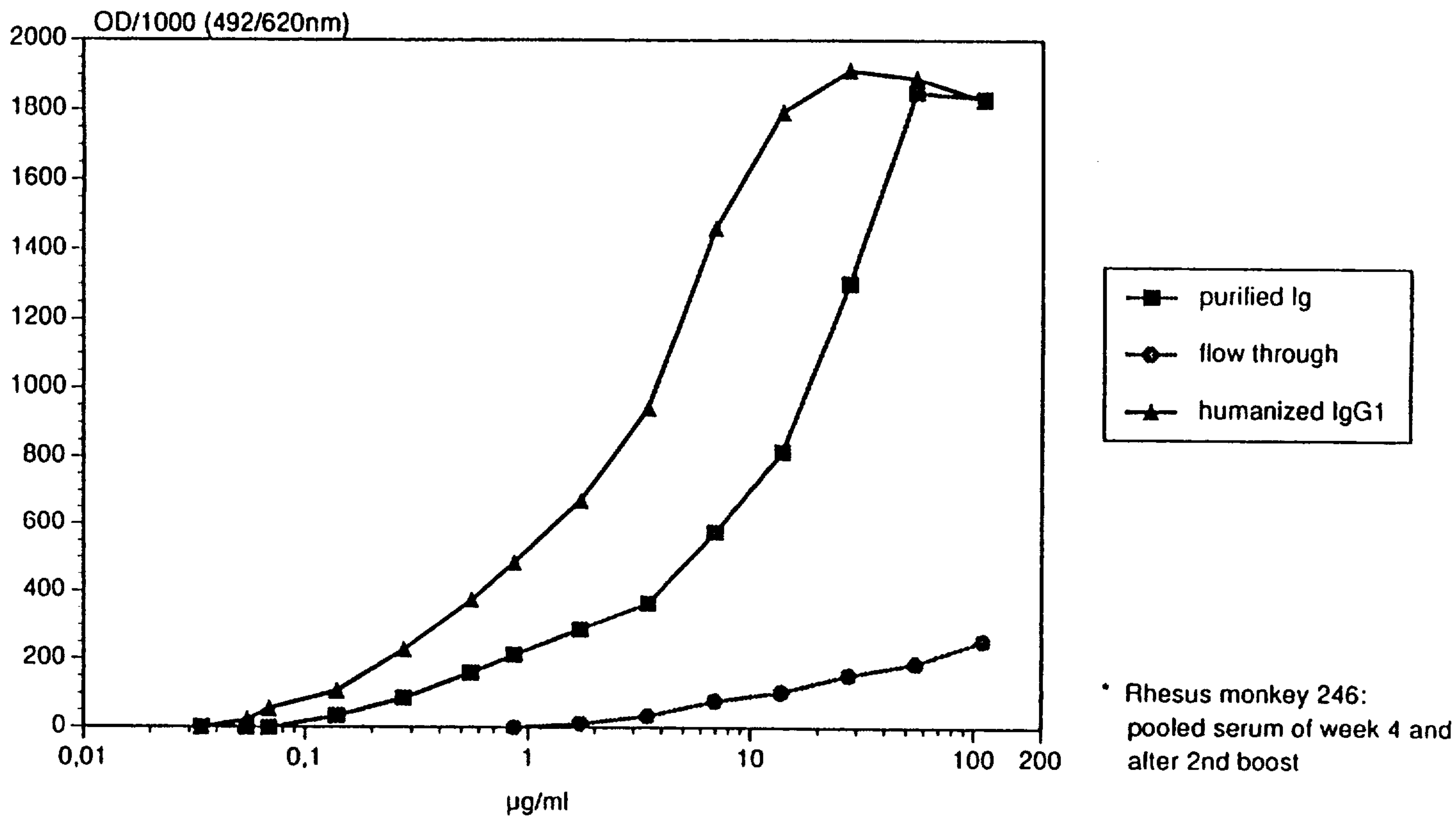


* Rhesus monkey 246:
pooled serum of week 4 and 9
alter 2nd boost

Figure 23

Rhesus monkey Ig immunopurified on anti-id BR55-2#E4*: binding to WM9 (melanoma) and SKBR5 (breast cancer) cells (cell-ELISA)

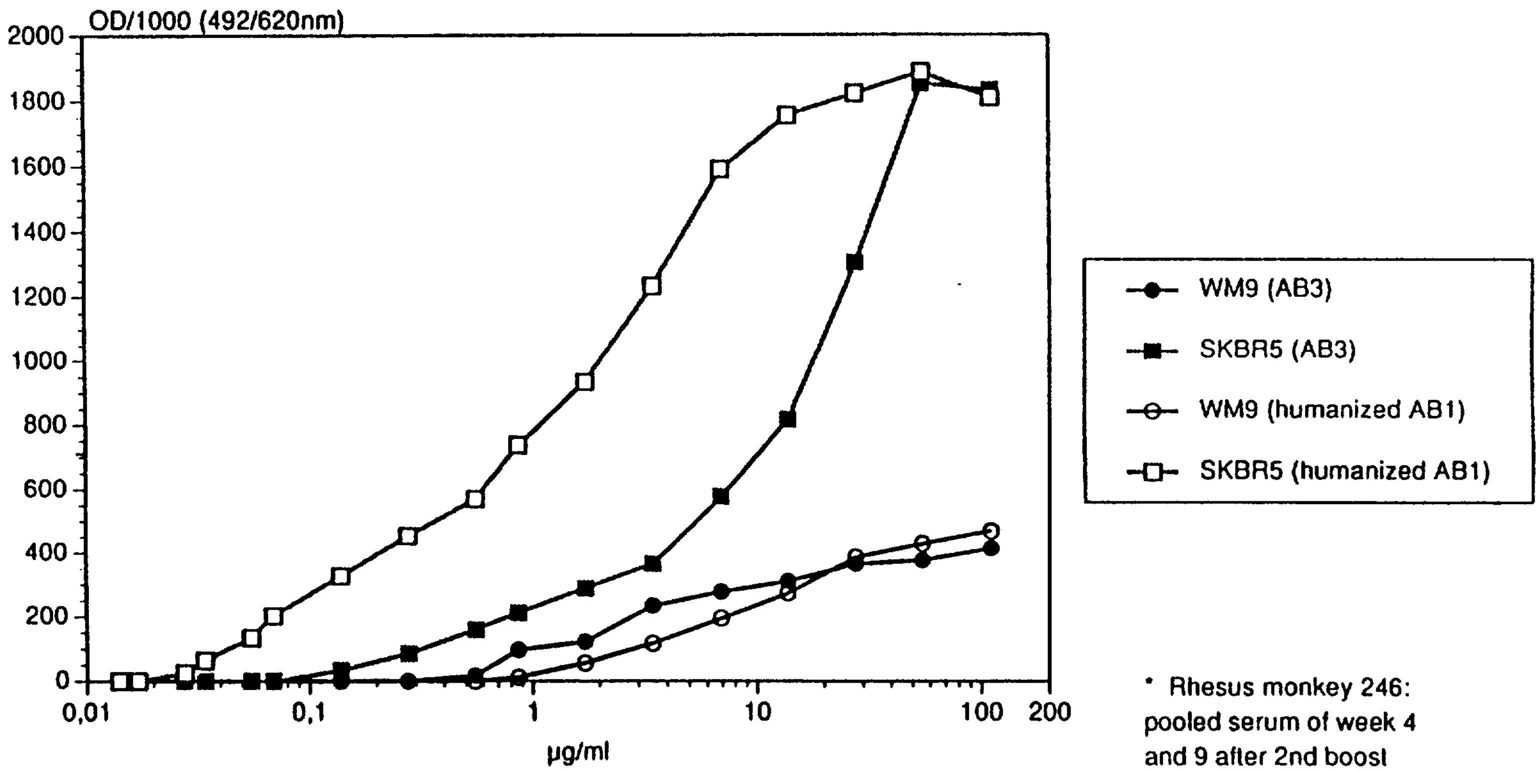


* Rhesus monkey 246: pooled serum of week 4 and 9 after 2nd boost

Figure 24

# Rhesus monkey Ig immunopurified on anti-id BR55-2#E4*: binding to SKBR5 - and WM9 - cell membranes (ELISA)

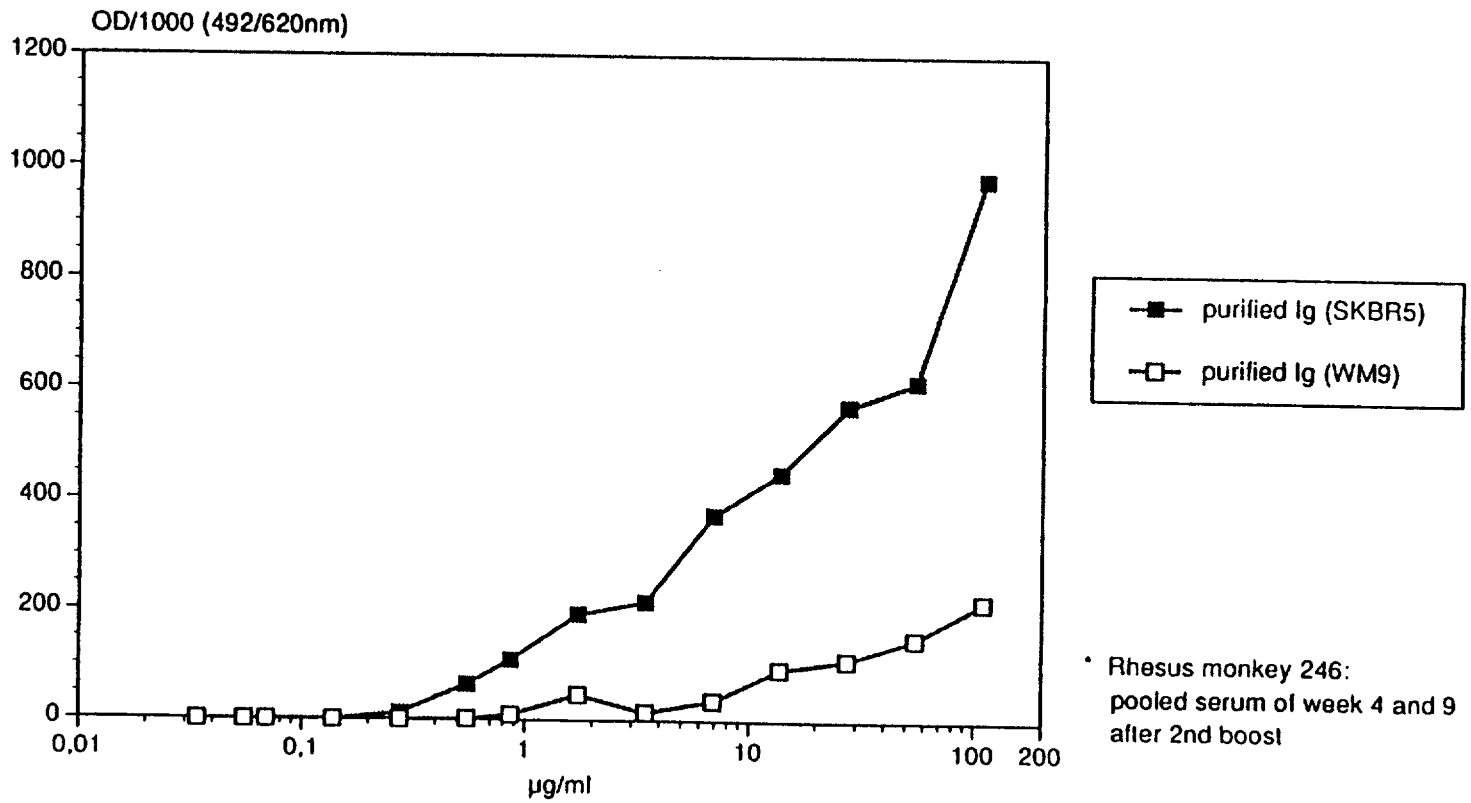


* Rhesus monkey 246: pooled serum of week 4 and 9 after 2nd boost

Figure 25

Rhesus monkey Ig immunopurified on anti-id BR55-2#E4*: binding to different cell lines (cell-ELISA)

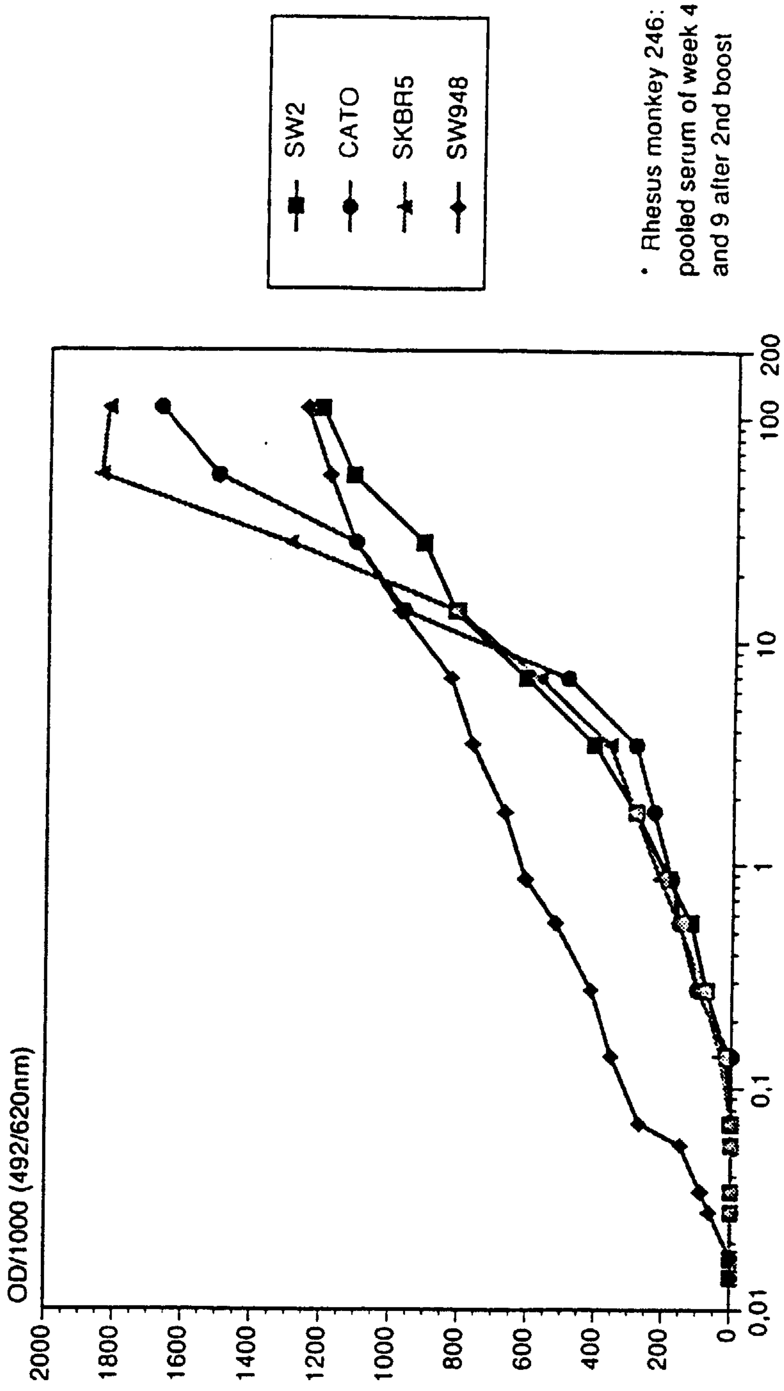

Figure 26

# ADCC to SKBR5 breast cancer cell line mediated by human PBMC and BR55-2/murine IgG3 or immunopurified Ig of a rhesus monkey* immunized with anti-id BR55-2#E4

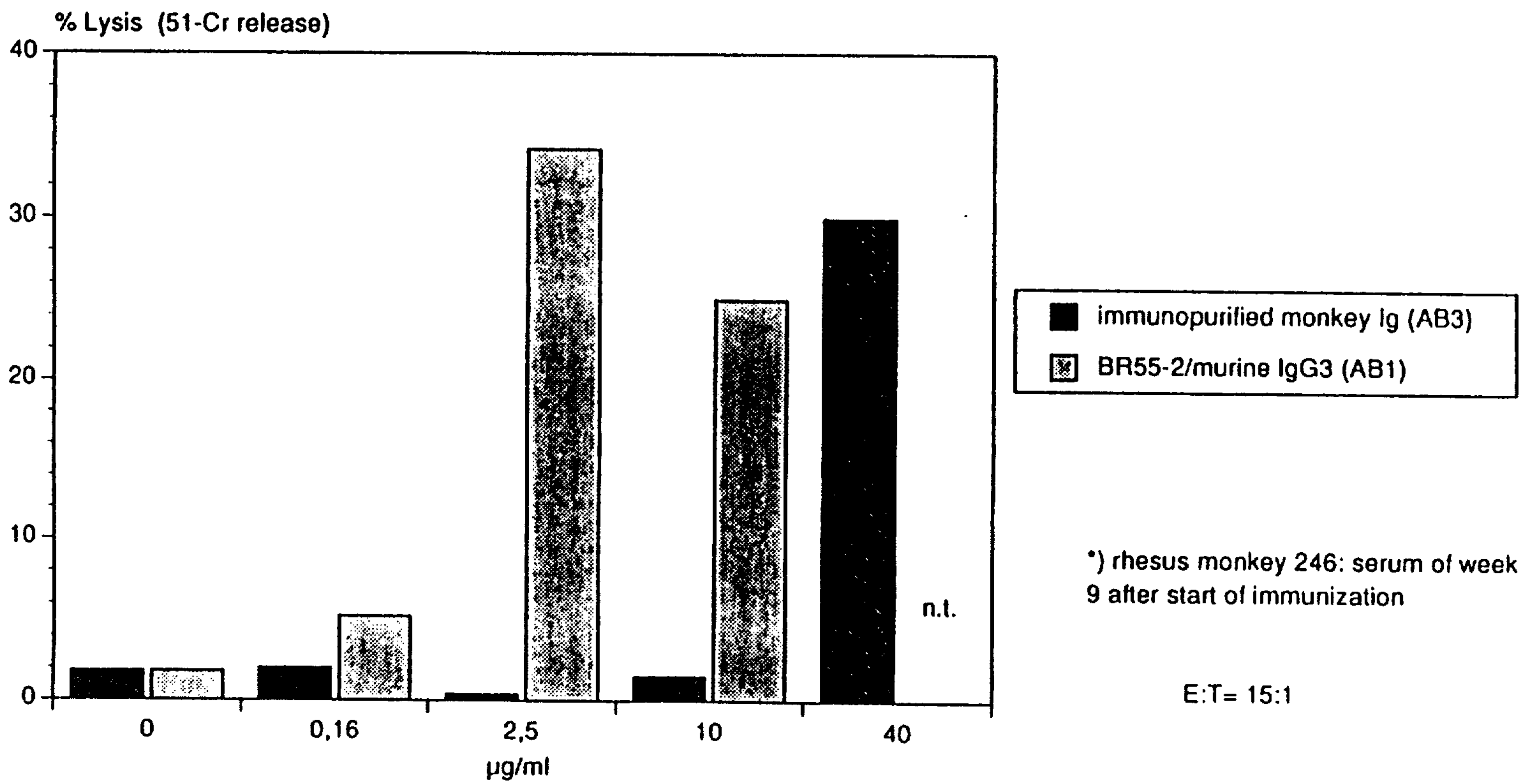


*) rhesus monkey 246: serum of week 9 after start of immunization

E:T= 15:1

Figure 27

ELISA for the determination of
BR 55-2/murine IgG3 in human serum
Standard curve

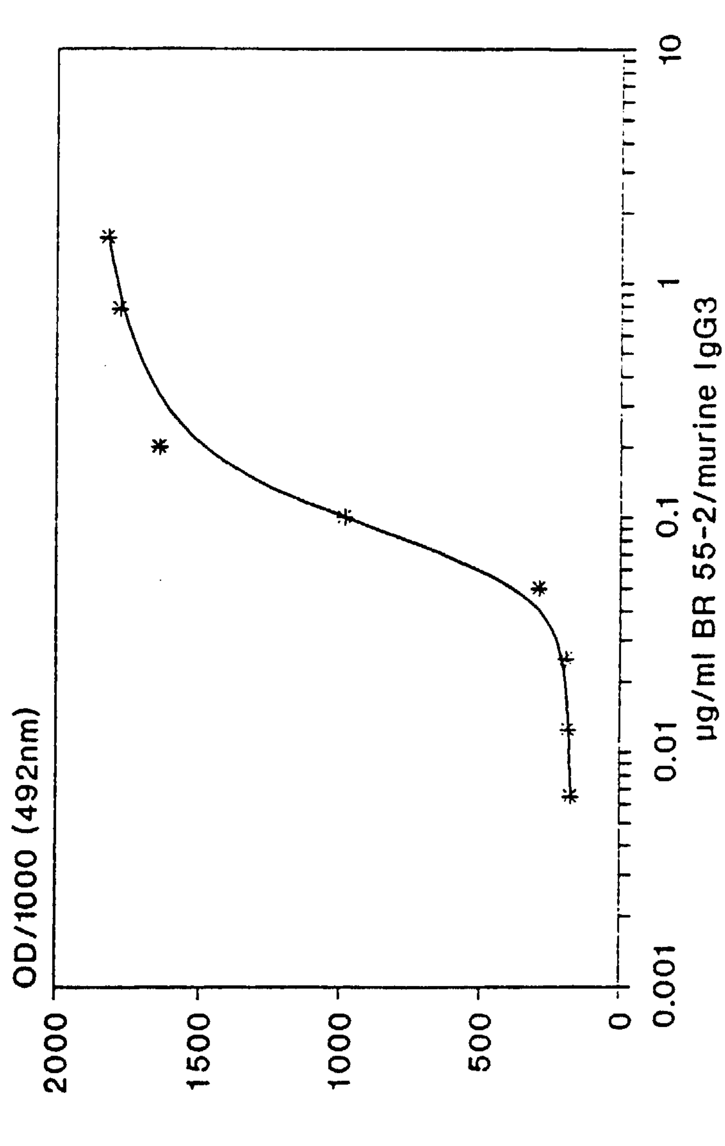

Figure 28

ELISA for the determination of BR 55-2/chimeric human IgG1 in human serum - standard curve

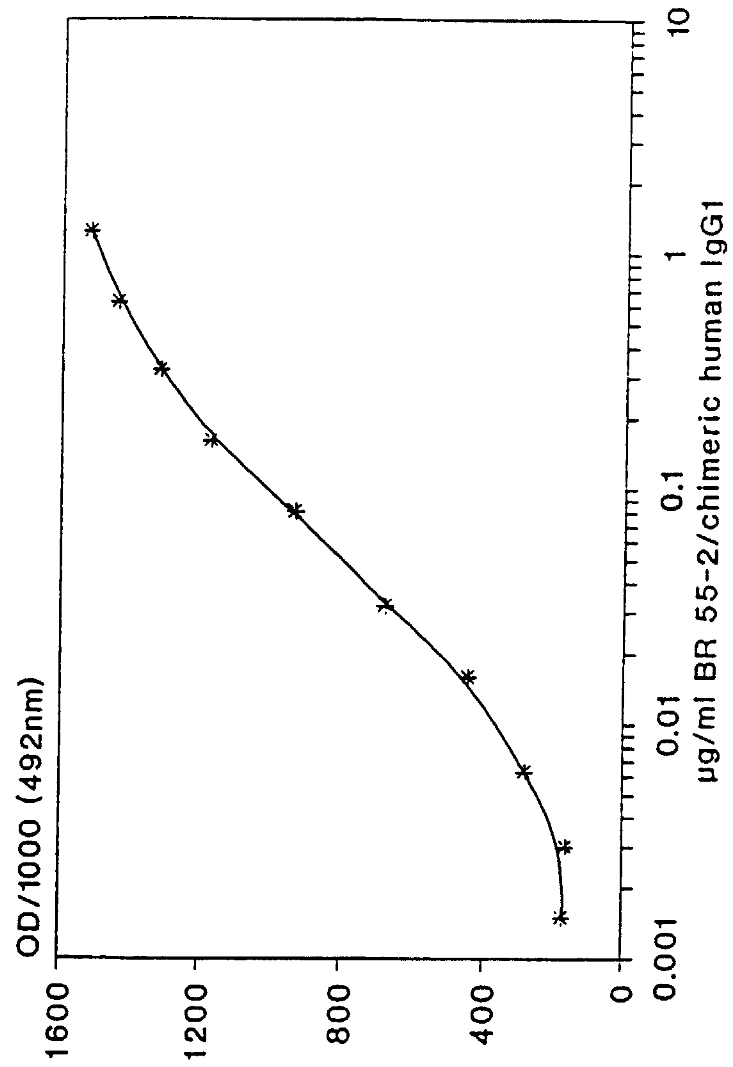

Figure 29

ELISA for the determination of BR 55-2/chimeric human IgG3 in human serum - standard curve

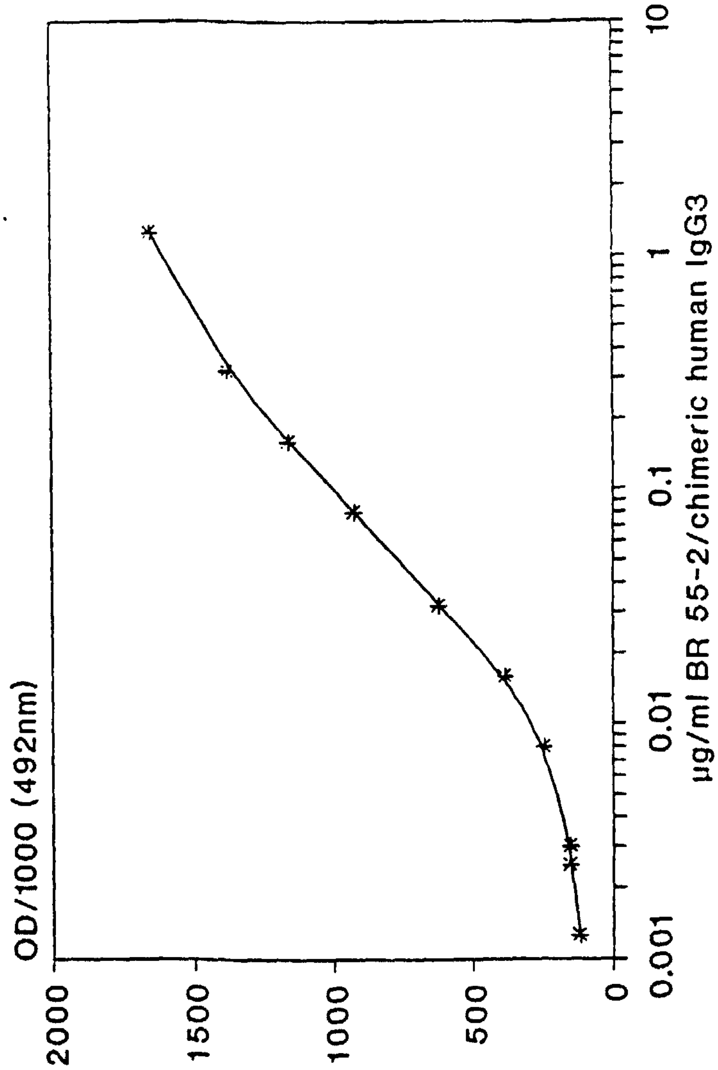

Figure 30

Binding of BR55-2/chimeric IgG1 and BR55-2/humanized IgG1 to anti-id BR55-2#E4

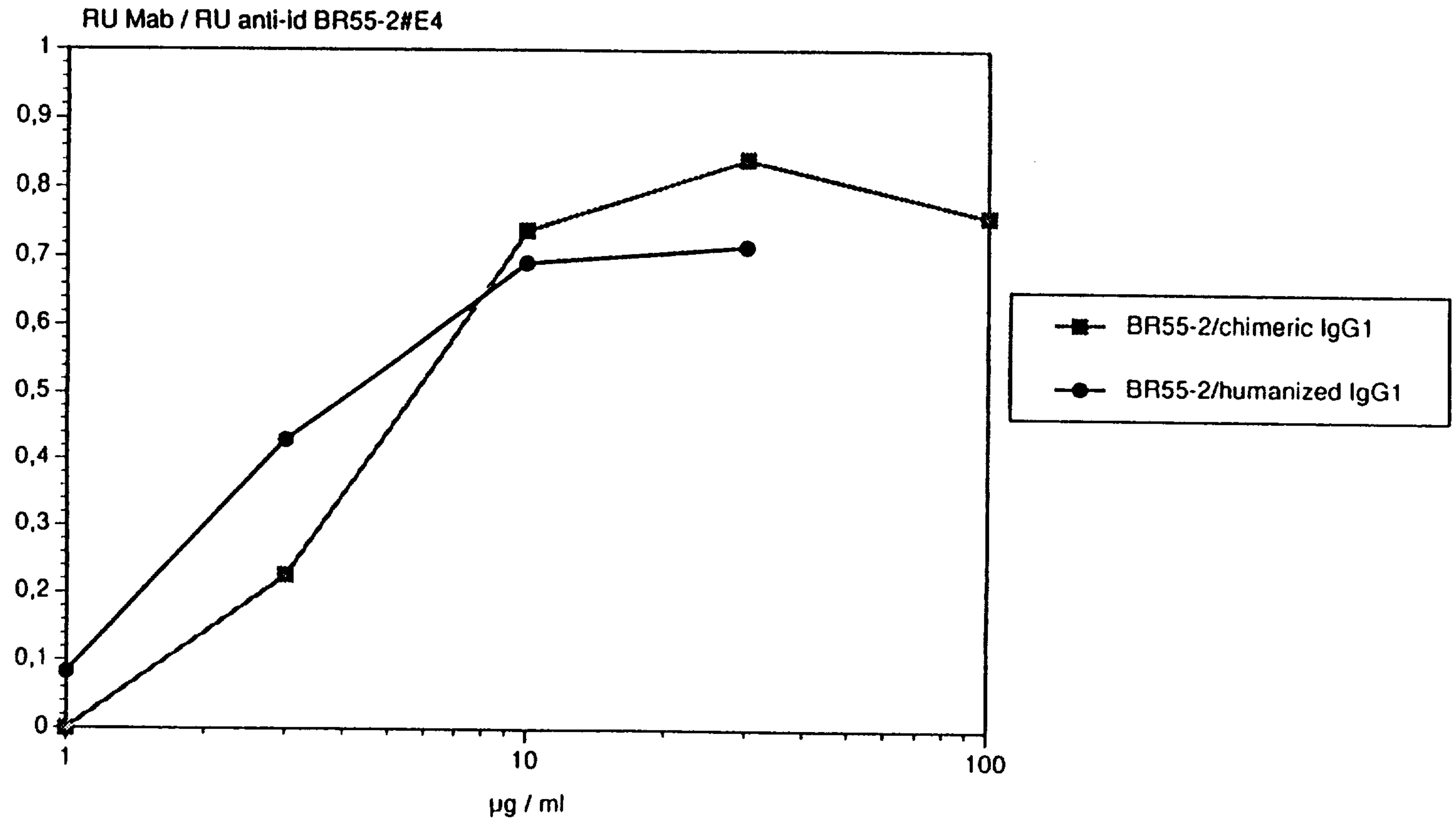

Figure 31

# Binding of BR55-2/chimeric IgG1 and BR55-2/humanized IgG1 to anti-id BR55-2#C11

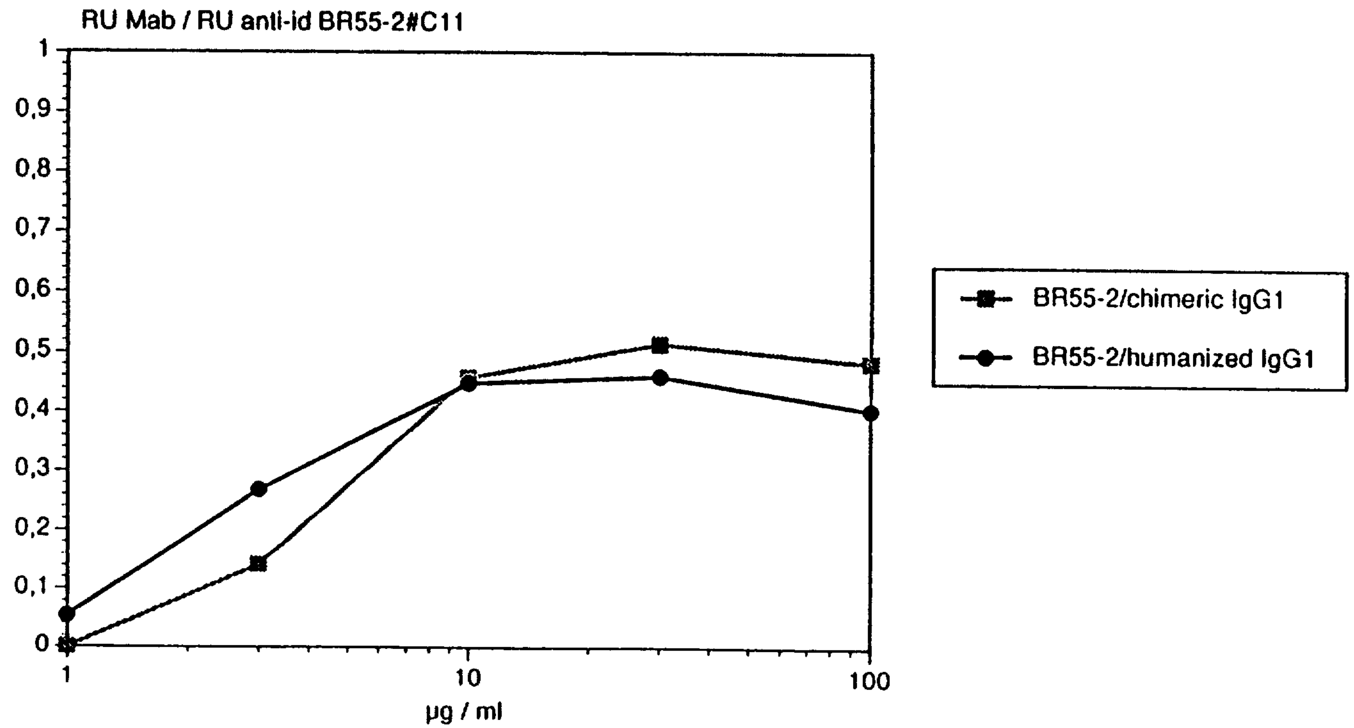

Figure 32

# Binding of BR55-2/chimeric IgG1 and BR55-2/humanized IgG1 to anti-id BR55-2#B3

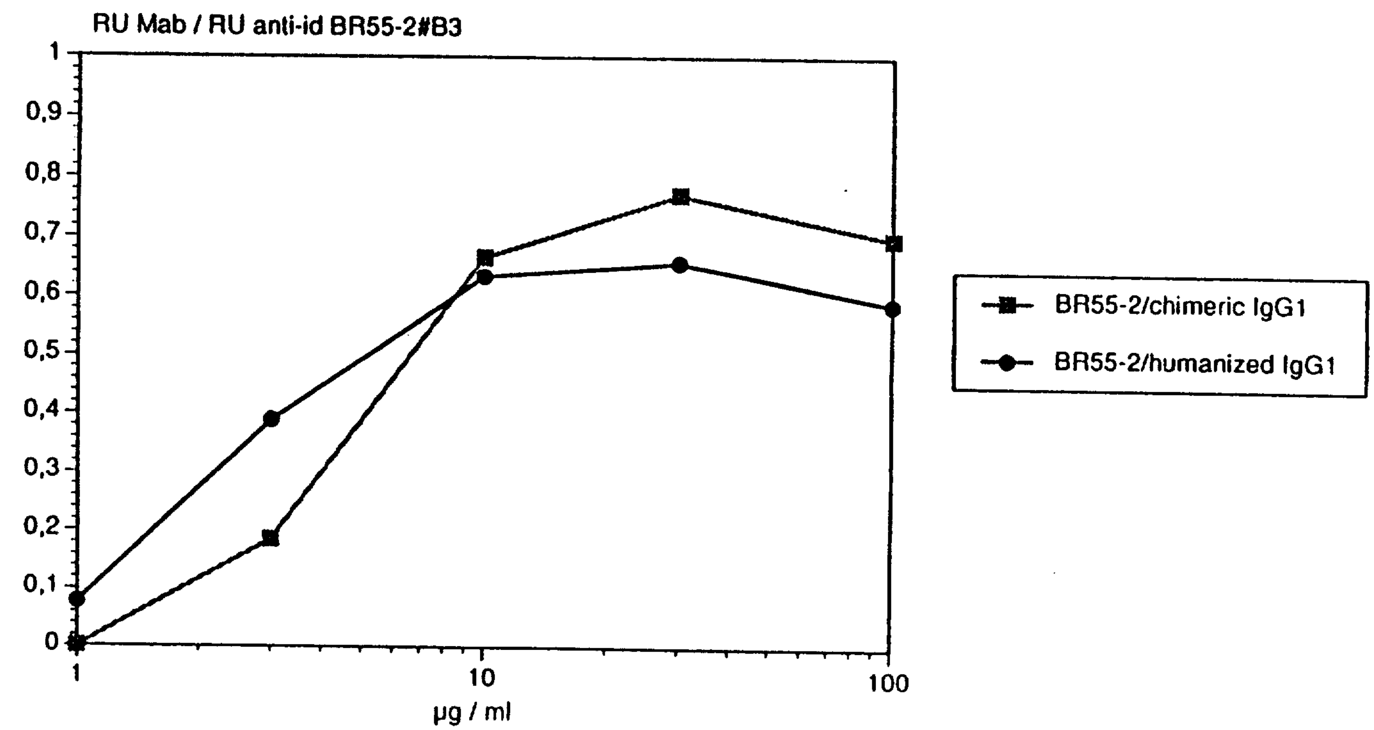

Figure 33

## Binding of BR55-2/chimeric IgG1 and BR55-2/humanized IgG1 to anti-id BR55-2#B9

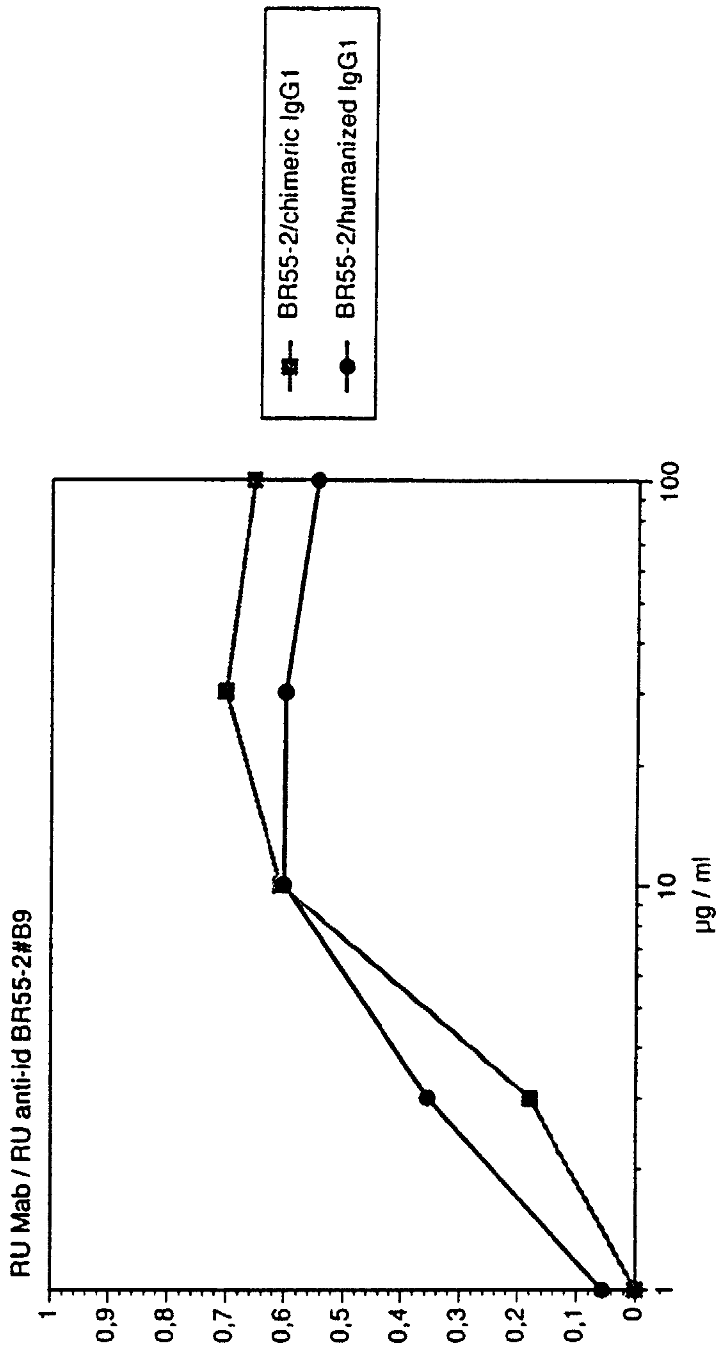

Figure 34

# Binding of BR55-2/chimeric IgG1 and BR55-2/humanized IgG1 to anti-id BR55-2#G6

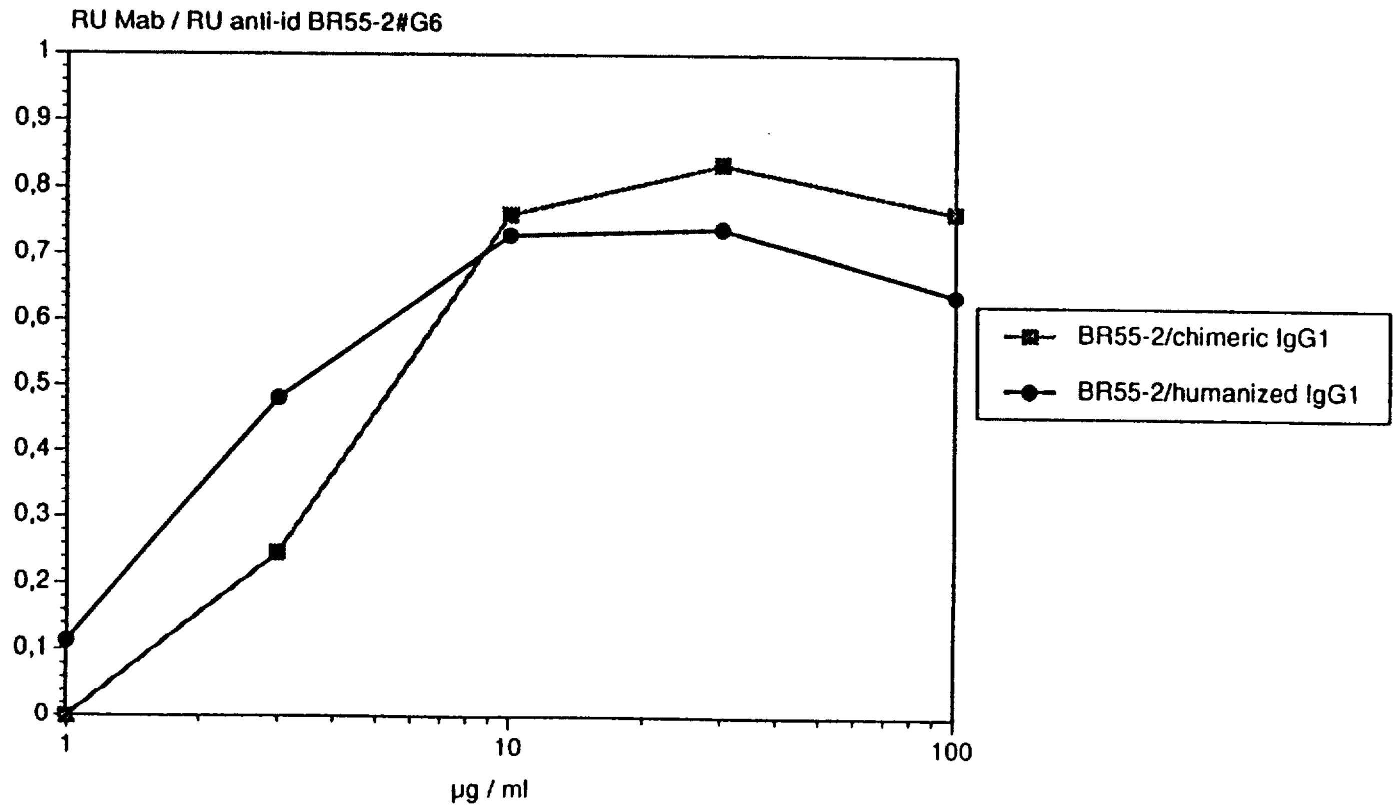

Figure 35

# Binding of BR55-2/chimeric IgG1 and BR55-2/humanized IgG1 to anti-id BR55-2#G9

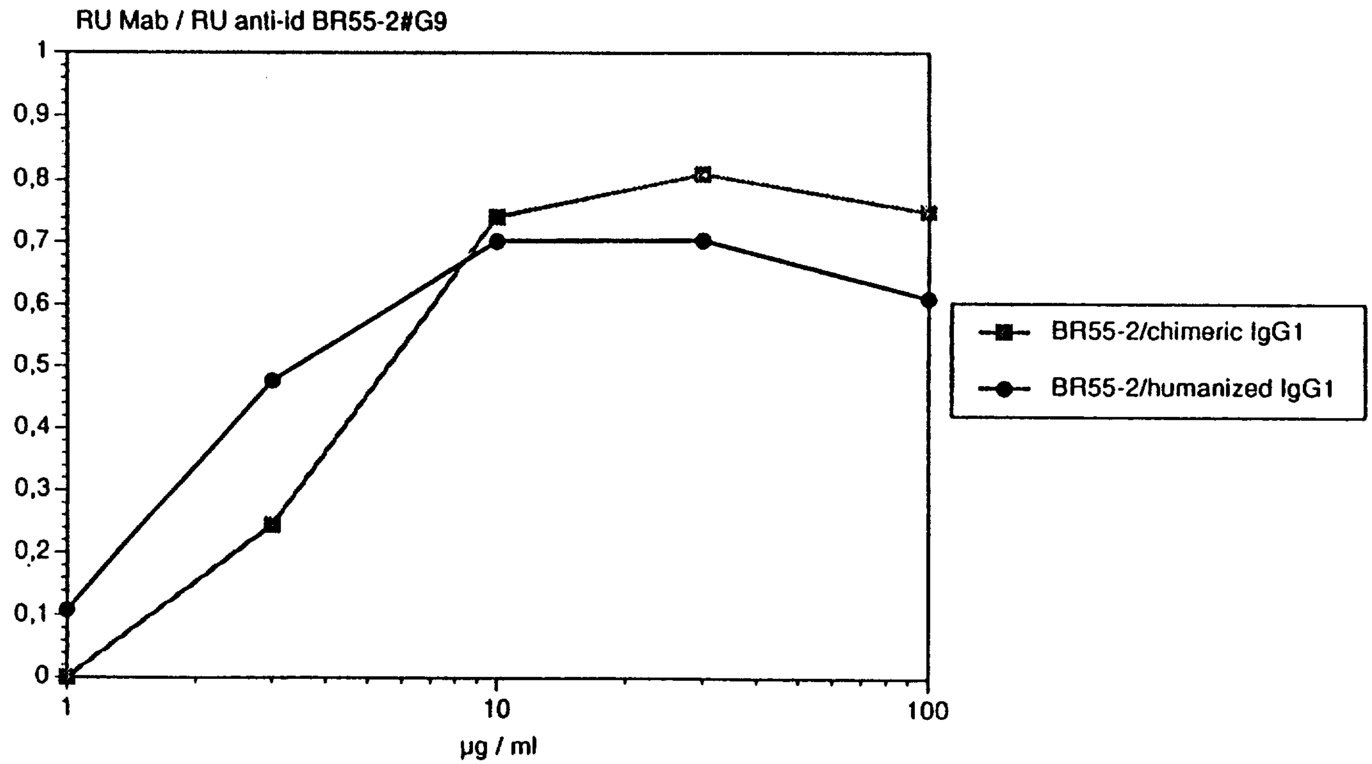

Figure 36

ELISA for the determination of
BR 55-2/humanized IgG1 in human serum
Standard curve

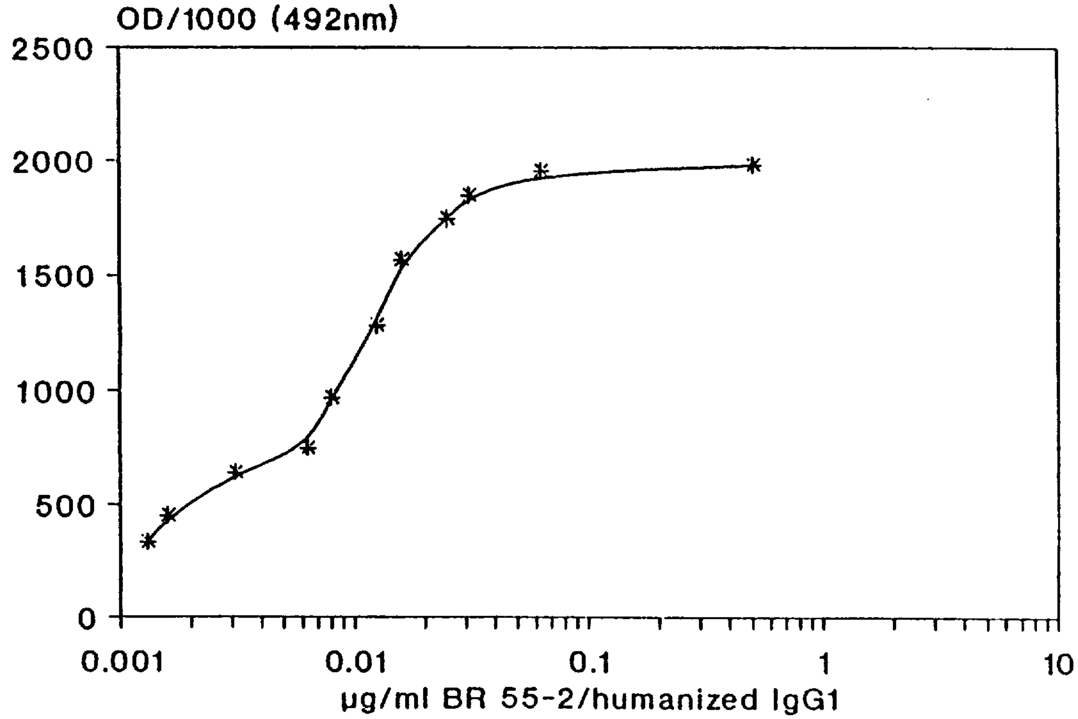

Figure 37

Binding competition of BR55-2/murineIgG3 to a-ID BR55-2 by BR55-2/chim.human IgG1 and BR55-2/chim.human IgG3

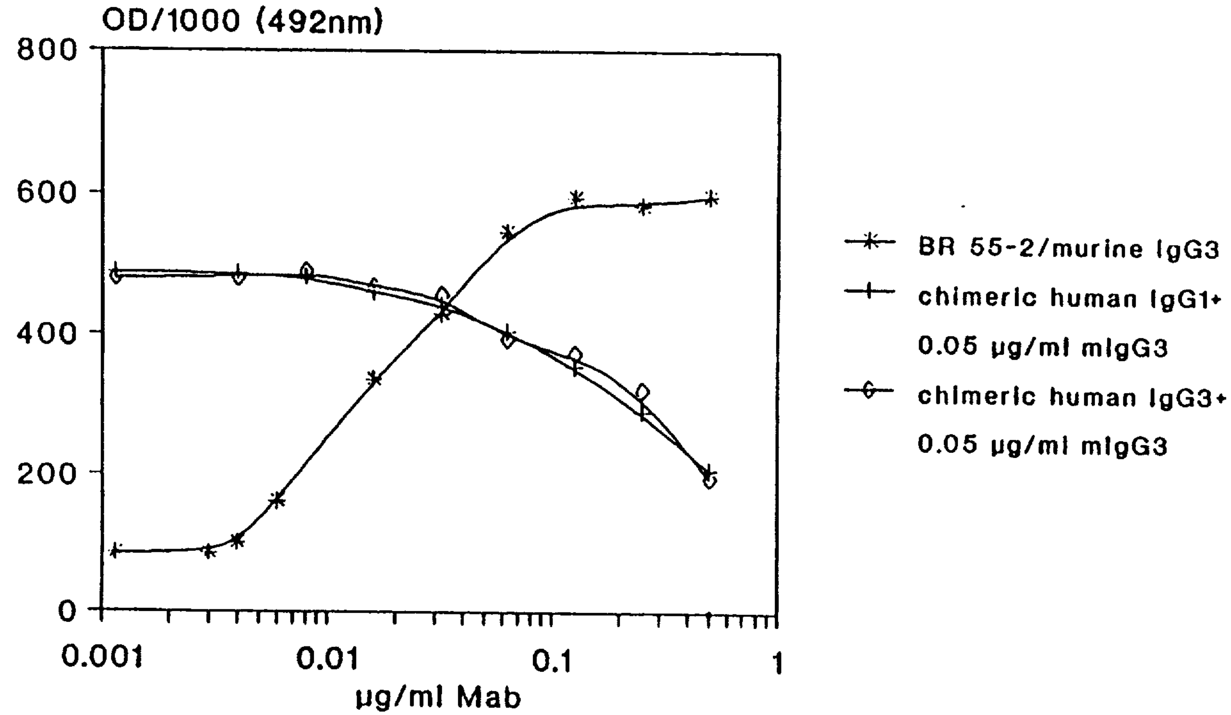

Figure 38

Complement dependent cytotoxicity mediated by BR 55-2/murine IgG3 Inhibition by anti-ID BR 55-2 E4

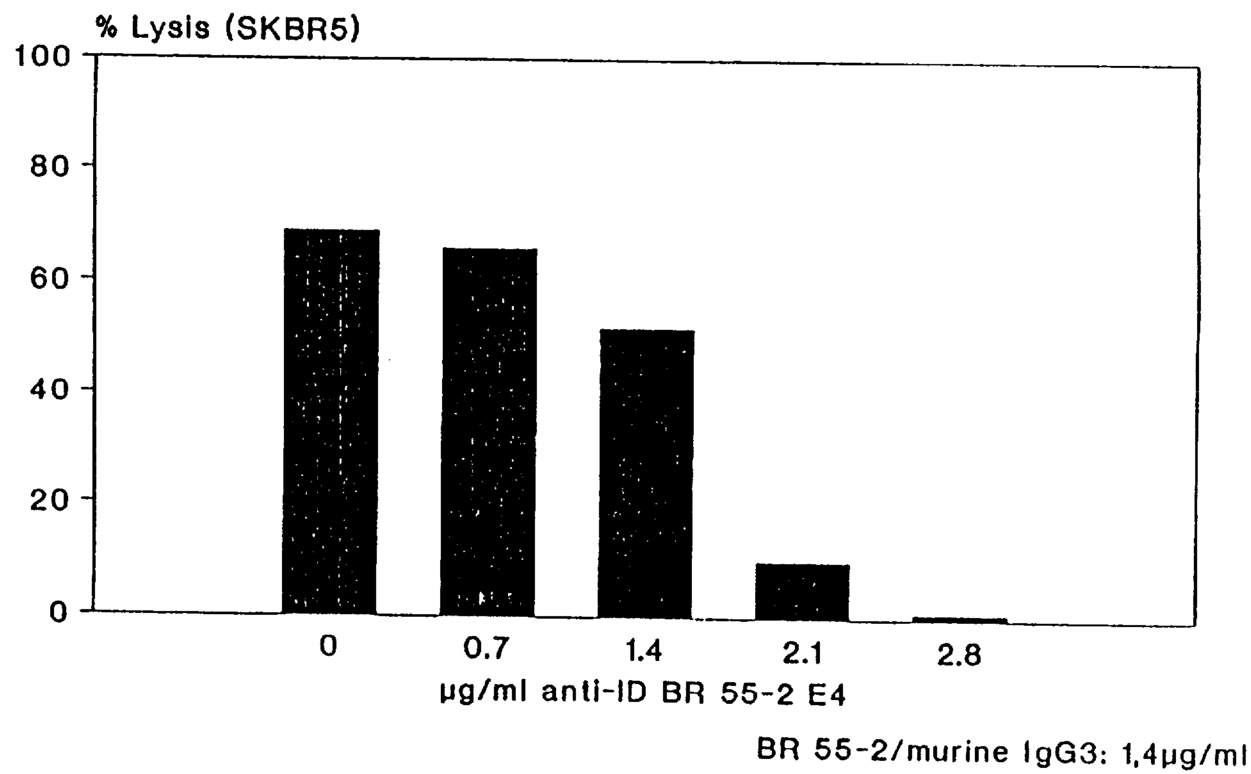

Figure 39

SDS-PAGE
Reducing conditions

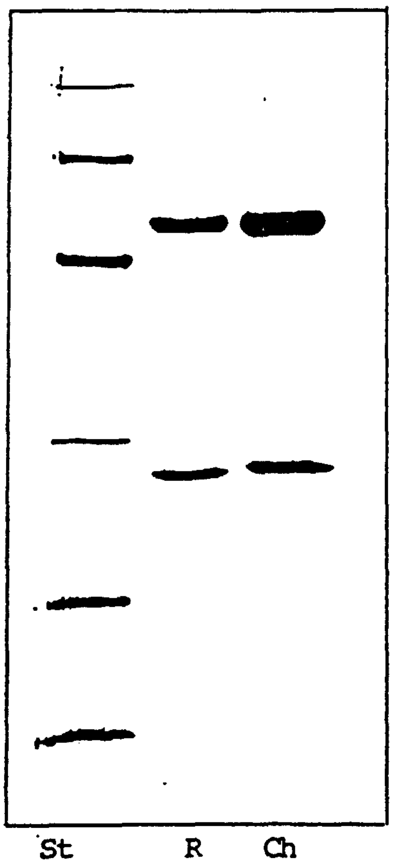


St = Standards
R = Reference Mab
Ch = BR55-2/chimeric human IgG1